(19) Europäisches Patentamt
European Patent Office
Office européen des brevets

(11) Veröffentlichungsnummer: **0 402 923 A2**

# (12) EUROPÄISCHE PATENTANMELDUNG

(21) Anmeldenummer: **90111244.1**

(22) Anmeldetag: **13.06.90**

(51) Int. Cl.5: **C07C 211/60, A61K 31/135, C07C 233/43, A61K 31/16, C07D 209/16, C07D 333/20, C07D 307/52, C07D 307/14, C07C 233/44, C07C 271/20, C07C 275/40**

(30) Priorität: **15.06.89 DE 3919624**

(43) Veröffentlichungstag der Anmeldung:
**19.12.90 Patentblatt 90/51**

(84) Benannte Vertragsstaaten:
**AT BE CH DE DK ES FR GB GR IT LI LU NL SE**

(71) Anmelder: **BOEHRINGER INGELHEIM KG**

**D-6507 Ingelheim am Rhein(DE)**
(84) **AT BE CH DE DK ES FR GB GR IT LI LU NL SE**

Anmelder: **BOEHRINGER INGELHEIM INTERNATIONAL G.M.B.H.**

**D-6507 Ingelheim am Rhein(DE)**
(84) **GB**

(72) Erfinder: **Grauert, Matthias, Dr. Dipl.-Chem.**
**Eltviller Strasse 2**
**D-6507 Ingelheim am Rhein(DE)**
Erfinder: **Merz, Herbert, Dr. Dipl.-Chem.**
**Rotweinstrasse 53**
**D-6507 Ingelheim am Rhein(DE)**
Erfinder: **Mierau, Joacim, Dr.**
**An den Weiden 3**
**D-6500 Mainz 33(DE)**
Erfinder: **Schingnitz, Günter, Dr.**
**Unter den Gärten 10**
**D-6550 Bad Kreuznach(DE)**
Erfinder: **Schneider, Claus, Dr. Dipl.-Chem.**
**Im Konrad-Adenauer-Ring 21**
**D-6904 Eppelheim(DE)**

## (54) Neue 2,5-Diaminotetraline, Verfahren zu ihrer Herstellung und ihre Verwendung als Arzneimittel.

(57) Die Erfindung betrifft neue 2,5-Diaminotetraline, Verfahren zur ihrer Herstellung und ihre Verwendung als Arzneimittel.

EP 0 402 923 A2

## Neue 2,5-Diaminotetraline, Verfahren zu ihrer Herstellung und ihre Verwendung als Arzneimittel

### 1. Definitionen

Die Erfindung betrifft neue 2,5-Diaminotetraline (2,5-Diamino-1,2,3,4-tetrahydronaphthaline), ihre Herstellung und Verwendung als Arzneimittel.
Die erfindungsgemäßen 1,5-Diaminotetraline entsprechen der allgemeinen Formel 1

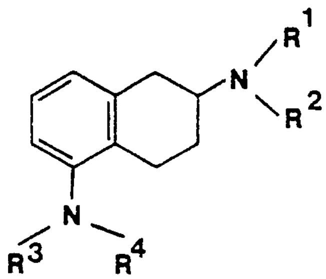

1

worin
$R^1$ Wasserstoff, $C_1$-$C_{12}$-Alkyl, $C_3$-$C_{12}$-Alkenyl, $C_3$-$C_{12}$-Alkinyl, -$(CH_2)_a$-Cycloalkyl, Aralkyl und a eine Zahl 1,2,3,4,5,6,7,8,9,10,11,12:
$R^2$ $C_1$-$C_{12}$-Alkyl, $C_3$-$C_{12}$-Alkenyl, $C_3$-$C_{12}$-Alkinyl, -$(CH_2)_b$-Cycloalkyl,

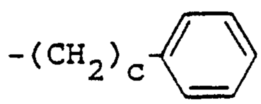 ,

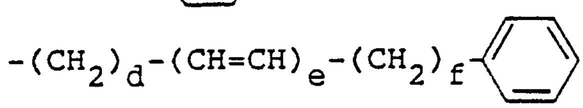 ,

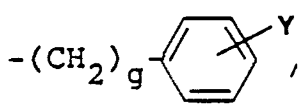 ,

-$(CH_2)_h$-Heteroaryl, Acyl
und
b eine Zahl 1,2,3,4,5,6,7,8,9,10,11,12,
c eine Zahl 1,2,3,4,5,6,7,8,9,10,11,12,
d eine Zahl 1,2,3,4,5,6,
e eine Zahl 1,2,3,
f eine Zahl 0,1,2,3,4,
g eine Zahl 1,2,3,4,5,6,
h eine Zahl 1,2,3,4,5,6,7,8,9,10,11,12,13,14,15, 16,17,18,19,20,
und
Y $C_1$-$C_{12}$-Alkyl, Halogen, Alkoxy, Hydroxy;
$R^3$ Wasserstoff, $C_1$-$C_{12}$-Alkyl, $C_3$-$C_{12}$-Alkenyl, $C_3$-$C_{12}$-Alkinyl, -$(CH_2)_i$-Cycloalkyl, Aralkyl, Formyl, Acyl, Alkylcarbonyl, Alkyloxycarbonyl,

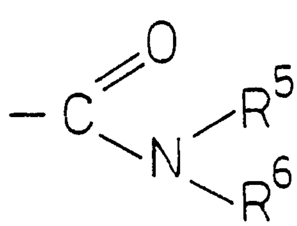 ,

$R^5$ Alkyl,
$R^6$ Alkyl,
$R^5$ und $R^6$ auch zusammen mit dem Stickstoffatom einen Heterocyclus, der noch ein weiteres Heteroatom - wie z.B. Stickstoff, Sauerstoff oder Schwefel - enthalten kann -beispielsweise einen Morpholin-, Piperidin- oder Piperazinring - bilden können
und
i eine Zahl 1,2,3,4,5,6,7,8,9,10,11,12;
$R^4$ Wasserstoff oder $C_1$-$C_{12}$-Alkyl
bedeutet.

Bevorzugt werden Verbindungen der allgemeinen Formel 1, worin $R^1$ Wasserstoff, $C_1$-$C_6$-Alkyl, $C_3$-$C_6$-Alkenyl, $C_3$-$C_6$-Alkinyl -$(CH_2)_a$-Cycloalkyl, Aralkyl mit 1 bis 6 Kohlenstoffatomen in aliphatischen Teil und a eine Zahl 1,2,3,4,5,6;
$R^2$ $C_1$-$C_6$-Alkyl, $C_3$-$C_6$-Alkenyl, $C_3$-$C_6$-Alkinyl, -$(CH_2)_b$-Cycloalkyl,

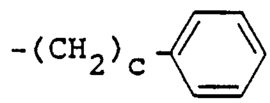 ,

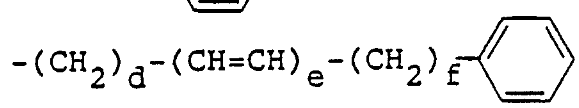 ,

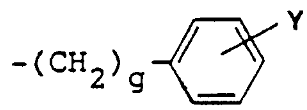 ,

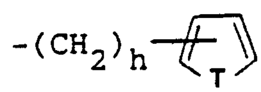 ,

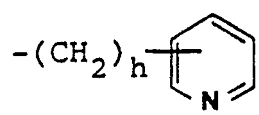 ,

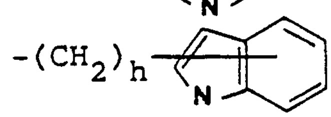 ,

Acyl
und
b eine Zahl 1,2,3,4,5,6,
c eine Zahl 1,2,3,4,5,6,7,8,9,10,
d eine Zahl 1,2,3,
e eine Zahl 1,2,
f eine Zahl 0,1,2,
g eine Zahl 1,2,3,4,
h eine Zahl 1,2,3,4,5,6,7,8,9,10,12,13,14
und
Y $C_1$-$C_6$-Alkyl, Halogen, Niederalkoxy, Hydroxy,
T Sauerstoff, Schwefel, Stickstoff;
$R^3$ Wasserstoff, $C_1$-$C_6$-Alkyl, $C_3$-$C_6$-Alkenyl, $C_3$-$C_6$-Alkinyl, -$(CH_2)_i$-Cycloalkyl, Phenylalkyl, Formyl, Aryl, Alkylcarbonyl, Alkoxycarbonyl,

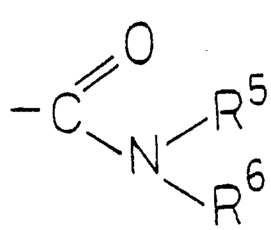 ,

$R^5$ Niederalkyl,

$R^6$ Niederalkyl,
$R^5$ und $R^6$ auch zusammen mit dem Stickstoffatom einen Heterocyclus, der noch ein weiteres Heteroatom - wie z.B. Stickstoff, Sauerstoff oder Schwefel enthalten kann -beispielsweise einen Morpholin-, Piperidin- oder Piperazinring - bilden können
und
i eine Zahl 1,2,3,4,5,6;
$R^4$ Wasserstoff oder Niederalkyl,
bedeutet.

Besonders bevorzugt sind Verbindungen der allgemeinen Formel 1, worin
$R^1$ Wasserstoff, Methyl, Ethyl, Propyl, Allyl, Propargyl, Cyclopropylmethyl, Benzyl;
$R^2$ Methyl, Ethyl, Propyl, Allyl, Propargyl, Cyclopropylmethyl,

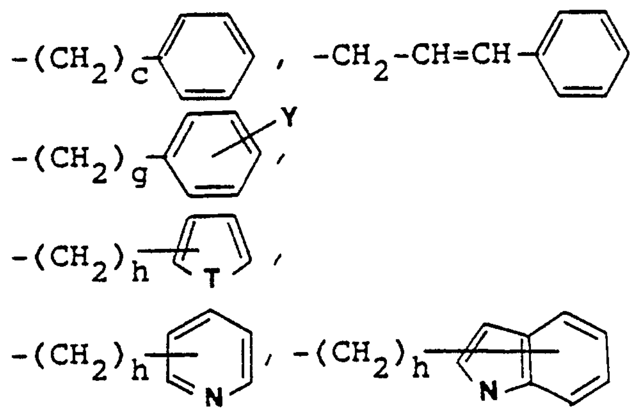

Acyl
und
c eine Zahl 1,2,3,4,5,6,7,8,9,
g eine Zahl 2,3,
h eine Zahl 1,2,3,4,5,6,7,8,9,10,11,12,
Y Methyl, Fluor, Chlor, Brom, Methoxy, Hydroxy,
T Sauerstoff oder Schwefel;
$R^3$ Wasserstoff, Methyl, Ethyl, Propyl, Allyl, Propargyl, Cyclopropylmethyl, Benzyl, Phenylethyl, Phenylpropyl, Formyl, Acyl, Methylcarbonyl, Ethylcarbonyl, Trifluormethylcarbonyl, Methoxycarbonyl, Aminocarbonyl;
$R^4$ Wasserstoff, Methyl, Ethyl, n-Propyl und iso-Propyl bedeutet.

Die erfindungsgemäßen 2,5-Diaminotetraline (2,5-Diamino-1,2,3,4-tetrahydronaphthaline) weisen mindestens ein C-Atom mit einem Asymmetriezentrum auf und können je nach Substitutionsmuster auch mehrere Asymmetriezentren besitzen und daher in verschiedenen stereochemischen Formen existieren.

Als Beispiele seien folgende Isomeren der substituierten 2,5-Diaminotetraline der allgemeinen Formel Ia und Ib genannt

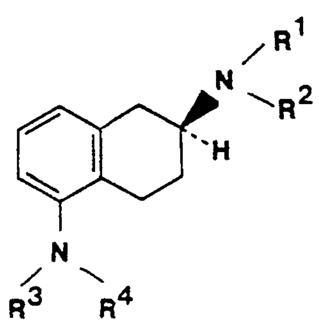

1a

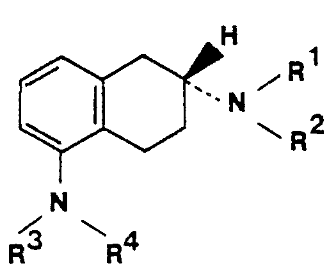

1b

Gegenstand der Erfindung sind die einzelnen Isomeren, deren Mischungen sowie die entsprechenden physiologisch geeigneten Säureadditionssalze mit anorganischen oder organischen Säuren. Bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Soweit nicht im einzelnen abweichende Angaben gemacht werden, werden die allgemeinen Definitionen im folgenden Sinn gebraucht:

Alkyl steht im allgemeinen für einen unverzweigten oder verzweigten Kohlenwasserstoffrest mit 1 bis 12 Kohlenstoffatomen, der gegebenenfalls mit einem Halogenatom oder mehreren Halogenatomen - vorzugsweise Fluor - substituiert sein kann, die untereinander gleich oder verschieden sein können, wobei Niederalkylreste bevorzugt sind. Niederalkyl steht im allgemeinen für einen verzweigten oder unverzweigten Kohlenwasserstoffrest mit 1 bis etwa 6 Kohlenstoffatomen. Als Beispiel seien Methyl, Ethyl, Propyl, Isopropyl, Butyl, Isobutyl, Pentyl, Isopentyl, Neopentyl, Hexyl, Isohexyl, Heptyl, Isoheptyl, Octyl sowie Isooctyl genannt.

Alkenyl steht im allgemeinen für einen geradkettigen oder verzweigten Kohlenwasserstoffrest mit 3 bis 12 Kohlenstoffatomen und mit einer oder mehreren, bevorzugt mit einer oder zwei Doppelbindung(en), der gegebenenfalls mit einem Halogenatom oder mehreren Halogenatomen - vorzugsweise Fluor - substituiert sein kann, die untereinander gleich oder verschieden sein können. Bevorzugt ist ein Niederalkenylrest mit 3 bis etwa 6 Kohlenstoffatomen und einer oder zwei Doppelbindung(en). Besonders bevorzugt ist ein Alkenylrest mit 3 oder 4 Kohlenstoffatomen und einer Doppelbindung. Als Beispiele seien Allyl, Propenyl, Isopropenyl, Pentenyl, Isopentenyl, Hexenyl, Isohexenyl, Heptenyl, Isoheptenyl, Octenyl und Isooctenyl genannt.

Alkinyl steht im allgemeinen für einen geradkettigen oder verzweigten Kohlenwasserstoffrest mit 3 bis 12 Kohlenstoffatomen und mit einer oder mehreren, bevorzugt mit einer oder zwei Dreifachbindung(en).

Bevorzugt ist ein Niederalkinylrest mit 3 bis etwa 6 Kohlenstoffatomen und einer oder zwei Dreifachbindung(en), der gegebenenfalls mit einem Halogenatom oder mehreren Halogenatomen substituiert sein kann, die untereinander gleich oder verschieden sein können. Besonders bevorzugt ist ein Alkinylrest mit 3 oder 4 Kohlenstoffatomen und einer Dreifachbindung. Als Beispiele seien Propargyl und Butinyl(2) genannt.

Cycloalkyl steht im allgemeinen für einen gesättigten oder ungesättigten cyclischen Kohlenwasserstoffrest mit 3 bis 9 Kohlenstoffatomen, der gegebenenfalls mit einem Halogenatom oder mehreren Halogenatomen substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl und Cyclooctyl, Cyclooctenyl, Cyclooctadienyl, Cyclononinyl genannt.

Aralkyl steht im allgemeinen für einen über eine Alkylenkette gebundenen Arylrest mit 7 bis 14 Kohlenstoffatomen, wobei der Aromat mit einer oder mehreren Niederalkylgruppe(n), Alkoxygruppe(n), Alkoxycarbonylgruppe(n), Hydroxygruppe(n), Cyanogruppe(n), Nitrogruppe(n), Aminogruppe(n) und/oder einem oder mehreren Halogenatom(en) - untereinander gleich oder verschieden - substituiert sein kann. Bevorzugt werden Aralkylreste mit 1 bis 6 Kohlenstoffatomen im aliphatischen Teil und 6 bis 10 Kohlenstoffatomen im aromatischen Teil. Als bevorzugte Aralkylreste seien genannt: Benzyl, Naphthylmethyl, Phenethyl und Phenylpropyl.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxyrest mit 1 bis etwa 6 Kohlenstoffatomen. Besonders bevorzugt ist ein Alkoxyrest mit 1 bis 4 Kohlenstoffatomen. Als Beispiele seien Methoxy, Ethoxy, Propoxy, Isopropoxy, Butoxy, Isobutoxy, tert.-Butoxy, Pentoxy, Isopentoxy, Hexoxy, Isohexoxy, Heptoxy, Isoheptoxy, Octoxy oder Isooctoxy genannt.

Acyl steht im allgemeinen für Benzoyl oder für Alkylcarbonylreste - wie geradkettiges oder verzweigtes Niederalkyl mit 1 bis etwa 6 Kohlenstoffatomen, die über eine Carbonylgruppe gebunden sind, wobei der Alkylrest gegebenenfalls mit einem oder mehreren Halogenatom(en), die untereinander gleich oder verschieden sein können, substituiert sein kann. Bevorzugt sind Alkylreste mit bis zu 4 Kohlenstoffatomen. Als Beispiele seien genannt: Acetyl, Trifluoracetyl, Ethylcarbonyl, Propylcarbonyl, Isopropylcarbonyl, Butylcarbonyl sowie Isobutylcarbonyl.

Alkoxycarbonyl kann beispielsweise durch die Formel

- C -O-Alkyl
  ||
  O

dargestellt werden.

Alkyl steht hierbei für einen geradkettigen oder verzweigten Kohlenwasserstoffrest mit 1 bis 12

Kohlenstoffatomen. Bevorzugt wird ein Niederalkoxycarbonylrest mit 1 bis 6 Kohlenstoffatomen.

Besonders bevorzugt wird ein Alkoxycarbonylrest mit 1 bis 4 Kohlenstoffatomen im Alkylrest. Als Beispiele seien folgende Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Heteroaryl im Rahmen der oben angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind. Als besondere Heteroarylreste seien beispielsweise Thienyl, Furyl, Pyridyl, Pyrimidyl, Pyrazinyl, Pyridazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Halogen bedeutet - sofern nicht anders angegeben -Fluor, Chlor, Brom und in zweiter Linie Jod.

## 2. Pharmakologische Eigenschaften

Die neuen Verbindungen der allgemeinen Formel 1 bzw. ihre pharmakologisch verträglichen Säureadditionssalze verkörpern zentral angreifende Dopamin-Agonisten. In Abhängigkeit von ihren individuellen chemischen Strukturen zeigen sie unterschiedliche Selektivitäten für morphologisch und/oder funktional differenzierte Dopamin-Rezeptoren und daraus resultierende verschiedene Wirkungsprofile. Sie können daher bei verschiedenen Erkrankungen, die durch Störungen dopaminerger Systeme verursacht werden, therapeutisch genutzt werden, z.B. bei Schizophrenie, Morbus Parkinson, Prolactin-Überfunktion und Bluthochdruck.

Als wertvoll haben sich dabei Verbindungen bzw. deren Hydrochloride der allgemeinen Formel 1, bei denen $R^1$ und $R^2$ unabhängig voneinander Wasserstoff (mit Ausnahme von $R^2$), Methyl, Ethyl, Propyl, Phenyl, Benzyl, Phenethyl, Phenylpropyl oder einen über eine Alkylkette mit 1 bis 5 Kohlenstoffatomen gebundenen Heteroarylrest bedeutet, wobei Furan, Thiophen, Pyridin und Indol als Heterocyclen bevorzugt werden und $R^3$ Wasserstoff, Methyl oder Acetyl und $R^4$ Wasserstoff bedeutet.

(+)-5-Amino-2-methylamino-tetralin
(-)-5-Amino-2-methylamino-tetralin
(+)-5-Amino-2-ethylamino-tetralin
(-)-5-Amino-2-ethylamino-tetralin
(+)-5-Amino-2-n-propylamino-tetralin
(-)-5-Amino-2-n-propylamino-tetralin
(+)-5-Amino-2-isopropylamino-tetralin
(-)-5-Amino-2-isopropylamino-tetralin
(+)-5-Amino-2-n-butylamino-tetralin
(-)-5-Amino-2-n-butylamino-tetralin
(+)-5-Amino-2-(2-methylpropyl)amino-tetralin
(-)-5-Amino-2-(2-methylpropyl)amino-tetralin
(+)-5-Amino-2-tert.-butylamino-tetralin
(-)-5-Amino-2-tert.-butylamino-tetralin
(+)-5-Amino-2-benzylamino-tetralin
(-)-5-Amino-2-benzylamino-tetralin
(+)-5-Amino-2-[(2-phenyl-ethyl)-amino]-tetralin
(-)-5-Amino-2-[(2-phenyl-ethyl)-amino]-tetralin
(+)-5-Amino-2-[(3-phenyl-propyl)-amino]-tetralin
(-)-5-Amino-2-[(3-phenyl-propyl)-amino]-tetralin
(+)-5-Amino-2-[(4-phenyl-butyl)-amino]-tetralin
(-)-5-Amino-2-[(4-phenyl-butyl)-amino]-tetralin
(+)-5-Amino-2-[(5-phenyl-pentyl)-amino]-tetralin
(-)-5-Amino-2-[(5-phenyl-pentyl)-amino]-tetralin
(+)-5-Amino-2-[(2-furyl)-methyl-amino]-tetralin
(-)-5-Amino-2-[(2-furyl)-methyl-amino]-tetralin
(+)-5-Amino-2-[(3-furyl)-methyl-amino]-tetralin
(-)-5-Amino-2-[(3-furyl)-methyl-amino]-tetralin
(+)-5-Amino-2-[2-(2-furyl)-ethyl-amino]-tetralin
(-)-5-Amino-2-[2-(2-furyl)-ethyl-amino]-tetralin

(+)-5-Amino-2-[2-(3-furyl)-ethyl-amino]-tetralin
(-)-5-Amino-2-[2-(3-furyl)-ethyl-amino]-tetralin
(+)-5-Amino-2-[3-(2-furyl)-propyl-amino]-tetralin
(-)-5-Amino-2-[3-(2-furyl)-propyl-amino]-tetralin
(+)-5-Amino-2-[3-(3-furyl)-propyl-amino]-tetralin
(-)-5-Amino-2-[3-(3-furyl)-propyl-amino]-tetralin
(+)-5-Amino-2-[4-(2-furyl)-butyl-amino]-tetralin
(-)-5-Amino-2-[4-(2-furyl)-butyl-amino]-tetralin
(+)-5-Amino-2-[4-(3-furyl)-butyl-amino]-tetralin
(-)-5-Amino-2-[4-(3-furyl)-butyl-amino]-tetralin
(+)-5-Amino-2-[5-(2-furyl)-pentyl-amino]-tetralin
(-)-5-Amino-2-[5-(2-furyl)-pentyl-amino]-tetralin
(+)-5-Amino-2-[5-(3-furyl)-pentyl-amino]-tetralin
(-)-5-Amino-2-[5-(3-furyl)-pentyl-amino]-tetralin
(+)-5-Amino-2-[(2-thienyl)-methyl-amino]-tetralin
(-)-5-Amino-2-[(2-thienyl)-methyl-amino]-tetralin
(+)-5-Amino-2-[(3-thienyl)-methyl-amino]-tetralin
(-)-5-Amino-2-[(3-thienyl)-methyl-amino]-tetralin
(+)-5-Amino-2-[2-(2-thienyl)-ethyl-amino]-tetralin
(-)-5-Amino-2-[2-(2-thienyl)-ethyl-amino]-tetralin
(+)-5-Amino-2-[2-(3-thienyl)-ethyl-amino]-tetralin
(-)-5-Amino-2-[2-(3-thienyl)-ethyl-amino]-tetralin
(+)-5-Amino-2-[3-(2-thienyl)-propyl-amino]-tetralin
(-)-5-Amino-2-[3-(2-thienyl)-propyl-amino]-tetralin
(+)-5-Amino-2-[3-(3-thienyl)-propyl-amino]-tetralin
(-)-5-Amino-2-[3-(3-thienyl)-propyl-amino]-tetralin
(+)-5-Amino-2-[4-(2-thienyl)-butyl-amino]-tetralin
(-)-5-Amino-2-[4-(2-thienyl)-butyl-amino]-tetralin
(+)-5-Amino-2-[4-(3-thienyl)-butyl-amino]-tetralin
(-)-5-Amino-2-[4-(3-thienyl)-butyl-amino]-tetralin
(+)-5-Amino-2-[5-(2-thienyl)-pentyl-amino]-tetralin
(-)-5-Amino-2-[5-(2-thienyl)-pentyl-amino]-tetralin
(+)-5-Amino-2-[5-(3-thienyl)-pentyl-amino]-tetralin
(-)-5-Amino-2-[5-(3-thienyl)-pentyl-amino]-tetralin
(+)-5-Amino-2-[(2-pyridyl)-methyl-amino]-tetralin
(-)-5-Amino-2-[(2-pyridyl)-methyl-amino]-tetralin
(+)-5-Amino-2-[(3-pyridyl)-methyl-amino]-tetralin
(-)-5-Amino-2-[(3-pyridyl)-methyl-amino]-tetralin
(+)-5-Amino-2-[(4-pyridyl)-methyl-amino]-tetralin
(-)-5-Amino-2-[(4-pyridyl)-methyl-amino]-tetralin
(+)-5-Amino-2-[2-(2-pyridyl)-ethyl-amino]-tetralin
(-)-5-Amino-2-[2-(2-pyridyl)-ethyl-amino]-tetralin
(+)-5-Amino-2-[2-(3-pyridyl)-ethyl-amino]-tetralin
(-)-5-Amino-2-[2-(3-pyridyl)-ethyl-amino]-tetralin
(+)-5-Amino-2-[2-(4-pyridyl)-ethyl-amino]-tetralin
(-)-5-Amino-2-[2-(4-pyridyl)-ethyl-amino]-tetralin
(+)-5-Amino-2-[3-(2-pyridyl)-propyl-amino]-tetralin
(-)-5-Amino-2-[3-(2-pyridyl)-propyl-amino]-tetralin
(+)-5-Amino-2-[3-(3-pyridyl)-propyl-amino]-tetralin
(-)-5-Amino-2-[3-(3-pyridyl)-propyl-amino]-tetralin
(+)-5-Amino-2-[3-(4-pyridyl)-propyl-amino]-tetralin
(-)-5-Amino-2-[3-(4-pyridyl)-propyl-amino]-tetralin
(+)-5-Amino-2-[4-(2-pyridyl)-butyl-amino]-tetralin
(-)-5-Amino-2-[4-(2-pyridyl)-butyl-amino]-tetralin
(+)-5-Amino-2-[4-(3-pyridyl)-butyl-amino]-tetralin
(-)-5-Amino-2-[4-(3-pyridyl)-butyl-amino]-tetralin
(+)-5-Amino-2-[4-(4-pyridyl)-butyl-amino]-tetralin
(-)-5-Amino-2-[4-(4-pyridyl)-butyl-amino]-tetralin

(+)-5-Amino-2-[5-(2-pyridyl)-pentyl-amino]-tetralin
(-)-5-Amino-2-[5-(2-pyridyl)-pentyl-amino]-tetralin
(+)-5-Amino-2-[5-(3-pyridyl)-pentyl-amino]-tetralin
(-)-5-Amino-2-[5-(3-pyridyl)-pentyl-amino]-tetralin
(+)-5-Amino-2-[5-(4-pyridyl)-pentyl-amino]-tetralin
(-)-5-Amino-2-[5-(4-pyridyl)-pentyl-amino]-tetralin
(+)-5-Amino-2-[(2-indolyl)-methyl-amino]-tetralin
(-)-5-Amino-2-[(2-indolyl)-methyl-amino]-tetralin
(+)-5-Amino-2-[(3-indolyl)-methyl-amino]-tetralin
(-)-5-Amino-2-[(3-indolyl)-methyl-amino]-tetralin
(+)-5-Amino-2-[2-(2-indolyl)-ethyl-amino]-tetralin
(-)-5-Amino-2-[2-(2-indolyl)-ethyl-amino]-tetralin
(+)-5-Amino-2-[2-(3-indolyl)-ethyl-amino]-tetralin
(-)-5-Amino-2-[2-(3-indolyl)-ethyl-amino]-tetralin
(+)-5-Amino-2-[3-(2-indolyl)-propyl-amino]-tetralin
(-)-5-Amino-2-[3-(2-indolyl)-propyl-amino]-tetralin
(+)-5-Amino-2-[3-(3-indolyl)-propyl-amino]-tetralin
(-)-5-Amino-2-[3-(3-indolyl)-propyl-amino]-tetralin
(+)-5-Amino-2-[4-(2-indolyl)-butyl-amino]-tetralin
(-)-5-Amino-2-[4-(2-indolyl)-butyl-amino]-tetralin
(+)-5-Amino-2-[4-(3-indolyl)-butyl-amino]-tetralin
(-)-5-Amino-2-[4-(3-indolyl)-butyl-amino]-tetralin
(+)-5-Amino-2-[5-(2-indolyl)-pentyl-amino]-tetralin
(-)-5-Amino-2-[5-(2-indolyl)-pentyl-amino]-tetralin
(+)-5-Amino-2-[5-(3-indolyl)-pentyl-amino]-tetralin
(-)-5-Amino-2-[5-(3-indolyl)-pentyl-amino]-tetralin
(+)-5-Amino-2-dimethylamino-tetralin
(-)-5-Amino-2-dimethylamino-tetralin
(+)-5-Amino-2-ethylmethyl-amino-tetralin
(-)-5-Amino-2-ethylmethyl-amino-tetralin
(+)-5-Amino-2-methyl-n-propyl-amino-tetralin
(-)-5-Amino-2-methyl-n-propyl-amino-tetralin
(+)-5-Amino-2-isopropylmethyl-amino-tetralin
(-)-5-Amino-2-isopropylmethyl-amino-tetralin
(+)-5-Amino-2-n-butylmethyl-amino-tetralin
(-)-5-Amino-2-n-butylmethyl-amino-tetralin
(+)-5-Amino-2-[N-methyl-N-(2-methylpropyl)-amino]-tetralin
(-)-5-Amino-2-[N-methyl-N-(2-methylpropyl)-amino]-tetralin
(+)-5-Amino-2-(N-tert.-butyl-N-methyl-amino)-tetralin
(-)-5-Amino-2-(N-tert.-butyl-N-methyl-amino)-tetralin
(+)-5-Amino-2-(N-benzyl-N-methyl-amino)-tetralin
(-)-5-Amino-2-(N-benzyl-N-methyl-amino)-tetralin
(+)-5-Amino-2-[N-methyl-N-(2-phenyl-ethyl)-amino]-tetralin
(-)-5-Amino-2-[N-methyl-N-(2-phenyl-ethyl)-amino]-tetralin
(+)-5-Amino-2-[N-methyl-N-(3-phenyl-propyl)-amino]-tetralin
(-)-5-Amino-2-[N-methyl-N-(3-phenyl-propyl)-amino]-tetralin
(+)-5-Amino-2-[N-methyl-N-(4-phenyl-butyl)-amino]-tetralin
(-)-5-Amino-2-[N-methyl-N-(4-phenyl-butyl)-amino]-tetralin
(+)-5-Amino-2-[N-methyl-N-(5-phenyl-pentyl)-N-amino]-tetralin
(-)-5-Amino-2-[N-methyl-N-(5-phenyl-pentyl)-N-amino]-tetralin
(+)-5-Amino-2-{N-[(2-furyl)-methyl]-N-methyl-amino}-tetralin
(-)-5-Amino-2-{N-[(2-furyl)-methyl]-N-methyl-amino}-tetralin
(+)-5-Amino-2-{N-[(3-furyl)-methyl]-N-methyl-amino}-tetralin
(-)-5-Amino-2-{N-[(3-furyl)-methyl]-N-methyl-amino}-tetralin
(+)-5-Amino-2-{N-[2-(2-furyl)-ethyl]-N-methyl-amino}-tetralin
(-)-5-Amino-2-{N-[2-(2-furyl)-ethyl]-N-methyl-amino}-tetralin
(+)-5-Amino-2-{N-[2-(3-furyl)-ethyl]-N-methyl-amino}-tetralin
(-)-5-Amino-2-{N-[2-(3-furyl)-ethyl]-N-methyl-amino}-tetralin

(+)-5-Amino-2-{N-[3-(2-furyl)-propyl]-N-methyl-amino}-tetralin
(-)-5-Amino-2-{N-[3-(2-furyl)-propyl]-N-methyl-amino}-tetralin
(+)-5-Amino-2-{N-[3-(3-furyl)-propyl]-N-methyl-amino}-tetralin
(-)-5-Amino-2-{N-[3-(3-furyl)-propyl]-N-methyl-amono}-tetralin
(+)-5-Amino-2-{N-[4-(2-furyl)-butyl]-N-methyl-amino}-tetralin
(-)-5-Amino-2-{N-[4-(2-furyl)-butyl]-N-methyl-amino}-tetralin
(+)-5-Amino-2-{N-[4-(3-furyl)-butyl]-N-methyl-amino}-tetralin
(-)-5-Amino-2-{N-[4-(3-furyl)-butyl]-N-methyl-amino}-tetralin
(+)-5-Amino-2-{N-[5-(2-furyl)-pentyl]-N-methyl-amino}-tetralin
(-)-5-Amino-2-{N-[5-(2-furyl)-pentyl]-N-methyl-amino}-tetralin
(+)-5-Amino-2-{N-[5-(3-furyl)-pentyl]-N-methyl-amino}-tetralin
(-)-5-Amino-2-{N-[5-(3-furyl)-pentyl]-N-methyl-amino}-tetralin
(+)-5-Amino-2-{N-methyl-N-[(2-thienyl)-methyl]-amino}-tetralin
(-)-5-Amino-2-{N-methyl-N-[(2-thienyl)-methyl]-amino}-tetralin
(+)-5-Amino-2-{N-methyl-N-[(3-thienyl)-methyl]-amino}-tetralin
(-)-5-Amino-2-{N-methyl-N-[(3-thienyl)-methyl]-amino}-tetralin
(+)-5-Amino-2-{N-methyl-N-[2-(2-thienyl)-ethyl]-amino}-tetralin
(-)-5-Amino-2-{N-methyl-N-[2-(2-thienyl)-ethyl]-amino}-tetralin
(+)-5-Amino-2-{N-methyl-N-[2-(3-thienyl)-ethyl]-amino}-tetralin
(-)-5-Amino-2-{N-methyl-N-[2-(3-thienyl)-ethyl]-amino}-tetralin
(+)-5-Amino-2-{N-methyl-N-[3-(2-thienyl)-propyl]-amino}-tetralin
(-)-5-Amino-2-{N-methyl-N-[3-(2-thienyl)-propyl]-amino}-tetralin
(+)-5-Amino-2-{N-methyl-N-[3-(3-thienyl)-propyl]-amino}-tetralin
(-)-5-Amino-2-{N-methyl-N-[3-(3-thienyl)-propyl]-amino}-tetralin
(+)-5-Amino-2-{N-methyl-N-[4-(2-thienyl)-butyl]-amino}-tetralin
(-)-5-Amino-2-{N-methyl-N-[4-(2-thienyl)-butyl]-amino}-tetralin
(+)-5-Amino-2-{N-methyl-N-[4-(3-thienyl)-butyl]-amino}-tetralin
(-)-5-Amino-2-{N-methyl-N-[4-(3-thienyl)-butyl]-amino}-tetralin
(+)-5-Amino-2-{N-methyl-N-[5-(2-thienyl)-pentyl]-amino}-tetralin
(-)-5-Amino-2-{N-methyl-N-[5-(2-thienyl)-pentyl]-amino}-tetralin
(+)-5-Amino-2-{N-methyl-N-[5-(3-thienyl)-pentyl]-amino}-tetralin
(-)-5-Amino-2-{N-methyl-N-[5-(3-thienyl)-pentyl]-amino}-tetralin
(+)-5-Amino-2-{N-methyl-N-[(2-pyridyl)-methyl]-amino}-tetralin
(-)-5-Amino-2-{N-methyl-N-[(2-pyridyl)-methyl]-amino}-tetralin
(+)-5-Amino-2-{N-methyl-N-[(3-pyridyl)-methyl]-amino}-tetralin
(-)-5-Amino-2-{N-methyl-N-[(3-pyridyl)-methyl]-amino}-tetralin
(+)-5-Amino-2-{N-methyl-N-[(4-pyridyl)-methyl]-amino}-tetralin
(-)-5-Amino-2-{N-methyl-N-[(4-pyridyl)-methyl]-amino}-tetralin
(+)-5-Amino-2-{N-methyl-N-[2-(2-pyridyl)-ethyl]-amino}-tetralin
(-)-5-Amino-2-{N-methyl-N-[2-(2-pyridyl)-ethyl]-amino}-tetralin
(+)-5-Amino-2-{N-methyl-N-[2-(3-pyridyl)-ethyl]-amino}-tetralin
(-)-5-Amino-2-{N-methyl-N-[2-(3-pyridyl)-ethyl]-amino}-tetralin
(+)-5-Amino-2-{N-methyl-N-[2-(4-pyridyl)-ethyl]-amino}-tetralin
(-)-5-Amino-2-{N-methyl-N-[2-(4-pyridyl)-ethyl]-amino}-tetralin
(+)-5-Amino-2-{N-methyl-N-[3-(2-pyridyl)-propyl]-amino}-tetralin
(-)-5-Amino-2-{N-methyl-N-[3-(2-pyridyl)-propyl]-amino}-tetralin
(+)-5-Amino-2-{N-methyl-N-[3-(3-pyridyl)-propyl]-amino}-tetralin
(-)-5-Amino-2-{N-methyl-N-[3-(3-pyridyl)-propyl]-amino}-tetralin
(+)-5-Amino-2-{N-methyl-N-[3-(4-pyridyl)-propyl]-amino}-tetralin
(-)-5-Amino-2-{N-methyl-N-[3-(4-pyridyl)-propyl]-amino}-tetralin
(+)-5-Amino-2-{N-methyl-N-[4-(2-pyridyl)-butyl]-amino}-tetralin
(-)-5-Amino-2-{N-methyl-N-[4-(2-pyridyl)-butyl]-amino}-tetralin
(+)-5-Amino-2-{N-methyl-N-[4-(3-pyridyl)-butyl]-amino}-tetralin
(-)-5-Amino-2-{N-methyl-N-[4-(3-pyridyl)-butyl]-amino}-tetralin
(+)-5-Amino-2-{N-methyl-N-[4-(4-pyridyl)-butyl]-amino}-tetralin
(-)-5-Amino-2-{N-methyl-N-[4-(4-pyridyl)-butyl]-amino}-tetralin
(+)-5-Amino-2-{N-methyl-N-[5-(2-pyridyl)-pentyl]-amino}-tetralin
(-)-5-Amino-2-{N-methyl-N-[5-(2-pyridyl)-pentyl]-amino}-tetralin

(+)-5-Amino-2-{N-methyl-N-[5-(3-pyridyl)-pentyl]-amino}-tetralin
(-)-5-Amino-2-{N-methyl-N-[5-(3-pyridyl)-pentyl]-amino}-tetralin
(+)-5-Amino-2-{N-methyl-N-[5-(4-pyridyl)-pentyl]-amino}-tetralin
(-)-5-Amino-2-{N-methyl-N-[5-(4-pyridyl)-pentyl]-amino}-tetralin
(+)-5-Amino-2-{N-[(2-indolyl)-methyl]-N-methyl-amino}-tetralin
(-)-5-Amino-2-{N-[(2-indolyl)-methyl]-N-methyl-amino}-tetralin
(+)-5-Amino-2-{N-[(3-indolyl)-methyl]-N-methyl-amino}-tetralin
(-)-5-Amino-2-{N-[(3-indolyl)-methyl]-N-methyl-amino}-tetralin
(+)-5-Amino-2-{N-[2-(2-indolyl)-ethyl]-N-methyl-amino}-tetralin
(-)-5-Amino-2-{N-[2-(2-indolyl)-ethyl]-N-methyl-amino}-tetralin
(+)-5-Amino-2-{N-[2-(3-indolyl)-ethyl]-N-methyl-amino}-tetralin
(-)-5-Amino-2-{N-[2-(3-indolyl)-ethyl]-N-methyl-amino}-tetralin
(+)-5-Amino-2-{N-[3-(2-indolyl)-propyl]-N-methyl-amino}-tetralin
(-)-5-Amino-2-{N-[3-(2-indolyl)-propyl]-N-methyl-amino}-tetralin
(+)-5-Amino-2-{N-[3-(3-indolyl)-propyl]-N-methyl-amino}-tetralin
(-)-5-Amino-2-{N-[3-(3-indolyl)-propyl]-N-methyl-amino}-tetralin
(+)-5-Amino-2-{N-[4-(2-indolyl)-butyl]-N-methyl-amino}-tetralin
(-)-5-Amino-2-{N-[4-(2-indolyl)-butyl]-N-methyl-amino}-tetralin
(+)-5-Amino-2-{N-[4-(3-indolyl)-butyl]-N-methyl-amino}-tetralin
(-)-5-Amino-2-{N-[4-(3-indolyl)-butyl]-N-methyl-amino}-tetralin
(+)-5-Amino-2-{N-[5-(2-indolyl)-pentyl]-N-methyl-amino}-tetralin
(-)-5-Amino-2-{N-[5-(2-indolyl)-pentyl]-N-methyl-amino}-tetralin
(+)-5-Amino-2-{N-[5-(3-indolyl)-pentyl]-N-methyl-amino}-tetralin
(-)-5-Amino-2-{N-[5-(3-indolyl)-pentyl]-N-methyl-amino}-tetralin
(+)-5-Amino-2-ethylmethylamino-tetralin
(-)-5-Amino-2-ethylmethylamino-tetralin
(+)-5-Amino-2-diethyl-amino-tetralin
(-)-5-Amino-2-diethyl-amino-tetralin
(+)-5-Amino-2-ethyl-n-propyl-amino-tetralin
(-)-5-Amino-2-ethyl-n-propyl-amino-tetralin
(+)-5-Amino-2-ethylisopropyl-amino-tetralin
(-)-5-Amino-2-ethylisopropyl-amino-tetralin
(+)-5-Amino-2-n-butylethyl-amino-tetralin
(-)-5-Amino-2-n-butylethyl-amino-tetralin
(+)-5-Amino-2-[N-ethyl-N-(2-methylpropyl)-amino]-tetralin
(-)-5-Amino-2-[N-ethyl-N-(2-methylpropyl)-amino]-tetralin
(+)-5-Amino-2-(N-tert.-butyl-N-ethyl-amino)-tetralin
(-)-5-Amino-2-(N-tert.-butyl-N-ethyl-amino)-tetralin
(+)-5-Amino-2-(N-benzyl-N-ethyl-amino)-tetralin
(-)-5-Amino-2-(N-benzyl-N-ethyl-amino)-tetralin
(+)-5-Amino-2-[N-ethyl-N-(2-phenyl-ethyl)-amino]-tetralin
(-)-5-Amino-2-[N-ethyl-N-(2-phenyl-ethyl)-amino]-tetralin
(+)-5-Amino-2-[N-ethyl-N-(3-phenyl-propyl)-amino]-tetralin
(-)-5-Amino-2-[N-ethyl-N-(3-phenyl-propyl)-amino]-tetralin
(+)-5-Amino-2-[N-ethyl-N-(4-phenyl-butyl)-amino]-tetralin
(-)-5-Amino-2-[N-ethyl-N-(4-phenyl-butyl)-amino]-tetralin
(+)-5-Amino-2-[N-ethyl-N-(5-phenyl-pentyl)-N-amino]-tetralin
(-)-5-Amino-2-[N-ethyl-N-(5-phenyl-pentyl)-N-amino]-tetralin
(+)-5-Amino-2-[N-ethyl-N-[(2-furyl)-methyl]-amino]-tetralin
(-)-5-Amino-2-[N-ethyl-N-[(2-furyl)-methyl]-amino]-tetralin
(+)-5-Amino-2-[N-ethyl-N-[(3-furyl)-methyl]-amino]-tetralin
(-)-5-Amino-2-{N-ethyl-N-[(3-furyl)-methyl]-amino}-tetralin
(+)-5-Amino-2-{N-ethyl-N-[2-(2-furyl)-ethyl]-amino}-tetralin
(-)-5-Amino-2-{N-ethyl-N-[2-(2-furyl)-ethyl]-amino}-tetralin
(+)-5-Amino-2-{N-ethyl-N-[2-(3-furyl)-ethyl]-amino}-tetralin
(-)-5-Amino-2-{N-ethyl-N-[2-(3-furyl)-ethyl]-amino}-tetralin
(+)-5-Amino-2-{N-ethyl-N-[3-(2-furyl)-propyl]-amino}-tetralin
(-)-5-Amino-2-{N-ethyl-N-[3-(2-furyl)-propyl]-amino}-tetralin

( + )-5-Amino-2-{N-ethyl-N-[3-(3-furyl)-propyl]-amino}-tetralin
(-)-5-Amino-2-{N-ethyl-N-[3-(3-furyl)-propyl]-amino}-tetralin
( + )-5-Amino-2-{N-ethyl-N-[4-(2-furyl)-butyl]-amino}-tetralin
(-)-5-Amino-2-{N-ethyl-N-[4-(2-furyl)-butyl]-amino}-tetralin
( + )-5-Amino-2-{N-ethyl-N-[4-(3-furyl)-butyl]-amino}-tetralin
(-)-5-Amino-2-{N-ethyl-N-[4-(3-furyl)-butyl]-amino}-tetralin
( + )-5-Amino-2-{N-ethyl-N-[5-(2-furyl)-pentyl]-amino}-tetralin
(-)-5-Amino-2-{N-ethyl-N-[5-(2-furyl)-pentyl]-amino}-tetralin
( + )-5-Amino-2-{N-ethyl-N-[5-(3-furyl)-pentyl]-amino}-tetralin
(-)-5-Amino-2-{N-ethyl-N-[5-(3-furyl)-pentyl]-amino}-tetralin
( + )-5-amino-2-{N-ethyl-N-[(2-thienyl)-methyl]-amino}-tetralin
(-)-5-Amino-2-{N-ethyl-N-[(2-thienyl)-methyl]-amino}-tetralin
( + )-5-Amino-2-{N-ethyl-N-[(3-thienyl)-methyl]-amino}-tetralin
(-)-5-Amino-2-{N-ethyl-N-[(3-thienyl)-methyl]-amino}-tetralin
( + )-5-Amino-2-{N-ethyl-N-[2-(2-thienyl)-ethyl]-amino}-tetralin
(-)-5-Amino-2-{N-ethyl-N-[2-(2-thienyl)-ethyl]-amino}-tetralin
( + )-5-Amino-2-{N-ethyl-N-[2-(3-thienyl)-ethyl]-amino}-tetralin
(-)-5-Amino-2-{N-ethyl-N-[2-(3-thienyl)-ethyl]-amino}-tetralin
( + )-5-Amino-2-{N-ethyl-N-[3-(2-thienyl)-propyl]-amino}-tetralin
(-)-5-Amino-2-{N-ethyl-N-[3-(2-thienyl)-propyl]-amino}-tetralin
( + )-5-Amino-2-{N-ethyl-N-[3-(3-thienyl)-propyl]-amino}-tetralin
(-)-5-Amino-2-{N-ethyl-N-[3-(3-thienyl)-propyl]-amino}-tetralin
( + )-5-Amino-2-{N-ethyl-N-[4-(2-thienyl)-butyl]-amino}-tetralin
(-)-5-Amino-2-{N-ethyl-N-[4-(2-thienyl)-butyl]-amino}-tetralin
( + )-5-Amino-2-{N-ethyl-N-[4-(3-thienyl)-butyl]-amino}-tetralin
(-)-5-Amino-2-{N-ethyl-N-[4-(3-thienyl)-butyl]-amino}-tetralin
( + )-5-Amino-2-{N-ethyl-N-[5-(2-thienyl)-pentyl]-amino}-tetralin
(-)-5-Amino-2-{N-ethyl-N-[5-(2-thienyl)-pentyl]-amino}-tetralin
( + )-5-Amino-2-{N-ethyl-N-[5-(3-thienyl)-pentyl]-amino}-tetralin
(-)-5-Amino-2-{N-ethyl-N-[5-(3-thienyl)-pentyl]-amino}-tetralin
( + )-5-Amino-2-{N-ethyl-N-[(2-pyridyl)-methyl]-amino}-tetralin
(-)-5-Amino-2-{N-ethyl-N-[(2-pyridyl)-methyl]-amino}-tetralin
( + )-5-Amino-2-{N-ethyl-N-[(3-pyridyl)-methyl]-amino}-tetralin
(-)-5-Amino-2-{N-ethyl-N-[(3-pyridyl)-methyl]-amino}-tetralin
( + )-5-Amino-2-{N-ethyl-N-[(4-pyridyl)-methyl]-amino}-tetralin
(-)-5-Amino-2-{N-ethyl-N-[(4-pyridyl)-methyl]-amino}-tetralin
( + )-5-Amino-2-{N-ethyl-N-[2-(2-pyridyl)-ethyl]-amino}-tetralin
(-)-5-Amino-2-{N-ethyl-N-[2-(2-pyridyl)-ethyl]-amino}-tetralin
( + )-5-Amino-2-{N-ethyl-N-[2-(3-pyridyl)-ethyl]-amino}-tetralin
(-)-5-Amino-2-{N-ethyl-N-[2-(3-pyridyl)-ethyl]-amino}-tetralin
( + )-5-Amino-2-{N-ethyl-N-[2-(4-pyridyl)-ethyl]-amino}-tetralin
(-)-5-Amino-2-{N-ethyl-N-[2-(4-pyridyl)-ethyl]-amino}-tetralin
( + )-5-Amino-2-{N-ethyl-N-[3-(2-pyridyl)-propyl]-amino}-tetralin
(-)-5-Amino-2-{N-ethyl-N-[3-(2-pyridyl)-propyl]-amino}-tetralin
( + )-5-Amino-2-{N-ethyl-N-[3-(3-pyridyl)-propyl]-amino}-tetralin
(-)-5-Amino-2-{N-ethyl-N-[3-(3-pyridyl)-propyl]-amino}-tetralin
( + )-5-Amino-2-{N-ethyl-N-[3-(4-pyridyl)-propyl]-amino}-tetralin
(-)-5-Amino-2-{N-ethyl-N-[3-(4-pyridyl)-propyl]-amino}-tetralin
( + )-5-Amino-2-{N-ethyl-N-[4-(2-pyridyl)-butyl]-amino}-tetralin
(-)-5-Amino-2-{N-ethyl-N-[4-(2-pyridyl)-butyl]-amino}-tetralin
( + )-5-Amino-2-{N-ethyl-N-[4-(3-pyridyl)-butyl]-amino}-tetralin
(-)-5-Amino-2-{N-ethyl-N-[4-(3-pyridyl)-butyl]-amino}-tetralin
( + )-5-Amino-2-{N-ethyl-N-[4-(4-pyridyl)-butyl]-amino}-tetralin
(-)-5-Amino-2-{N-ethyl-N-[4-(4-pyridyl)-butyl]-amino}-tetralin
( + )-5-Amino-2-{N-ethyl-N-[5-(2-pyridyl)-pentyl]-amino}-tetralin
(-)-5-Amino-2-{N-ethyl-N-[5-(2-pyridyl)-pentyl]-amino}-tetralin
( + )-5-Amino-2-{N-ethyl-N-[5-(3-pyridyl)-pentyl]-amino}-tetralin
(-)-5-Amino-2-{N-ethyl-N-[5-(3-pyridyl)-pentyl]-amino}-tetralin

(+)-5-Amino-2-{N-ethyl-N-[5-(4-pyridyl)-pentyl]-amino}-tetralin
(-)-5-Amino-2-{N-ethyl-N-[5-(4-pyridyl)-pentyl]-amino}-tetralin
(+)-5-Amino-2-{N-ethyl-N-[(2-indolyl)-methyl]-amino}-tetralin
(-)-5-Amino-2-{N-ethyl-N-[(2-indolyl)-methyl]-amino}-tetralin
(+)-5-Amino-2-{N-ethyl-N-[(3-indolyl)-methyl]-amino}-tetralin
(-)-5-Amino-2-{N-ethyl-N-[(3-indolyl)-methyl]-amino}-tetralin
(+)-5-Amino-2-{N-ethyl-N-[2-(2-indolyl)-ethyl]-amino}-tetralin
(-)-5-Amino-2-{N-ethyl-N-[2-(2-indolyl)-ethyl]-amino}-tetralin
(+)-5-Amino-2-{N-ethyl-N-[2-(3-indolyl)-ethyl]-amino}-tetralin
(-)-5-Amino-2-{N-ethyl-N-[2-(3-indolyl)-ethyl]-amino}-tetralin
(+)-5-Amino-2-{N-ethyl-N-[3-(2-indolyl)-propyl]-amino}-tetralin
(-)-5-Amino-2-{N-ethyl-N-[3-(2-indolyl)-propyl]-amino}-tetralin
(+)-5-Amino-2-{N-ethyl-N-[3-(3-indolyl)-propyl]-amino}-tetralin
(-)-5-Amino-2-{N-ethyl-N-[3-(3-indolyl)-propyl]-amino}-tetralin
(+)-5-Amino-2-{N-ethyl-N-[4-(2-indolyl)-butyl]-amino}-tetralin
(-)-5-Amino-2-{N-ethyl-N-[4-(2-indolyl)-butyl]-amino}-tetralin
(+)-5-Amino-2-{N-ethyl-N-[4-(3-indolyl)-butyl]-amino}-tetralin
(-)-5-Amino-2-{N-ethyl-N-[4-(3-indolyl)-butyl]-amino}-tetralin
(+)-5-Amino-2-{N-ethyl-N-[5-(2-indolyl)-pentyl]-amino}-tetralin
(-)-5-Amino-2-{N-ethyl-N-[5-(2-indolyl)-pentyl]-amino}-tetralin
(+)-5-Amino-2-{N-ethyl-N-[5-(3-indolyl)-pentyl]-amino}-tetralin
(-)-5-Amino-2-{N-ethyl-N-[5-(3-indolyl)-pentyl]-amino}-tetralin

Als besonders wertvoll haben sich folgende Verbindungen - als enantiomerenreine Substanzen wie auch als Mischungen oder Racemate bzw. deren pharmakologisch wirksamen Salze erwiesen:

A: (±)-5-Amino-2-(3-phenylpropyl-amino)-tetralin
B: (±)-5-Amino-2-[N-(3-phenylpropyl)-N-n-propyl-amino]tetralin.
C: (±)-5-Amino-2-(4-phenylbutyl-amino)-tetralin
D: (±)-5-Amino-2-[N-(4-phenylbutyl)-N-n-propyl-amino]tetralin

## 2.1 Bestimmung der Dopaminsynthesehemmung

Die Methodik wird gemäß J.R. Walters und R.H. Roth [Naunyn - Schmiedeberg's Arch. Pharmacol. 296, (1976) 5] durchgeführt: Hierzu erhalten 5 Tiere jeweils 10 mg/kg s.c. der zu untersuchenden Substanz. Nach 5 Minuten erfolgt die Gabe von 750 mg/kg i.p. γ-Butyrolacton, um über die Blockade der präsynaptischen Impulsleitung den Einfluß postsynaptischer Rückkopplungsschleifen auf die Dopaminsyntheserate auszuschließen. Die Gabe von γ-Butyrolacton führt zu einer beträchtlichen Steigerung der DOPA- bzw. Dopaminsynthese. Zur Hemmung der Decarboxylierung von DOPA werden nach weiteren 5 Minuten 200 mg/kg i.p. 3-Hydroxybenzyl-hydrazin-Hydrochlorid appliziert. 40 Minuten nach Substanzgabe werden die Tiere getötet und das Corpus striatum präpariert. Die Messung des DOPA-Gehaltes erfolgt mit Hilfe von HPLC mit elektrochemischer Detektion (Standard: Dihydroxybenzylamin).

Bestimmt wird die durch die Testsubstanz bewirkte prozentuale Hemmung der durch γ-Butyrolacton stimulierten Dopaakkumulierung gegenüber den mit 0,9 proz. Kochsalzlösung behandelten Kontrolltieren.

Die Ergebnisse dieses Versuches sind in nachfolgender Tabelle zusammengestellt:

Tabelle

| Substanz | Dosis (mg/kg s.c.) | Hemmung der Dopaakkumulierung in % gegenüber den mit Kochsalz behandelten Kontrolltieren |
|---|---|---|
| A | 10 | 45,5 |
| B | 10 | 63,5 |

## 3. Herstellungsverfahren

Die Verbindungen der allgemeinen Formel 1 können nach verschiedenen, an sich bekannten Verfahren hergestellt werden. Bevorzugt werden die im Reaktionsschema (Seite 25) aufgeführten Verfahren. Schlüsselverbindungen sind dabei 5-Amino-2-tetralone (2), die z.B. analog der

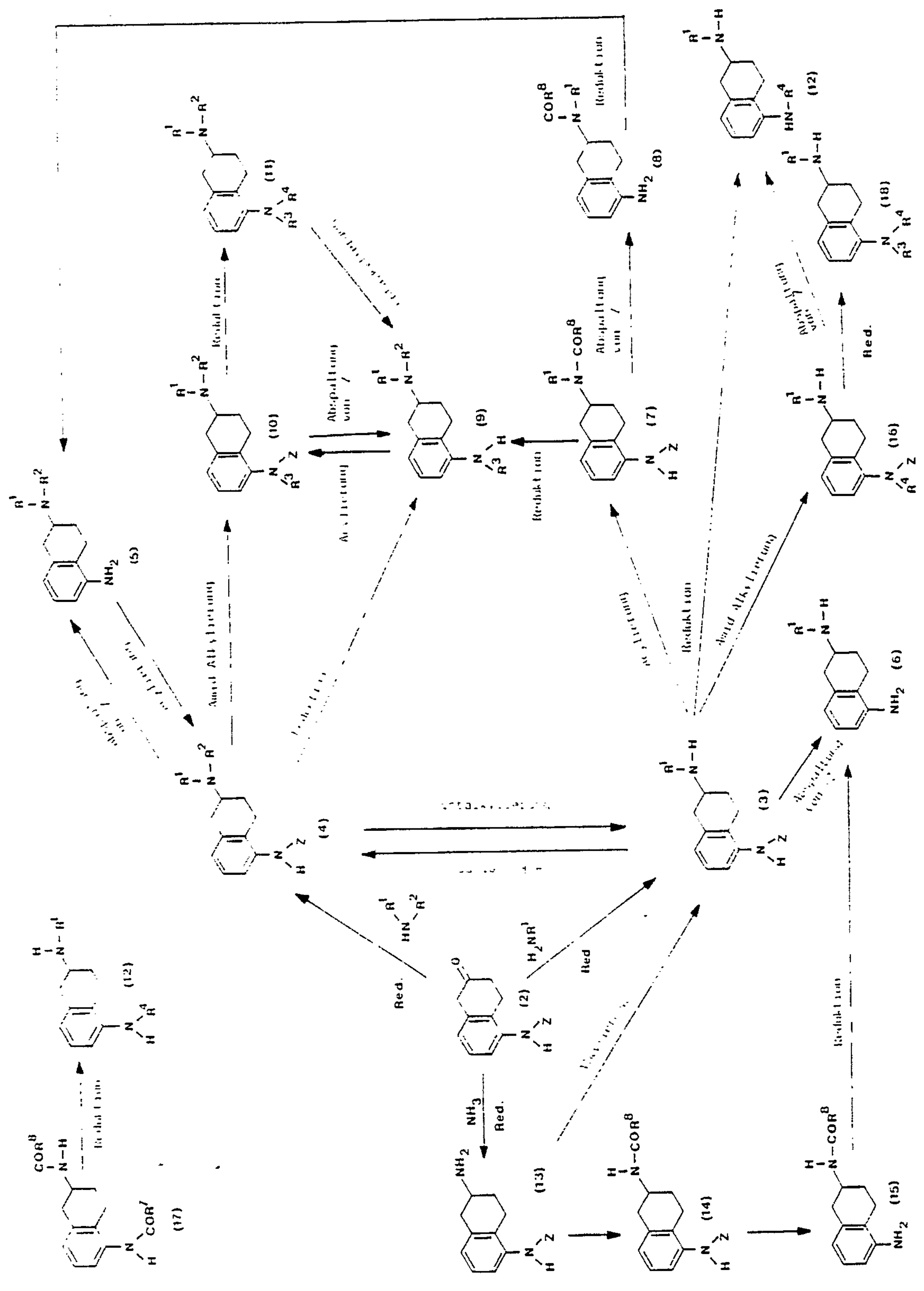

5-Acetylamino-Verbindung ((2), -Z = $-COCH_3$) hergestellt werden können (J.W. Cornforth et al., J. Chem. Soc. 1955, 3348). Dabei ist -Z ein Säurerest, der erhalten bleiben kann, wenn er die in der allgemeinen Formel 1 für $-R^3$ angegebene Bedeutung Acyl- hat. Er kann aber auch die Rolle einer Vorstufe $-CO-R^7$

spielen, die in einen der in der allgemeinen Formel 1 definierten Substituenten -$R^3$ oder -$R^4$ umgewandelt wird. Er kann schließlich auch die Funktion einer Schutzgruppe [T.W. Greene, Protective Groups in Organic Synthesis, John Wiley and Sons, New York 1981] haben, die im Verlauf der Synthese von Verbindungen der allgemeinen Formel 1 wieder abgespalten und durch -H ersetzt wird.

Bei den Verbindungen der allgemeinen Formel 1 handelt es sich um chirale Substanzen, die in racemischen (±)-Formen oder enantiomerenrein als (+)- und (-)-Formen vorliegen können. Letztere erhält man durch stereoselektive Synthesen, Racematspaltung von Zwischenprodukten oder Racematspaltungen von racemischen Substanzen der allgemeinen Formel 1.

Die Verbindungen der allgemeinen Formel 1 werden nach an sich bekannten Methoden isoliert, gereinigt und als Basen oder mit therapeutisch akzeptablen Säuren in Form von Säureadditionsverbindungen kristallisiert.

Die einzelnen, im Reaktionsschema Seite 25 angegebenen Verfahren bzw. Reaktionsschritte werden nachfolgend erläutert und in Abschnitt 4. mit Beispielen belegt.

## 3.1 2-Amino-5-Z-amino-tetraline (13), (3) und (4) aus entsorechenden 5-Z-Amino-2-tetralonen (2)

Durch reduktive Aminierung von 5-Z-Amino-2-tetralonen (2) mit Ammoniak bzw. den betreffenden primären oder sekundären Aminen enthält man über intermediäre Ketimine, Schiffsche Basen oder Enamine die gewünschten Verbindungen (13), (3) oder (4). Die Zwischenprodukte können isoliert und dann zu den Endprodukten hydriert oder reduziert werden. Einfacher ist es, beide Reaktionsschritte in einem Eintopf-Verfahren durchzuführen.

Die Zwischenverbindungen bilden sich in geeigneten Lösungsmitteln, vorzugsweise in Anwesenheit eines Katalysators und eines das Reaktionswasser bindenden Mittels. Als Lösungsmittel eignen sich alle inerten organischen Lösungsmittel, die unter den gewählten Reaktionsbedingungen unverändert oder zumindest weitgehend unverändert bleiben und die nicht nachteilig in den Reaktionsablauf eingreifen. Dazu gehören Amide -wie zum Beispiel Dimethylformamid oder Hexamethylphosphorsäuretriamid - oder Ester - wie Essigsäuremethylester oder Essigsäureethylester - oder Ether - wie beispielsweise Diethylether, tert.-Butylmethylether, Di-n-butylether, Glykoldimethylether (Glyme), Diglykolidmethylether (Diglyme), Tetrahydrofuran und Dioxan - und bevorzugt Alkohole - besonders bevorzugt Methanol und Ethanol. Diese und andere Lösungsmittel können auch als Gemische verwendet werden. Die Ausgangsverbindungen werden vorzugsweise in Lösung umgesetzt, können aber auch in Suspension zur Reaktion gebracht werden. Als Katalysatoren eignen sich Säuren - insbesondere Protonensäuren.

Hierunter fallen bevorzugt Mineralsäuren wie zum Beispiel Salzsäure, Phosphorsäure oder Schwefelsäure oder organische Säuren mit 1 bis 6 C-Atomen, die gegebenenfalls durch Fluor, Chlor und/oder Brom substituiert sein können. Beispiele derartiger Säuren sind Ameisensäure, Essigsäure, Trifluoressigsäure, Trichloressigsäure oder Propionsäure. Zu den bevorzugten Säuren gehören ebenso Sulfonsäuren mit $C_1$-$C_4$-Alkylresten oder Arylresten, die gegebenenfalls durch Halogenatome substituiert sein können, wie zum Beispiel Methansulfonsäure, Trifluormethansulfonsäure, Ethansulfonsäure, Benzolsulfonsäure, Toluolsulfonsäure. Besonders bevorzugt werden Chlor- oder Bromwasserstoffsäure. Diese Säuren können in katalytischen Mengen eingesetzt werden, vorzugsweise werden jedoch äquivalente Mengen oder es wird ein Überschuß verwendet. Es ist vorteilhaft, das sich bei der Reaktion bildende Wasser aus dem Gleichgewicht zu entfernen, z.B. durch Abdestillieren oder Zugabe von wasserbindenden Mitteln, z.B. Phosphorpentoxid oder vorzugsweise Molekularsieben. Die Reaktion kann innerhalb eines weiten Temperaturbereichs durchgeführt werden, der in der Praxis nach unten durch zu geringe Reaktionsgeschwindigkeit und nach oben durch Überhandnehmen von Nebenreaktionen begrenzt ist. Ein günstiger Bereich liegt zwischen 0 und 100° C, vorzugsweise zwischen 20 und 50° C. Die Reakion wird in der Regel unter Normaldruck durchgeführt, man kann aber auch bei Über- und Unterdruck arbeiten. Die Reaktionspartner werden in äquimolaren Mengen eingesetzt, in Abhängigkeit von den Reaktanden ist aber ein Überschuß der Amin-Komponente, vorzugsweise bis zum 5-fachen, günstig.

Die Weiterumsetzung der Zwischenprodukte erfolgt z.B. durch katalytische Hydrierung in geeigneten Lösungsmitteln, vorzugsweise in Methanol oder Ethanol. Es können jedoch auch andere Lösungsmittel wie Carbonsäuren, Ether u.a. sowie Lösungsmittelgemische verwendet werden. Als Katalysatoren sind bekannte Hydrierungskatalysatoren, z.B. solche auf der Basis von Palladium, Platin oder Nickel, geeignet. Bei der Reduktion mit Wasserstoff wird die Reaktion unter den für die katalytische Hydrierung in Abhängigkeit von den jeweils eingesetzten Katalysatoren, Lösungsmitteln und Reaktanden bekannten Reaktionsbedingungen durchgeführt [K. Harada in Patai, "The Chemistry of the Carbon-Nitrogen Double-Bond", Interscience Publishers, London 1970, S. 276 u. zit. Lit.; P.N. Rylander, Catalytic Hydrogenation over Platinum Metals,

Academic Press, New York 1967, S. 123; F. Möller und R. Schröter in Houben-Weyl, Methoden der organischen Chemie, Band XI/1, Georg Thieme Verlag, Stuttgart 1957, S. 602; W.S. Emerson, Org. Reactions 4 (1949) 174; E.M. Hancock und A.C. Cope, Org. Synth., Coll. Vol. III (1955) 501; J.C. Robinson und H.R. Snyder, Org. Synth., Coll, Vol. III (1955) 717; D.M. Malcolm und C.R. Noller, Org. Synth., Coll. Vol. IV (1963) 603]. Besonders bevorzugt wird die Hydrierung mit Palladium auf Kohle (10 % Pd-Gehalt) in Methanol. In der Regel gelingt die Hydrierung schon bei Normaldruck und Raumtemperatur. Man kann aber auch diese Parameter variieren. Bevorzugt arbeitet man bei Drucken von 1 - 5 bar und Temperaturen zwischen 0 und 100° C und insbesondere bei 20 - 50° C. Alternativ können die Zwischenprodukte auch mit Hilfe anderer Verfahren reduziert werden. Bevorzugt eignen sich dazu komplexe Hydride, insbesondere solche des Aluminiums oder Bors wie z.B. Lithiumaluminiumhydrid, Natriumborhydrid und besonders bevorzugt Natriumcyanborhydrid. Dabei arbeitet man in Lösungsmitteln, die für die betreffenden Hydride geeignet sind, bei Lithiumaluminiumhydrid z.B. in Ethern wie Diethylether oder Tetrahydrofuran, bei Natriumborhydrid und Natriumcyanborhydrid z.B. in Wasser oder Alkoholen, vorzugsweise Methanol oder Ethanol. Die Reduktion mit komplexen Hydriden kann in bekannter Weise katalysiert werden, insbesondere mit Protonsäuren und vorzugsweise mit den zur Bildung der Zwischenprodukte benutzten Säuren beim Eintopfverfahren. Die Reaktionstemperatur kann in weiten Grenzen variiert werden, die in der Praxis nach unten durch zu geringe Reaktionsgeschwindigkeit und nach oben durch Überhandnehmen von Nebenreaktionen gegeben sind. Bevorzugt werden Temperaturen in einem Bereich zwischen -50 und +150° C, insbesondere solche zwischen 0 und 75° C. In der Regel benötigt man berechnete Mengen der Reduktionsmittel; ein Überschuß, vorzugsweise von 10 -25 %, kann sich aber als vorteilhaft erweisen.

Für den Fall, daß der Substituent -Z die in der allgemeinen Formel 1 definierte Bedeutung von -$R^3$ = Acyl hat, gehören die erhaltenen Endprodukte (3) und (4) zu den beanspruchten Substanzen. Falls -Z eine der anderen, der in der allgemeinen Formel 1 definierten Bedeutungen hat, schließt sich eine Überführung von -Z in -$R^3$ oder -$R^4$ an (siehe Reaktionsschema Seite 25).

3.2 2.5-Diamino-tetraline (5) und (6) durch Spaltung der entsprechenden Z-Derivate (4) und (5)

Aus dem Stand der Technik sind zahlreiche Methoden zur Abspaltung von Z - im Falle Z außer Acyl die unter $R^3$ angegebene Bedeutung hat - bekannt [T.W. Greene, Protective Groups in Organic Synthesis, John Wiley and Sons, New York 1981 und zit. Lit.].

Die Spaltung erfolgt am einfachsten durch saure oder alkalische Hydrolyse. Zur sauren Hydrolyse verwendet man vorzugsweise starke Mineralsäuren wie Schwefelsäure, Halogenwasserstoffsäuren wie z.B. Bromwasserstoff und bevorzugt Chlorwasserstoffsäure in wäßriger, alkoholischer oder wäßrig-alkoholischer Lösung. Besonders einfach und bewährt ist das Kochen in konstant siedender Salzsäure (ca. 6 n HCl) oder in wäßrig-ethanolischer Salzsäure. Man kann auch bei höheren oder tieferen Temperaturen arbeiten, deren Bereich für die Praxis nach unten durch zu geringe Reaktionsgeschwindigkeit und nach oben durch Überhandnehmen von Nebenreaktionen begrenzt ist.

Für die alkalische Hydrolyse verwendet man zweckmäßigerweise starke Basen, wie z.B. Alkali- oder Erdalkalihydroxide - bevorzugt Kalium-, Natrium- oder Bariumhydroxid in wäßriger, alkoholischer oder wäßrig-alkoholischer Lösung. Um die Reaktion - was sich u.U. als notwendig erweisen kann - bei höheren Temperaturen durchführen zu können, kann man in Glykol oder Diglykol arbeiten. Die Reaktionstemperatur kann in weiten Grenzen variiert werden, die nach unten durch zu geringe Reaktionsgeschwindigkeit und nach oben durch Überhandnehmen von Nebenreaktionen gegeben sind. Die Temperatur liegt zweckmäßigerweise in einem Intervall zwischen 0 und 200° C und vorzugsweise zwischen 50 und 150° C.

3.3 2-Alkylamino- und 2-Dialkylamino-5-Z-amino-tetraline (3) und (4) durch Alkylierung von 2-Amino-5-Z-amino-tetralinen (13) bzw. (3)

Zu den anwendbaren Alkylierungsverfahren gehört die reduktive Aminierung von Aldehyden oder Ketonen mit den Aminen (13) oder (3).

Dabei verfährt man im Prinzip wie in Abschnitt 3.1 beschrieben. Für reduktive Aminierungen von Formaldehyd, aus denen N-Methyl-Derivate von (3) oder (4) resultieren, bietet sich außerdem die Reaktion von Leukart-Wallach an, bei der Ameisensäure als Reduktionsmittel fungiert. Die Reaktionsbedingungen für derartige reduktive Aminierungen sind bekannt [Autorenkolektiv, Organikum, VEB Deutscher Verlag der Wissenschaften, Berlin 1986 (16. Auflage) S. 491; C. Ferri, Reaktionen der organischen Synthese, Georg Thieme Verlag, Stuttgart 1978, S. 133 und zit. Lit.; F. Möller und R. Schröter in Houben-Weyl, Methoden

der organischen Chemie, Band XI/I, Georg Thieme Verlag, Stuttgart 1957, S. 648].

Alternativ werden die primären bzw. sekundären 2-Amino-Verbindungen (13) und (3) mit Alkylierungsmitteln $R^1X$ oder $R^2X$ umgesetzt. Dabei bedeutet X ein als Anion $X^-$ austretendes Nucleofug ("leaving group") wie z.B. X = Cl, Br, I, $O-SO_2-OR$, $O-SO_2-CH_3$, $O-SO_2-C_6H_5$ oder $O-SO-C_6H_4-CH_3$. Man verwendet das Alkylierungsmittel in berechneten Mengen oder in einem Überschuß, vorzugsweise von 10 - 25 %, und führt die Umsetzung zweckmäßig in einem geeigneten Lösungsmittel oder Lösungsmittelgemisch durch. Geeignete Lösungsmittel sind z.B. Alkohole, Ether oder halogenierte - bevorzugt chlorierte - Kohlenwasserstoffe. Bevorzugt werden Methanol, Ethanol, Tetrahydrofuran und Dimethylformamid. Besonders bewährt haben sich Gemischeaus Dimethylformamid und Tetrahydrofuran. Zur Erzielung einer vollständigen Umsetzung benötigt man in der Regel den Zusatz eines säurebindenden Mittels wie z.B. Kaliumcarbonat oder -hydrogencarbonat, Natriumcarbonat, Lithiumcarbonat, Calciumcarbonat, und vorzugsweise Natriumhydrogencarbonat. Die Reaktionstemperatur kann in weiten Grenzen variiert werden, die in der Praxis nach unten durch zu geringe Reaktionsgeschwindigkeit und nach oben durch Überhandnehmen von Nebenreaktionen gegeben sind. In der Praxis angewandte Temperaturen liegen zwischen 0 und 200° C, vorzugsweise zwischen 50 und 150° C.

Für den Fall, daß der Substituent -Z die für die allgemeine Formel 1 definierte Bedeutung von $-R^3$ = Acyl hat, gehören die erhaltenen Verbindungen (3) und (4) zu den in der allgemeinen Formel 1 beanspruchten Substanzen. Falls -Z eine der anderen, der unter 3 definierten Bedeutungen hat, schließt sich eine Überführung von -Z in $-R^3$ oder $-R^4$ an (siehe Reaktionsschema S. 25).

3.4 2-Alkylamino-5-Z-amino-tetraline (3) durch Entalkylierung von 2-Dialkylamino-5-Z-amino-tetralinen (4)

Tertiäre Amine können mit Hilfe von verschiedenen Verfahren zu sekundären Aminen entalkyliert werden. Von präparativem Wert sind vor allem Entmethylierungen und Entbenzylierungen.

Für Entmethylierungen sind zahlreiche Reagenzien bekannt, von denen vor allem Bromcyan, Phosgen, Chlorameisensäureester und verwandte Substanzen Bedeutung für die Laborpraxis haben. Die Art und Weise der Durchführung der Reaktionen richtet sich nach der Natur des Entmethylierungsmittels.

Von großem praktischem Wert sind Entalkylierungen in Form von katalytischen Entbenzylierungen [s.a.: T.W. Greene, Protective Groups in Organic Synthesis, John Wiley and Sons, New York 1981 und dort zitierte Literatur]. Man arbeitet dabei zweckmäßig in Lösungsmitteln wie Wasser, Alkoholen, Essigsäure oder Mischungen derselben. Es sind aber auch noch andere Lösungsmittel geeignet. Man verwendet typische Entbenzylierungskatalysatoren auf der Basis von beispielsweise Palladium, Platin oder Nickel. Die Reaktion erfolgt in der Regel schon bei Raumtemperatur und Normaldruck. Etwas höhere Temperaturen und Drucke können sich aber u.U. günstig auf den Reaktionsverlauf auswirken. Man kann dabei z.B. auf 100° C bzw. 10 bar gehen, doch werden im allgemeinen Temperaturen von 20 -50° C und Drucke von 1 - 5 bar bevorzugt.

Falls bei den Entalkylierungsprodukten (3) der Substituent -Z die für allgemeine Formel 1 definierte Bedeutung $-R^3$ = Acyl- hat, gehören diese zu den in der allgemeinen Formel 1 beanspruchten Substanzen. Hat -Z dagegen eine andere der für $R^3$ bzw. $R^4$ definierten Bedeutungen, schließt sich eine Überführung von -Z in $-R^3$ oder $-R^4$ an (siehe Reaktionsschema Seite 25).

3.5 5-Amino-(2-alkylamino- und 2-dialkylamino)-tetraline (6) und (5) durch Reduktion von 2-Acylamino-Vorstufen (15) bzw. (8)

Die Ausgangsverbindungen (15) und (8) können z.B. auf folgende Weise hergestellt werden (siehe Reaktionsschema Seite 25): 2-Amino-5-Z-amino-tetraline (13) bzw. (3), die man nach dem unter 3.1 beschriebenen Verfahren erhält, werden am 2-Amino-Stickstoff zu entsprechenden 2-Acylamino-Derivaten (14) bzw. (7) modifiziert. Letztere ergeben nach selektiver Abspaltung von -Z die Verbindungen (15) und (8). In diesen Ausgangsverbindungen (15) und (8) sind die Acyl-Reste $-COR^8$ an der 2-Amino-Funktion so beschaffen, daß die durch Reduktion daraus entstehenden N-Substituenten $-R^1$ bzw. $-R^2$ der in der allgmeinen Formel 1 definierten Bedeutung entsprechen.

Die Umsetzung der Verbindungen (15) und (8) zu den entsprechenden Verbindungen (6) und (5) geschieht mit Hilfe von Reduktionsmitteln. Derartige Reduktionen von Säureamiden sind aus dem Stand der Technik bekannt und können auf dem Wege der elektrochemischen Reduktion, durch Reduktion mit Alkalimetallen sowie durch katalytische Reduktion [R. Schröter in Houben-Weyl, Methoden der organischen Chemie, Band XI/1, Georg Thieme Verlag, Stuttgart 1957, S. 574] oder mit Diboran bzw. Borwasserstoff-Derivaten [J. Fuhrhop und G. Penzlin, Organic Synthesis - Concepts - Methods -Starting Materials, VCH-Verlagsgesellschaft, Weinheim 1986, S. 90] erfolgen. Von den hierzu zur Verfügung stehenden Verfahren sind für die Praxis insbesondere Reduktionen mit komplexen Hydriden geeignet. Bevorzugt werden komplexe Hydride von Aluminium und Bor, besonders bevorzugt wird Lithiumaluminiumhydrid. Die Umsetzungen erfolgen in geeigneten inerten Lösungsmitteln, die sich nach der Natur des Hydrids richten. Umsetzungen mit Lithiumaluminiumhydrid erfolgen bevorzugt in Ethern, z.B. Diethylether, Diisopropylether und besonders bevorzugt in Tetrahydrofuran gegebenenfalls in Gegenwart eines Katalysators. [N.G. Gaylord, Reduction with Complex Metal Hydrides, Wiley New York 1965; A. Hájos, Complex Hydrides, Elsevier New York 1979; V. Bažant, M. Čapka, M. Černy, V. Chvalovsky, K. Kochloefl, M. Kraus und J. Malek, Tetrahedron Lett. 9 (1968) 3303]. In der Regel ist es günstig, über die berechnete Menge hinaus einen Überschuß des Hydrids zu verwenden, der zwischen 5 und 100 %, vorzugsweise zwischen 10 und 50 % der berechneten Menge liegt. Die Reaktionspartner werden gewöhnlich unter Eiskühlung zusammengegeben und anschließend erhitzt. Die Temperaturen sind dabei in weiten Grenzen variierbar und werden für die Praxis nach unten durch zu geringe Reaktionsgeschwindigkeit und nach oben durch Überhandnehmen von Nebenreaktionen begrenzt.

3.6 2-Alkylamino- und 2-Dialkylamino-5-alkylamino-tetraline (12) und (9) durch Reduktion von 5-Z-Amino-Vorstufen (3) bzw. (4)

Man erhält aus Verbindungen (3) bzw. (4) durch Reduktion erfindungsgemäßer Verbindungen (12) bzw. (9), wenn die Substituenten -Z so beschaffen sind (-Z = $-COR^7$), daß sie bei der Reaktion in Substituenten $-R^3$ oder $-R^4$ übergehen, die in der allgemeinen Formel 1 beansprucht werden.

Im übrigen verfährt man dabei entsprechend den Angaben des vorausgehenden Abschnitts 3.5.

3.7 5-Z-(N-Alkylamino)-2-dialkylamino-tetraline (10) und (16) durch Amid-Alkylierung von 5-Z-Amino-2-dialkylamino-tetralinen (4) und (3)

Ausgangsverbindungen sind die Substanzen (4) und (3), bei denen -NH-Z eine Amidgruppe bedeutet. Zur selektiven Alkylierung am Amid-Stickstoff setzt man zweckmäßigerweise Salze der Amid-Funktion ein. Geeignete Salze sind insbesondere die Alkali-Salze, vorzugsweise die Natrium-Salze, die sich z.B. bei der Einwirkung von Natriumhydrid oder Natriumamid auf die Verbindungen (4) und (3) bilden. Diese Salze können vor dem Alkylierungsschritt isoliert werden. Einfacher ist es, sie in geeigneten Lösungsmitteln in situ zu erzeugen und zu alkylieren.Als Reaktionsmedien eignen sich hierzu Ether - wie Glykolidmethylether (Glyme), Diglykolidmethylether (Diglyme), und bevorzugt Tetrahydrofuran - oder Sulfoxide - wie Dimethylsulfoxid - oder Säureamide, unter denen Dimethylformamid besonders bevorzugt wird. [G. Spielberger in Houben-Weyl, Methoden der organischen Chemie, Georg Thieme Verlag, Stuttgart 1957, S. 96; W.F. Fone, J. Org. Chem. 14 (1949) 1099; R.A.W. Johnstone, D.W. Payling und C. Thomas, J. Chem. Soc. [C] 1969, 2223].

Zur Alkylierung verwendet man Alkylierungsmittel $R^3$-X und verfährt im übrigen wie in Abschnitt 3.3 beschrieben. Die Reaktion läuft bereits bei Raumtemperatur ab, man kann aber auch bei tieferen Temperaturen arbeiten, sofern man noch akzeptable Reaktionsgeschwindigkeiten erzielt, oder aber bei höheren Temperaturen, soweit störende Nebenreaktionen noch nicht eintreten. Bevorzugt wird ein Temperaturintervall von -25 bis +150 °C und besonders bevorzugt wird in einen Temperaturbereich von 0 - 75 °C gearbeitet.

Falls bei den Reaktionsprodukten der Substituent -Z in der allgemeinen Formel 1 für $R^3$ definierte Bedeutung Acyl hat, gehören sie zu den in der allgemeinen Formel 1 beanspruchten Substanzen. Hat -Z dagegen eine der anderen, in der allgemeinen Formel 1 definierten Bedeutungen, schließt sich eine Überführung von -Z in $-R^4$ an.

3.8 5-Acylamino-2-dialkylamino-tetraline (4) und (10) durch Acylierung von 5-Amino-2-dialkylamino-tetralinen (5) und (9)

Man setzt die erfindungsgemäßen Verbindungen (5) und (9) durch Acylierung am Anilin-Stickstoff zu Verbindungen (4) und (10) um, die ebenfalls zu den in der allgemeinen Formel 1 beanspruchten Substanzen gehören, wenn -Z die definierte Bedeutung $-R^3$ = Acyl hat.

Für solche Acylierungen stehen verschiedene Verfahren zur Verfügung [C. Ferri, Reaktionen der organischen Synthese, Georg Thieme Verlag, Stuttgart 1978, S. 222 ff]. Vorteilhaft für die Praxis sind Umsetzungen mit typischen Acylierungsmitteln wie z.B. Carbonsäure-halogeniden, bevorzugt Carbonsäure-chloriden, oder Carbonsäure-anhydriden. Man arbeitet in geeigneten inerten Lösungsmitteln in Gegenwart säurebindender Mittel. Als inerte Lösungsmittel werden im allgemeinen organische Solvenzien eingesetzt, die sich unter den angewandten Reaktionsbedingungen nicht verändern, wie zum Beispiel Kohlenwasserstoffe - wie Benzol, Toluol, Xylol oder Erdölfraktionen - oder Halogenkohlenwasserstoffe - wie z.B. Methylenchlorid, Chloroform oder Tetrachlorkohlenwasserstoff oder bevorzugt Ether - wie Diethylether, Glykoldimethylether (Glyme), Diglykolidimethylether (Diglyme) und Tetrahydrofuran. Man kann aber auch nach der Methode von Schotten-Baumann sogar in Wasser in Gegewart von Soda oder Natronlauge arbeiten. [B.C. Challis und J.A. Challis in: Zabicky (Ed.), The Chemistry of Amides, Interscience, New York, N.Y. 1970 S. 731 ff]. Vorteilhaft sind Acylierungen nach der Variante von Einhorn in Pyridin [Autorenkollektiv, Organikum, VEB Deutscher Verlag der Wissenschaften, Berlin, 1988, (17. Auflage) S. 407; L. Gattermann und T. Wieland: Die Praxis des organischen Chemikers, Walter de Gruyter, Berlin, 1982, (43. Auflage) S. 673], das einerseits gute Lösungseigenschaften hat und andererseits als säurebindendes Mittel fungiert. Die Reaktionstemperatur kann in weiten Grenzen variiert werden, die nach unten durch zu geringe Reaktionsgeschwindigkeit und nach oben durch Überhandnehmen von Nebenreaktionen gegeben sind. In der Praxis arbeitet man bei -50 bis +150 °C und vorzugsweise in einem Bereich zwischen 0 und 100 °C.

Man kann auch spezielle Acylierungsmethoden heranziehen, z.B. mit Carbonsäuren, die man bei höheren Temperaturen, vorzugsweise 150 - 200 °C auf die Verbindungen (5) oder (9) einwirken läßt [A.L.J. Beckwith in: Zabicky (Ed.), The Chemistry of Amides, Interscience, New York, N.Y. 1970, S. 105 ff]. Schonender ist es, die Carbonsäuren intermediär in reaktionsfähige Derivate zu überführen, die man unter milderen Bedingungen, vorzugsweise bei 0 - 50 °C, umsetzen kann. Geeignet für solche Aktivierungen von Carbonsäuren sind z.B. Dicyclohexylcarbodiimid oder Carbonyldiimidazol. Die intermediären Carbonsäure-Derivate können isoliert werden, oder man setzt sie vorzugsweise nach ihrer Bildung in situ weiter um.

### 3.9 5-Alkylamino-2-(mono- und dialkyl-)-amino-tetraline (12) und (9) durch Reduktion von Diamid-Vorstufen (17) bzw. (7)

Zur Herstellung der Diamide (17) und (7) geht man aus von Zwischenprodukten (15) bzw. (3). Die Substituenten $-COR^7$, $-COR^8$ und -Z sind so beschaffen, daß sie durch Reduktion in die in der allgmeinen Formel 1 definierten Substituenten $-R^1$ - $R^4$ übergehen. Die Überführung von (15) bzw. (3) in die Diamide (17) bzw. (7) erfolgt durch Acylierung analog dem in Abschnitt 3.8 beschriebenen Verfahren. Die Diamide werden analog zu den unter 3.5 beschriebenen Verfahren zu den gewünschten Verbindungen (12) und (9) reduziert.

### 3.10 Verbindungen (12) und (9) durch Abspaltung von -Z aus (16) bzw. (10)

Die Spaltung von Verbindungen (16) und (10) zu (12) bzw. (9) erfolgt nach den in Abschnitt 3.2 beschriebenen Verfahren.

### 3.11 2,5-Bis-(dialkylamino)-tetraline (11) durch Reduktion von Amid-Vorstufen (10)

In den Verbindungen von Typ (10) hat -Z die Bedeutung eines Säureesters $-COR^7$, der beim Reduktionsschritt analog zu den unter 3.5 angegebenen Verfahren in einen für die Formel (1) definierten Substituenten $-R^3$ oder $-R^4$ überführt werden kann.

### 3.12 5-Alkylamino-2-dialkylamino-tetraline (9) durch Entalkylierung von 5-Dialkylamino-Analogen (11)

Entalkylierungen von Verbindungen (11) zu Verbindungen (9) können nach Verfahren durchgeführt werden, wie sie in Abschnitt 3.4 beschrieben sind.

### 3.13 Enantiomerenreine Verbindungen (1)

Enantiomerenreine Verbindungen (1) ((-)-(1) und (+)-(1)) kann man z.B. aus den racemischen Verbindungen nach bekannten Methoden der Racematspaltung herstellen. Man kann auch ausgehend von enantiomerenreinen Zwischenprodukten zu enantiomerenreinen Verbindungen der allgemeinen Formel 1 gelangen. Es besteht schließlich die Möglichkeit, enantiomerenreine Verbindungen der allgemeinen Formel 1 durch stereoselektive Syntheseverfahren herzustellen.

3.2 Galenik

Die 2,5-Diamino-tetralin-Derivate der allgemeinen Formel I sowie ihre pharmakologisch unbedenklichen Säureadditionssalze können in bekannter Weise in die üblichen Formulierungen - wie Tabletten, Dragees, Pillen, Granulate, Aerosole, Siruppe, Emulsionen, Suspensionen und Lösungen unter Verwendung inerter pharmazeutisch geeigneter Trägerstoffe oder Lösungsmittel überführt werden. Hierbei soll der Anteil der pharmazeutisch wirksamen Verbindung(en) jeweils im Bereich von 0,5 bis 90 Gew.-% der Gesamtzusammensetzung liegen, d.h. in Mengen, die ausreichend sind, um den unten angegebenen Dosierungsbereich zu erreichen.

Die Formulierungen werden zum Beispiel durch Verstrecken der Wirkstoffe mit Lösungsmitteln und/oder Trägerstoffen, gegebenenfalls unter Verwendung von Emulgiermitteln und/oder Dispergiermitteln hergestellt, wobei beispielsweise bei der Verwendung von Wasser als Verdünnungsmittel gegebenenfalls organische Lösungsmittel als Lösungsvermittler bzw. Hilfslösungsmittel eingesetzt werden können.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurysulfat) erwähnt.

Die Applikation erfolgt in üblicher Weise, vorzugsweise oral oder parenteral insbesondere perlingual oder intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Die Tabletten können auch aus mehreren Schichten bestehen. Entsprechend können Dragées durch Überziehen von analog den Tabletten hergestellten Kernen mit den üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethyl-cellulose Netzmittel, beispielsweise Kondensationsprodukte von Fettalkoholen mit Ethylenoxid oder Schutzstoffe, wie p-Hydroxybenzoate, enthalten.Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise in der Weise hergestellt werden, daß man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit den üblichen Trägermitteln, wie Neutralfetten oder Polyäthylenglykol bzw. dessen Derivaten, herstellen.

Für den Fall der parenteralen Anwendung können Lösungen der Wirkstoffe unter Verwendung geeigneter flüssiger Trägermaterialien eingesetzt werden.

Die Dosierung für die orale Anwendung liegt bei 1-300 mg vorzugsweise zwischen 5 und 150 mg.

Trotzdem kann es gegebenenfalls erforderlich sein, von den genannten Mengen abzuweichen, und zwar

in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.Ferner können die Verbindungen der allgemeinen Formel 1 bzw. deren Säureadditionssalze auch mit andersartigen Wirkstoffen kombiniert werden.

Formulierungsbeispiele

| 1. Tabletten | |
|---|---|
| Verbindung nach Beispiel 4.2.27 | 10,0 mg |
| Maisstärke | 99,0 mg |
| sek. Calciumphosphat | 140,0 mg |
| Magnesiumstearat | 1,0 mg |
| | 250,0 mg |

Die Bestandteile werden in üblicher Weise zu Tabletten von 250 mg Gewicht verarbeitet.

| 2. Kapseln | |
|---|---|
| Verbindung nach Beispiel 4.2.27 | 150,0 mg |
| Maisstärke | 150,0 mg |
| | 300,0 mg |

Die fein gepulverten Komponenten werden intensiv vermischt. Jeweils 300 mg der Mischung werden in übliche Gelatinekapseln abgefüllt.

Die nachfolgenden Herstellungsbeispiele erläutern die Erfindung, ohne sie einzuschränken.

Die nachfolgend in den Abschnitten 4.1 - 4.10 beschriebenen Herstellungsbeispiele korrespondieren mit den oben unter 3.1 - 3.10 beschriebenen Herstellungsverfahren und erläutern die Erfindung ohne sie einzuschränken.

## 4. Beispiele

### 4.1 Verbindungen (3) und (4) aus (2)

#### Beispiel 4.1.1

5-Acetylamino-2-n-propylamino-tetralin Base, Hydrobromid, Methansulfonat und Hydrochlorid

40,6 g (0,20 Mol) 5-Acetylamino-2-tetralon, 35,5 g (0,60 Mol) n-Propylamin, 31 g Molekularsieb (0,4 nm, 10 mesh ASTM) und 580 ml Ethanol werden unter Rühren in Stickstoff-Atmosphäre tropfenweise mit 36,0 g (0,60 Mol) Eisessig versetzt. Die Temperatur wird durch Kühlung bei 30 - 35 °C gehalten. Nach vollendeter Zugabe des Eisessigs wird noch 3 Stunden bei Raumtemperatur weitergeführt. Dann wird vom Molekularsieb abgesaugt, das mit etwas Ethanol nachgewaschen wird. Das Filtrat wird bei 20 °C und 2 bar Wasserstoff in Gegenwart von 2,5 g Platinoxid hydriert.

Die Wasserstoffaufnahme ist nach Verbrauch der berechneten Menge beendet. Der Katalysator wird abgesaugt, mit Ethanol gewaschen, und das Filtrat im Rotationsverdampfer, zuletzt bei 80 °C und vollem

Wasserstrahlvakuum, eingeengt.

Base:

100 mg des Rohproduktes werden in 0,3 ml Aceton gelöst und die Lösung mit 1,0 ml Petrolether versetzt. Es kristallisiert die reine Base, die nach dem Stehen über Nacht abgesaugt, mit wenig Lösungsmittelgemisch gewaschen und bei 50° C getrocknet wird. Ausbeute 77 mg (77 %), Schmelzpunkt 90° C.

Hydrobromid:

100 mg (0,41 mMol) der Rohbase werden in 1 ml Ethanol mit der berechneten Menge (0,17 ml) 2,5 n ethanolischer Bromwasserstoffsäure gelöst. Nach Zugabe von Diethylether bis zur gerade beginnenden Trübung tritt Kristallisation ein. Nach dem Stehen über einen Zeitraum von ca. 12 Stunden bei Raumtemperatur wird abgesaugt, mit Ethanol/Ether gewaschen und bei 80° C getrocknet. Ausbeute 120 mg (92,3 % d. Th.), Schmelzpunkt 271° C.

Methansulfonat:

100 mg (0,41 mMol) der Rohbase werden in 1 ml Ethanol mit der berechneten Menge (0,17 ml) 2,5 n ethanolischer Methansulfonsäure gelöst. Nach tropfenweiser Zugabe von Diethylether bis zur gerade beginnenden Trübung tritt Kristallisation ein. Nach dem Stehen über einen Zeitraum von ca. 12 Stunden bei Raumtemperatur wird abgesaugt, mit Ethanol/Ether gewaschen und bei 80° C getrocknet. Ausbeute 130 mg (94,9 % d. Th.), Schmelzpunkt 167° C.

Hydrochlorid:

Der Rest der Rohbase wird mit 200 ml Ethanol gelöst und die Lösung mit der auf das eingesetzte 5-Acetyl-2-tetralon (0,2 Mol) berechneten Menge 37 %iger Salzsäure (19,7 g) versetzt. Aus der Lösung, die unter Rühren allmählich mit 400 ml Diethylether versetzt wird, kristallisiert das Hydrochlorid. Zur Vervollständigung der Kristallisation wird 2 Stunden im Eisbad gerührt, dann abgesaugt und mit Ethanol/Ether 1:2 gewaschen. Nach dem Trocknen, zunächst an der Luft bei Raumtemperatur, dann im Trockenschrank bei 80° C bis zur Gewichtskonstanz, werden 52,3 g Kristallisat mit einem Schmelzpunkt von 284° C erhalten, was einer Ausbeute von 92,4 % d. Th. entspricht. Eine aus Methanol/Ether umkristallisierte Probe schmilzt unverändert bei 284° C.

Beispiel 4.1.2

5-Acetylamino-2-n-butylamino-tetralin-hydrochlorid

Ausgehend von 2,03 g (0,01 Mol) 5-Acetylamino-2-tetralon und 2,2 g (0,03 Mol) n-Butylamin erhält man analog Beispiel 4.1.1 die Titelverbindung in einer Ausbeute von 2,0 g (67,4 % d. Th.) und mit einem Schmelzpunkt von 281° C, der sich nach dem Umkristallisieren aus Methanol/Ether nicht ändert.

Beispiel 4.1.3

5-Acetylamino-2-sec-butylamino-tetralin-hydrochlorid

Ausgehend von 4,06 g (0,02 Mol) 5-Acetylamino-2-tetralon und 4,4 g (0,06 Mol) sec-Butylamin erhält man analog Beispiel 4.1.1 die Titelverbindung in einer Ausbeute von 2,9 g (48,9 % d. Th.) und mit einem Schmelzpunkt von 228° C, der sich nach Umkristallisieren aus Methanol/Ether nicht ändert.

Beispiel 4.1.4

5-Acetylamino-2-(2-phenylethyl-amino)-tetralin Hydrochlorid und Base

Ausgehend von 4,06 g (0,02 Mol) 5-Acetylamino-2-tetralon und 7,3 g (0,06 Mol) 2-Phenylethylamin erhält man analog Beispiel 4.1.1 das Hydrochlorid der Titelverbindung in einer Ausbeute von 4,8 g (69,7 % d. Th.) und mit einem Schmelzpunkt von 288° C, der sich nach Umkristallisieren aus Methanol/Wasser/Ether nicht ändert.

Zur Überführung in die Base werden 2,8 g (0,0081 Mol) des Hydrochlorids mit 28 ml Wasser, 56 ml Methylenchlorid und 2 ml konz. Ammoniak geschüttelt. Nach Trennung der Phasen wird die wäßrige Schicht noch 2 mal mit je 14 ml Methylenchlorid extrahiert. Die vereinigten Methylenchlorid-Extrakte werden mit 20 ml Wasser gewaschen, mit Natriumsulfat getrocknet und im Rotationsverdampfer, zuletzt bei 80° C und vollem Wasserstrahlvakuum, bis zur Gewichstskonstanz eingedampft. Der Rückstand (2,5 g), der spontan kristallisiert, entspricht einer Ausbeute von 100 %. Schmelzpunkt 132 - 134° C.

Beispiel 4.1.5

5-Acetylamino-2-(3-phenylpropyl-amino)-tetralin Hydrochlorid und Base

Ausgehend von 4,06 g (0,02 Mol) 5-Acetylamino-2-tetralon und 8,1 g (0,06 Mol) 3-Phenylpropyl-amin erhält man analog Beispiel 4.1.1 das Hydrochlorid der Titelverbindung in einer Ausbeute von 5,4 g (75,2 % d. Th.) und mit einem Schmelzpunkt von 270° C, der sich nach Umkristallisieren aus Methanol/Wasser/Ether auf 274° C erhöht.

Aus 2,9 g des Hydrochlorids (0,0081 Mol) erhält man analog Beispiel 4.1.4 die Base in einer Ausbeute von 100 % und mit einem Schmelzpunkt von 75 - 76° C.

Beispiel 4.1.6

5-Acetylamino-2-(4-phenylbutyl-amino)-tetralin-hydrochlorid

Ausgehend von 3,4 g (0,0167 Mol) 5-Acetylamino-2-tetralon und 7,5 g (0,05 Mol) 4-Phenylbutylamin erhält man analog Beispiel 4.1.1 die Titelverbindung in einer Ausbeute von 4,94 g (79,3 % d. Th.) und mit einem Schmelzpunkt von 278° C , der sich nach Umkristallisieren aus Ethanol/Ether nicht ändert.

Beispiel 4.1.7

5-Acetylamino-2-(5-phenylpentyl-amino)-tetralin-hydrochlorid

Ausgehend von 2,03 g (0,01 Mol) 5-Acetamino-2-tetralon und 4,9 g (0,03 Mol) 5-Phenylpentyl-amin erhält man analog Beispiel 4.1.1 die Titelverbindung in einer Ausbeute von 3,25 g (84,0 % d. Th.) und mit einem Schmelzpunkt von 270° C, der sich nach Umkristallisieren aus Methanol/Ether nicht ändert.

Beispiel 4.1.8

5-Acetylamino-2-(6-phenylhexyl-amino)-tetralin-hydrochlorid

Ausgehend von 2,03 g (0,01 Mol) 5-Acetylamino-2-tetralon und 5,3 g (0,03 Mol) 6-Phenylhexyl-amin erhält man analog Beispiel 4.1.1 die Titelverbindung in einer Ausbeute von 3,5 g (87,3 % d. Th.) und mit einem Schmelzpunkt von 268° C, der sich nach Umkristallisieren aus Methanol/Ether nicht ändert.

Beispiel 4.1.9

5-Acetylamino-2-(9-phenylnonyl-amino)-tetralin-hydrochlorid

Ausgehend von 3,07 g (0,015 Mol) 5-Acetylamino-2-tetralon und 9,87 g (0,045 Mol) 9-Phenylnonyl-amin erhält man analog Beispiel 4.1.1 die Titelverbindung in einer Ausbeute von 5,35 g (80,5 % d. Th.) und mit einem Schmelzpunkt von 272° C, der nach Umkristallisieren aus Methanol/Ether auf 273° C ansteigt.

Beispiel 4.1.10

5-Acetylamino-2-[2-(3-indolyl)-ethyl-amino]-tetralin Base und Hydrochlorid

Ausgehend von 6,1 g (0,03 Mol) 5-Acetylamino-2-tetralin und 5,3 g (0,033 Mol) Tryptamin (3-(2-Aminoethyl)-indol) erhält man analog Beispiel 4.1.1 die Titelverbindung in Form der Base. Sie wird durch Säulenchromatographie an Kieselgel (150 g) unter Verwendung einer Mischung Methylenchlorid/Methanol/konz. Ammoniak 90:10:0,5 als Laufmittel gereinigt. Ausbeute 4,7 g (45,1 % d. Th.)

Zur Überführung in das Hydrochlorid werden 0,50 g der Base in 5 ml Methanol mit der berechneten Menge 2,5 n ethanolischer Salzsäure gelöst. Die Lösung wird mit Diethylether bis zur gerade eintretenden Trübung versetzt. Es kristallisiert das Hydrochlorid, das man in einer Ausbeute von 0,5 g (89,9 % d. Th.) erhält und das bei 256° C schmilzt. Nach dem Umkristallisieren aus Ethanol ändert sich der Schmelzpunkt nicht.

Beispiel 4.1.11

5-Acetylamino-2-[2-(2-thienyl)-ethyl-amino]-tetralin-hydrochlorid

2,03 g (0,01 Mol) 5-Acetylamino-2-tetralon, 3,2 g (0,02 Mol) 2-(2-Thienyl)-ethyl-amin-hydrochlorid und 0,75 g (0,012 Mol) Natriumcyanborhydrid werden in 30 ml Methanol gelöst und 24 Stunden bei Raumtemperatur gerührt. Anschließend wird durch tropfenweise Zugabe von konz. Salzsäure unter Rühren und Eiskühlung angesäuert. Nach 10 Minuten wird unter weiterem Rühren mit 100 ml Wasser und darauf portionsweise mit insgesamt 4,0 g wasserfreier Soda versetzt. Dann wird nacheinander mit 50 und zweimal mit jeweils 25 ml Methylenchlorid extrahiert. Die vereinigten Extrakte werden mit 20 ml Wasser gewaschen, mit Natriumsulfat getrocknet und nach dem Abtrennen des Trockenmittels im Rotationsverdampfer, zuletzt bei 90° C und vollem Wasserstrahlvakuum, eingeengt. Der Rückstand wird mit 5 ml Methanol und 4,0 ml 2,5 n ethanolischer Salzsäure gelöst und die Lösung mit Diethylether bis zur gerade einsetzenden Trübung versetzt. Nach Stehen über einen Zeitraum von ca. 12 Stunden bei 5° C wird die Titelverbindung abgesaugt, mit Ethanol/Ether gewaschen und bei 80° C bis zur Gewichtskonstanz getrocknet. Ausbeute 3,1 g (88,3 % d. Th.), Schmelzpunkt 276° C (unverändert nach Umkristallisieren aus Methanol/Ether).

Beispiel 4.1.12

5-Acetylamino-2-ethylamino-tetralin-hydrochlorid

Ausgehend von 2,03 g (0,01 Mol) 5-Acetylamino-2-tetralon und 2,45 g (0,03 Mol) Ethylamin-hydrochlorid erhält man analog Beispiel 4.1.11 die Titelverbindung in einer Ausbeute von 1,2 g (44,6 % d. Th.) und mit einem Schmelzpunkt von 351° C, der sich nach Umkristallisieren aus Methanol/Ether nicht ändert.

Beispiel 4.1.13

5-Acetylamino-2-allylamino-tetralin-hydrochlorid

Ausgehend von 3,04 g (0,015 Mol) 5-Acetylamino-2-tetralon und 4,2 g (0,045 Mol) Allylamin-hydrocholid erhält man analog Beispiel 4.1.11 die Titelverbindung in einer Ausbeute von 3,30 g (78, 4 % d. Th.) und mit einem Schmelzpunkt von 263° C, der sich beim Umkristallisieren aus Methanol/Wasser Ether nicht ändert.

Beispiel 4.1.14

5-Acetylamino-2-propargylamino-tetralin-hydrochlorid

Ausgehend von 2,03 g (0,01 Mol) 5-Acetylamino-2-tetralon und 1,37 g (0,015 Mol) Propargylamin-hydrochlorid erhält man analog Beispiel 4.1.11 die Titelverbindung in einer Ausbeute von 2,24 g (80,3 % d. Th.) und mit einem Schmelzpunkt von 225° C, der sich beim Umkristallisieren aus Methanol/Ether nicht ändert.

Beispiel 4.1.15

5-Acetylamin-2-cyclopropylmethylamino-tetralin-hydrochlorid

Ausgehend von 2,03 g (0,01 Mol) 5-Acetylamino-2-tetralon und 1,61 g (0,015 Mol) Cyclopropylmethylamin-hydrochlorid erhält man analog Beispiel 4.1.11 die Titelverbindung in einer Ausbeute von 1,62 g (55, 9 % d. Th.) und mit einem Schmelzpunkt von 266° C, der sich nach Umkristallisieren aus Methanol/Ether nicht ändert.

Beispiel 4.1.16

5-Acetylamino-2-benzylamino-tetralin-hydrochlorid

Ausgehend von 4,06 g (0,02 Mol) 5-Acetylamino-2-tetralon und 8,6 g (0,06 Mol) Benzylamin-hydrochlorid erhält man analog Beispiel 4.1.11 die Titelverbindung in einer Ausbeute von 5,6 g (84.5 % d. Th.) und mit einem Schmelzpunkt von 281° C, der sich nach Umkristallisieren aus Methanol/Ether nicht ändert.

Beispiel 4.1.17

5-Acetylamino-2-[2-(2-furyl)-ethyl-amino]-tetralin-hydrochlorid

Ausgehend von 0,61 g (0,0031 Mol) 5-Acetylamino-2-tetralon und 0,9 g (0,006 Mol) 2-(2-Furyl)-ethylamin-hydrochlorid erhält man analog Beispiel 4.1.11 die Titelverbindung in einer Ausbeute von 0,8 g (77,1 % d. Th.) und mit einem Schmelzpunkt von 256° C, der nach Umkristallisieren aus Methanol/Ether auf 257° C ansteigt.

Beispiel 4.1.18

5-Acetylamino-2-(cis-cinnamylamino)-tetralin-hydrochlorid

Ausgehend von 1,02 g (0,005 Mol) 5-Acetylamino-2-tetralon und 1,7 g (0,01 Mol) cis-Cinnamylamin-hydrochlorid erhält man analog Beispiel 4.1.11 die Titelverbindung in einer Ausbeute von 1.35 g (76,5 % d. Th.) und mit einem Schmelzpunkt von 246° C, der sich beim Umkristallisieren aus Methanol/Ether nicht

ändert.

Beispiel 4.1.19

5-Acetylamino-2-(trans-cinnamylamino)-tetralin-hydrochlorid

Ausgehend von 1,02 g (0,005 Mol) 5-Acetylamino-2-tetralon und 1,7 g (0,01 Mol) trans-Cinnamylamin-hydrochlorid erhält man analog Beispiel 4.1.11 die Titelverbindung in einer Ausbeute von 1,1 g (61,6 % d. Th.) und mit einem Schmelzpunkt von 257 °C, der sich nach Umkristallisieren aus Methanol/Ether nicht ändert.

Beispiel 4.1.20

5-Acetylamino-2-furfurylamino-tetralin-hydrochlorid

Ausgehend von 2,03 g (0,01 Mol) 5-Acetylamino-2-tetralon und 4,0 g (0,03 Mol) Furfurylamin-hydrochlorid erhält man analog Beispiel 4.1.11 die Titelverbindung in einer Ausbeute von 2,1 g (65,4 % d. Th.) und mit einem Schmelzpunkt von 296 °C, der sich beim Umkristallisieren aus Methanol/Ether nicht ändert.

Beispiel 4.1.21

5-Acetylamino-2-(2-p-tolyl-ethyl-amino)-tetralin-hydrochlorid

Ausgehend von 0,61 g (0,003 Mol) 5-Acetylamino-2-tetralon erhält man analog Beispiel 4.1.11 die Titelverbindung in einer Ausbeute von 0,68 g (63,0 % d. Th.) und mit einem Schmelzpunkt von 286 °C.

Beispiel 4.1.22

5-Acetylamino-2-(2-p-methoxyphenyl-ethyl-amino)-tetralin-hydrochlorid

Ausgehend von 1,02 g (0,005 Mol) 5-Acetylamino-2-tetralon erhält man analog Beispiel 4.1.11 die Titelverbindung in einer Ausbeute von 1,25 g (66,7 % d. Th.) und mit einem Schmelzpunkt von 279 °C.

Beispiel 4.1.23

5-Acetylamino-2-(2-p-fluorphenyl-ethyl-amino)-tetralin-hydrochlorid

Ausgehend von 0,61 g (0,003 Mol) 5-Acetylamino-2-tetralon erhält man analog Beispiel 4.1.11 die Titelverbindung in einer Ausbeute von 0,65 g (60,0 % d. Th.) und mit einem Schmelzpunkt von 280 °C.

Beispiel 4.1.24

5-Acetylamino-2-(2-m-methoxyphenyl-ethyl-amino)-tetralin-hydrochlorid

Ausgehend von 1,02 g (0,005 Mol) 5-Acetylamino-2-tetralon erhält man analog Beispiel 4.1.11 die Titelverbindung in einer Ausbeute von 1,25 g (66,7 % d. Th.) und mit einem Schmelzpunkt von 269 °C.

Beispiel 4.1.25

5-Acetylamino-2-dimethylamino-tetralin-hydrochlorid

Ausgehend von 2,03 g (0,01 Mol) 5-Acetylamino-2-tetralon und 2,45 g (0,03 Mol) Dimethylamin-hydrochlorid erhält man analog Beispiel 4.1.11 die Titelverbindung in einer Ausbeute von 1,35 g (50,2 % d. Th.) und mit einem Schmelzpunkt von 199° C, der sich nach dem Umkristallisieren aus Methanol Ether nicht ändert.

Beispiel 4.1.26

5-Acetylamino-2-(N-benzyl-N-methyl-amino)-tetralin-hydrochlorid

Ausgehend von 2,03 g (0,01 Mol) 5-Acetylamino-2-tetralon und 4,37 g (0,03 Mol) N-Benzyl-methylamin-hydrochlorid erhält man analog Beispiel 4.1.11 die Titelverbindung in einer Ausbeute von 2,0 g (58,1 % d. Th.) und mit einem Schmelzpunkt von 243° C, der sich nach Umkristallisieren aus Methanol Ether nicht ändert.

4.2 Verbindungen (5) und (6) aus (4) bzw. (3)

Beispiel 4.2.1

5-Amino-2-n-propylamino-tetralin (Dihydrochlorid und Dihydrobromid)

2,82 g (0,01 Mol) 5-Acetylamino-2-n-propylamino-tetralin-hydrochlorid (Beispiel 4.1.1) werden in 140 ml 6 n HCl 2,5 Stunden unter Rückfluß gekocht. Anschließend wird im Rotationsverdampfer, zuletzt bei 80° C und vollem Wasserstrahlvakuum, eingedampft. Der Rückstand wird mit 20 ml Methanol gelöst und die Lösung unter Rühren tropfenweise mit 20 ml Diethylether versetzt. Dabei kristallisiert die Titelverbindung als Dihydrochlorid. Nach dem Stehen über ca. 12 Stunden bei 0° C wird abgesaugt, mit Methanol Ether 1:1 gewaschen und zunächst an der Luft bei Raumtemperatur, dann bei 80° C bis zur Gewichtskonstanz getrocknet. Ausbeute 2,7 g (97,5 % d. Th.), Schmelzpunkt >300° C. Durch Umkristallisieren aus 50 ml Methanol, 10 ml Wasser und 100 ml Ether (Ausbeute 2,4 g) wird der Schmelzpunkt nicht verändert.

Analog erhält man durch Spaltung von 0,20 g (0,7 mMol) der Ausgangsverbindung mit 48 %iger Bromwasserstoffsäure die Titelverbindung als Dihydrobromid in einer Ausbeute von 0,21 g (81,7 % d. Th.) und mit einem Schmelzpunkt von >300° C.

Beispiel 4.2.2

5-Amino-2-n-butylamino-tetralin-dihydrochlorid

Ausgehend von 0,60 g (0,002 Mol) 5-Acetylamino-2-n-butylamino-tetralin-hydrochlorid (Beispiel 4.1.2) erhält man analog Beispiel 4.2.1 die Titelverbindung in einer Ausbeute von 0,57 g (97,8 % d. Th.) und mit einem Schmelzpunkt von 290° C, der sich nach Umkristallisieren aus Methanol/Wasser Ether nicht ändert.

Beispiel 4.2.3

5-Amino-2-sec-butylamino-tetralin-dihydrochlorid

Ausgehend von 0,50 g (0,0017 Mol) 5-Acetylamino-2-sec-butylamino-tetralin-hydrochlorid (Beispiel 4.1.3) erhält man analog Beispiel 4.2.1 die Titelverbindung in einer Ausbeute von 0,45 g (90,9 % d. Th.) und mit einem Schmelzpunkt von >300 °C, der sich nach Umkristallisieren aus Methanol/Ether nicht ändert.

Beispiel 4.2.4

5-Amino-2-(2-phenylethyl-amino)-tetralin-dihydrochlorid

Ausgehend von 1,00 g (0,0029 Mol) 5-Acetylamino-2-(2-phenylethyl-amino)-tetralin-hydrochlorid (Beispiel 4.1.4) erhält man analog Beispiel 4.2.1 die Titelverbindung in einer Ausbeute von 0,95 g (96,5 % d. Th.) und mit einem Schmelzpunkt von 325 °C, der sich nach Umkristallisieren aus Methanol/Wasser/Ether nicht ändert.

Beispiel 4.2.5

5-Amino-2-(3-phenylpropyl-amino)-tetralin-dihydrochlorid

Ausgehend von 1,00 g (0,0028 Mol) 5-Acetylamino-2-(3-phenylpropyl-amino)-tetralin-hydrochlorid (Beispiel 4.1.5) erhält man analog Beispiel 4.2.1 die Titelverbindung in einer Ausbeute von 0,90 g (91,4 % d. Th.) und mit einem Schmelzpunkt von 325 °C, der sich nach Umkristallisieren aus Methanol/Wasser/Ether nicht ändert.

Beispiel 4.2.6

5-Amino-2-(4-phenylbutyl-amino)-tetralin-dihydrochlorid

Ausgehend von 1,00 g (0,0027 Mol) 5-Acetylamino-2-(4-phenylbutylamino)-tetralin-hydrochlorid (Beispiel 4.1.6) erhält man analog Beispiel 4.2.1 die Titelverbindung in einer Ausbeute von 0,96 g (97,5 % d. Th.) und mit einem Schmelzpunkt von >300 °C, der sich nach Umkristallisieren aus Methanol/Ether nicht ändert.

Beispiel 4.2.7

5-Amino-2-(5-phenylpentyl-amino)-tetralin-dihydrochlorid

Ausgehend von 0,50 g (0,0013 Mol) 5-Acetylamino-2-(5-phenylpentyl-amino)-tetralin-hydrochlorid (Beispiel 4.1.7) erhält man analog Beispiel 4.2.1 die Titelverbindung in einer Ausbeute von 0,47 g (95,3 % d. Th.) und mit einem Schmelzpunkt von >300 °C, der sich beim Umkristallisieren aus Methanol/Wasser/Ether nicht ändert.

Beispiel 4.2.8

5-Amino-2-(6-phenylhexyl-amino)-tetralin-dihydrochlorid

Ausgehend von 0,50 g (0,0013 Mol) 5-Acetylamino-2-(6-phenylhexyl-amino)-tetralin-hydrochlorid (Beispiel 4.1.8) erhält man analog Beispiel 4.2.1 die Titelverbindung in einer Ausbeute von 0,47 g (95,3 % d. Th.) und mit einem Schmelzpunkt von >300 °C, der sich nach Umkristallisieren aus Methanol/Wasser/Ether nicht ändert.

Beispiel 4.2.9

5-Amino-2-(9-phenylnonyl-amino)-tetralin-dihydrochlorid

Ausgehend von 1,0 g (2,26 mMol) 5-Acetylamino-2-phenylnonyl-amino)-tetralin-hydrochlorid (Beispiel 4.1.9) erhält man analog Beispiel 4.2.1 die Titelverbindung in einer Ausbeute von 0.90 g (91.9 % d. Th.) und mit einem Schmelzpunkt von >300 °C, der sich nach Umkristallisieren aus Methanol/Wasser/Ether nicht ändert.

Beispiel 4.2.10

5-Amino-2-[2-(3-indolyl)-ethyl-amino]-tetralin-dihydrochlorid

Ausgehend von 0.7 g (0,002 Mol) 5-Acetylamino-2-[3-indolyl)-ethyl-amino]-tetralin (Beispiel 4.1.10) erhält man analog Beispiel 4.2.1 die Titelverbindung in einer Ausbeute von 0,6 g (79,4 % d. Th.) und mit einem Schmelzpunkt von >310 °C.

Beispiel 4.2.11

5-Amino-2-[2-(2-thienyl)-ethyl-amino]-tetralin-dihydrochlorid

Ausgehend von 0,70 g (0,0026 Mol) 5-Acetylamino-2-[2-(2-thienyl)-ethyl-amino]-tetralin-hydrochlorid (Beispiel 4.1.11) erhält man analog Beispiel 4.2.1 die Titelverbindung in einer Ausbeute von 0.60 g (66,7 % d. Th.) und mit einem Schmelzpunkt von > 300 °C, der sich nach Umkristallisieren aus Methanol/Wasser/Ether nicht ändert.

Beispiel 4.2.12

5-Amino-2-ethylamino-tetralin-dihydrochlorid

Ausgehend von 1,44 g (0,0054 Mol) 5-Acetylamino-2-ethylamino-tetralin-hydrochlorid (Beispiel 4.1.12) erhält man analog Beispiel 4.2.1 die Titelverbindung in einer Ausbeute von 1,3 g (91.6 % d. Th.) und mit einem Schmelzpunkt von > 300 °C, der sich nach Umkristallisieren aus Methanol/Ether nicht ändert.

Beispiel 4.2.13

5-Amino-2-allylamino-tetralin-dihydrochlorid

Ausgehend von 0,8 g (0,0029 Mol) 5-Acetylamino-2-allylamino-tetralin-hydrochlorid (Beispiel 4.1.13) erhält man analog Beispiel 4.2.1 die Titelverbindung in einer Ausbeute von 0,75 g (78,4 % d. Th.) und mit einem Schmelzpunkt von > 300 °C, der sich nach Umkristallisieren aus Methanol/Wasser/Ether nicht ändert.

Beispiel 4.2.14

5-Amino-2-propargylamino-tetralin-dihydrochlorid

Ausgehend von 1,24 g (0,0045 Mol) 5-Acetylamino-2-propargylamino-tetralin-hydrochlorid (Beispiel

4.1.14) erhält man analog Beispiel 4.2.1 die Titelverbindung in einer Ausbeute von 1,12 g (91,8 % d. Th.) und mit einem Schmelzpunkt von 260 °C.

Beispiel 4.2.15

5-Amino-2-cyclopropylmethylamino-tetralin-dihydrochlorid

Ausgehend von 0,90 g (0,0031 Mol) 5-Acetylamino-2-cyclopropylmethyl-amino-tetralin-hydrochlorid (Beispiel 4.1.15) erhält man analog Beispiel 4.2.1 die Titelverbindung in einer Ausbeute von 0,70 g (85,3 % d. Th.) und mit einem Schmelzpunkt von > 300 °C, der sich nach Umkristallisieren aus Methanol/Wasser. Ether nicht ändert.

Beispiel 4.2.16

5-Amino-2-benzylamino-tetralin-dihydrochlorid

Ausgehend von 0,50 g (0,0017 Mol) 5-Acetylamino-2-benzylamino-tetralin-hydrochlorid (Beispiel 4.1.16) erhält man analog Beispiel 4.2.1 die Titelverbindung in einer Ausbeute von 0,46 g (81,7 % d. Th.) und mit einem Schmelzpunkt von ca. 330 °C, der sich nach Umkristallisieren aus Methanol/Ether nicht ändert.

Beispiel 4.2.17

5-Amino-2-[2-(2-furyl)-ethyl-amino]-tetralin-dihydrochlorid

Ausgehend von 0,35 g (0,0011 Mol) 5-Acetylamino-2-[2-(2-furyl)-ethyl-amino]-tetralin-hydrochlorid erhält man analog Beispiel 4.2.1 die Titelverbindung in einer Ausbeute von 0,21 g (61,1 % d. Th.) und mit einem Schmelzpunkt von > 300 °C, der sich nach Umkristallisieren aus Methanol/Ether nicht ändert.

Beispiel 4.2.18

5-Amino-2-(cis-cinnamylamino)-tetralin-dihydrochlorid

Ausgehend von 0,30 g (0,85 mMol) 5-Acetylamino-2-cinnamylamino)-tetralin-hydrochlorid (Beispiel 4.1.18) erhält man analog Beispiel 4.2.1 die Titelverbindung in einer Ausbeute von 0,25 g (84,8 % d. Th.) und einem Schmelzpunkt von 297 °C, der sich nach Umkristallisieren aus Methanol/Ether nicht ändert.

Beispiel 4.2.19

5-Amino-2-(trans-cinnamylamino)-tetralin-dihydrochlorid

Ausgehend von 0,40 g (0,0011 Mol) 5-Acetylamino-(transcinnamylamino)-tetralin-hydrochlorid (Beispiel 4.1.19) erhält man analog Beispiel 4.2.1 die Titelverbindung in einer Ausbeute von 0,35 g (88,8 % d. Th.) und mit einem Schmelzpunkt von 308 °C, der sich nach Umkristallisieren aus Methanol/Wasser/Ether nicht ändert.

Beispiel 4.2.20

5-Amino-2-furfurylamino-tetralin-dihydrochlorid

Ausgehend von 1,50 g (0,0053) 5-Acetylamino-2-furfurylamino-tetralin-hydrochlorid (Beispiel 4.1.20) erhält man analog Beispiel 4.2.1 die Titelverbindung in einer Ausbeute von 1,35 g (80.8 % d. Th.) und mit einem Schmelzpunkt von > 300 ° C.

Beispiel 4.2.21

5-Amino-2-(2-tolyl-ethyl-amino)-tetralin-dihydrochlorid

Ausgehend von 0,36 g (0,001 Mol) 5-Acetylamino-2-(2-p-tolyl-ethyl-amino)-tetralin-hydrochlorid (Beispiel 4.1.21) erhält man analog Beispiel 4.2.1 die Titelverbindung in einer Ausbeute von 0,34 g (96.2 % d. Th.) und mit einem Schmelzpunkt von > 300 ° C.

Beispiel 4.2.22

5-Amino-2-(2-p-methoxyphenyl-ethyl-amino)-tetralin-dihydrochlorid

Ausgehend von 0,75 g (0,002 Mol) 5-Acetylamino-2-(2-p-methoxyphenyl-ethyl-amino)-tetralin-hydrochlorid (Beispiel 4.1.22) erhält man analog Beispiel 4.2.1 die Titelverbindung in einer Ausbeute von 0,67 g (90,7 % d. Th.) und mit einem Schmelzpunkt von > 300 ° C

Beispiel 4.2.23

5-Amino-2-(2-p-fluorphenyl-ethyl-amino)-tetralin-dihydrochlorid

Ausgehend von 0,36 g (0,001 Mol) 5-Acetylamino-2-(2-p-fluorphenyl-ethyl-amino)-tetralin-hydrochlorid (Beispiel 4.1.23) erhält man analog Beispiel 4.2.1 die Titelverbindung in einer Ausbeute von 0,32 g (89,5 % d. Th.) und mit einem Schmelzpunkt von > 300 ° C.

Beispiel 4.2.24

5-Amino-2-(2-m-methoxyphenyl-ethyl-amino)-tetralin-dihydrochlorid

Ausgehend von 0,75 g (0,002 Mol) 5-Acetylamino-2-(2-m-methoxyphenyl-ethyl-amino)-tetralin-hydrochlorid (Beispiel 4.1.24) erhält man analog Beispiel 4.2.1 die Titelverbindung in einer Ausbeute von 0,66 g (89,3 % d. Th.) und mit einem Schmelzpunkt von > 300 ° C.

Beispiel 4.2.25

5-Amino-2-dimethylamino-tetralin-dihydrochlorid

Ausgehend von 0,65 g (0,0024 Mol) 5-Acetylamino-2-dimethylamino-tetralin-hydrochlorid (Beispiel 4.1.25) erhält man analog Beispiel 4.2.1 die Titelverbindung in einer Ausbeute von 0,30 g (63,2 % d. Th.) und mit einem Schmelzpunkt von ca. 300 ° C, der sich nach Umkristallisieren aus Methanol/Ether nicht ändert.

Beispiel 4.2.26

5-Amino-2-di-n-propylamino-tetralin-dihydrochlorid

Ausgehend von 2,70 g (0,0083 Mol) 5-Acetylamino-2-di-n-propylamino-tetralin-hydrochlorid (Beispiel 4.3.4) erhält man analog Beispiel 4.2.1 die Titelverbindung in einer Ausbeute von 2,7 g (100 % d. Th.) und mit einem Schmelzpunkt von 295° C, der sich beim Umkristallisieren aus Methanol/Ether nicht ändert.

Beispiel 4.2.27

5-Amino-2-methylamino-tetralin-dihydrochlorid

Ausgehend von 1,14 g (0,0045 Mol) 5-Acetylamino-2-methylamino-tetralin-hydrochlorid (Beispiel 4.4.1) erhält man analog Beispiel 4.2.1 die Titelverbindung in einer Ausbeute von 1,05 g (93,8 % d. Th.) und mit einem Schmelzpunkt von > 300° C, der sich nach Umkristallisieren aus Methanol/Ether nicht ändert.

Beispiel 4.2.28

5-Amino-2-diethylamino-tetralin-dihydrochlorid

Ausgehend von 0,50 g (0,0017 Mol) 5-Acetylamino-2-diethylamino-tetralin-hydrochlorid (Beispiel 4.3.3) erhält man analog Beispiel 4.2.1 die Titelverbindung in einer Ausbeute von 0,44 g (88,9 % d. Th.) und mit einem Schmelzpunkt von 277° C, der sich nach Umkristallisieren aus Methanol/Ether nicht ändert.

Beispiel 4.2.29

5-Amino-2-(N-methyl-N-n-propyl-amino)-tetralin-dihydrochlorid

Ausgehend von 1,10 g (0,0037 Mol) 5-Acetylamino-2-(N-methyl-N-n-propyl-amino)-tetralin-hydrochlorid (Beispiel 4.3.7) erhält man analog Beispiel 4.2.1 die Titelverbindung in einer Ausbeute von 0,62 g (57,4 % d. Th.) und mit einem Schmelzpunkt von 284° C, der sich nach dem Umkristallisieren aus Methanol/Wasser/Ether nicht ändert.

Beispiel 4.2.30

5-Amino-(N-ethyl-N-n-propyl-amino)-tetralin-dihydrochlorid

Ausgehend von 1,00 g (0,0032 Mol) 5-Acetylamino-2-(N-ethyl-N-n-propyl-amino)-tetralin-hydrochlorid (Beispiel 4.3.2) erhält man analog Beispiel 4.2.1 die Titelverbindung in einer Ausbeute von 0,90 g (92,9 % d. Th.) und mit einem Schmelzpunkt von 284° C, der sich nach dem Umkristallisieren aus Methanol/Ether nicht ändert.

Beispiel 4.2.31

5-Amino-2-(N-n-butyl-N-n-propyl-amino)-tetralin-dihydrochlorid

Ausgehend von 1,25 g (0,0037 Mol) 5-Acetylamino-2-(N-n-butyl-N-n-propyl-amino)-tetralin-hydrochlorid

(Beispiel 4.3.1) erhält man analog Beispiel 4.2.1 die Titelverbindung in einer Ausbeute von 1,10 g (89,4 % d. Th.) und mit einem Schmelzpunkt von 270° C, der sich nach Umkristallisieren aus Methanol/Ether nicht ändert.

Beispiel 4.2.32

5-Amino-2-[N-n-propyl-N-2-(2-thienyl)-ethyl-amino]tetralin

Ausgehend von 2,20 g (0,0062 Mol) 5-Acetylamino-2-[N-n-propyl-N-2-(2-thienyl)-ethyl-amino]-tetralin erhält man analog Beispiel 4.2.1 die Titelverbindung in Form des Hydrochlorids, das nicht zur Kristallisation gebracht werden konnte. Auch die analog Beispiel 4.1.10 durch Säulenchromatographie gereinigte Base ließ sich nicht zur Kristallisation bringen. Ausbeute an amorpher Substanz: 1,4 g (72,2 % d. Th.).

Beispiel 4.2.33

5-Amino-2-(7-phenylheptyl-amino)-tetralin-dihydrochlorid

Ausgehend von 0,60 g (1,45 mMol) 5-Acetylamino-2-(7-phenylheptyl-amino)-tetralin-hydrochlorid (Beispiel 4.4.2) erhält man analog Beispiel 4.2.1 die Titelverbindung in einer Ausbeute von 0,58 g (97,6 % d. Th.) und mit einem Schmelzpunkt von > 300° C, der sich nach dem Umkristallisieren aus Methanol/Wasser Ether nicht ändert.

Beispiel 4.2.34

5-Amino-2-(8-phenyloctyl-amino)-tetralin-dihydrochlorid

Ausgehend von 0,50 g (1,16 mMol) 5-Acetylamino-2-(8-phenyloctyl-amino)-tetralin-hydrochlorid (Beispiel 4.4.3) erhält man analog Beispiel 4.2.1 die Titelverbindung in einer Ausbeute von 0,45 g (91,3 % d. Th.) und mit einem Schmelzpunkt von > 300° C, der sich nach dem Umkristallisieren aus Methanol/Wasser Ether nicht ändert.

4.3 Verbindungen (3) und (4) aus (13) bzw. (13)

Beispiel 4.3.1

5-Acetylamino-2-(N-n-butyl-N-n-propyl-amino)-tetralin-hydrochlorid

2,83 g (0,010 Mol) 5-Acetylamino-2-n-propylamino-tetralin-hydrochlorid (Beispiel 4.1.1), 2,76 g (0,015 Mol) n-Butyliodid und 1,68 g (0,020 Mol) Natriumhydrogencarbonat werden in einer Mischung aus 20 ml absolutem Dimethylformamid und 30 ml absolutem Tetrahydrofuran 48 Stunden unter Rühren und Rückfluß gekocht (Zeitpunkt der vollständigen Umsetzung dünnschichtchromato- graphisch ermittelt). Die Reaktionsmischung wird im Rotationsverdampfer, zuletzt bei 95° C und vollem Wasserstrahlvakuum, eingedampft. Der Rückstand wird mit 50 ml Methylenchlorid und 50 ml Wasser geschüttelt und die abgetrennte wäßrige Phase noch zweimal mit je 15 ml Methylenchlorid extrahiert. Die vereinigten Extrakte werden mit 20 ml Wasser gewaschen, mit Natriumsulfat getrocknet und im Rotationsverdampfer, zuletzt bei 90° C und vollem Wasserstrahlvakuum, eingedampft. Der Rückstand wird mit 10 ml Ethanol gelöst und die Lösung mit 4,0 ml 2,5 n ethanolischer Salzsäure (0,01 Mol HCl) angesäuert. Nach Zugabe von Diethylether bis zur gerade eintretenden Trübung kristalliert die Titelverbindung. Nach Stehen über Nacht bei 5° C wird abgesaugt, mit einer Mischung aus Ethanol und Ether gewaschen und bei 80° C bis zur Gewichtskonstanz getrocknet.

Ausbeute 2,56 g (75,5 % d. Th.), Schmelzpunkt 217 °C. Nach dem Umkristallisieren aus Methanol/Ether ändert sich der Schmelzpunkt nicht.

Beispiel 4.3.2

5-Acetylamino-2-(N-ethyl-N-n-propyl-amino)-tetralin-hydrochlorid

Ausgehend von 2,83 g (0,01 Mol) 5-Acetylamino-2-npropylamino-tetralin-hydrochlorid (Beispiel 4.1.1) erhält man analog Beispiel 4.3.1 die Titelverbindung in einer Ausbeute von 2,09 g (67,2 % d. Th.) und mit einem Schmelzpunkt von 206 °C, der sich nach Umkristallisieren aus Methanol/Ether auf 209 °C erhöht.

Beispiel 4.3.3

5-Acetylamino-2-diethylamino-tetralin-hydrochlorid

Ausgehend von 2,00 g (0,0074 Mol) 5-Acetylamino-2-ethylamino-tetralin (Beispiel 4.1.12) erhält man analog Beispiel 4.3.1 unter Verwendung von 2,8 g (0,018 Mol) Ethyliodid und einer Reaktionszeit von 32 Stunden die Titelverbindung in einer Ausbeute von 0,90 g (43,2 % d. Th.) und mit einem Schmelzpunkt von 225 °C, der sich nach Umkristallisieren aus Methanol/Ether nicht ändert.

Beispiel 4.3.4

5-Acetylamino-2-di-n-propylamino-tetralin

Ausgehend von 2,46 g (0,010 Mol) 5-Acetylamino-2-n-propylamino-tetralin (Beispiel 4.1.1) erhält man analog Beispiel 4.3.1 die Titelverbindung als Eindampfungsrückstand der Methylenchlorid-Extrakte, der kristallisiert (2,7 g, 93,6 % d. Th.). Eine aus Aceton umkristallisierte Probe schmilzt bei 101 °C.

Beispiel 4.3.5

5-Acetylamino-2-[(N-benzyl-N-(7-phenylheptyl)-amino]tetralin

Ausgehend von 3,31 g (0,010 Mol) 5-Acetylamino-2-benzylamino-tetralin-hydrochlorid (Beispiel 4.1.16) erhält man analog Beispiel 4.3.1 unter Verwendung von 5,62 g (0,022 Mol) 7-Phenyl-heptylbromid und nach einer Reaktionszeit von 60 Stunden die Titelverbindung, die ohne weitere Reinigung einer katalytischen Entbenzylierung unterworfen wird (vergl. Beispiel 4.4.2).

Beispiel 4.3.6

5-Acetylamino-2-[N-benzyl-N-(8-phenyloctyl)-amino]tetralin

Ausgehend von 3,31 g (0,010 Mol) 5-Acetylamino-2-benzylamino-tetralin-hydrochlorid (Beispiel 4.1.16) erhält man analog Beispiel 4.3.1 unter Verwendung von 5,92 g (0,022 Mol) 8-Phenyl-octylbromid nach einer Reaktionszeit von 55 Stunden die Titelverbindung, die ohne weitere Reinigung einer katalytischen Entbenzylierung unterworfen wird (vergl. Beispiel 4.4.3).

Beispiel 4.3.7

5-Acetylamino-2-(N-methyl-N-n-propyl-amino)-tetralin

2,46 g (0,010 Mol) 5-Acetylamino-2-n-propylaminotetralin (Beispiel 4.1.1) werden mit 2,34 g (0,050 Mol) Ameisensäure und 2,2 ml 30-%iger Formalin-Lösung (0,022 Mol Formaldehyd) 30 Minuten auf dem siedenden Wasserbad erhitzt. Die Reaktionsmischung wird mit 50 ml Wasser, 50 ml Methylenchlorid und 2 ml konz. Ammoniak geschüttelt. Die abgetrennte wäßrige Phase wird noch zweimal mit je 15 ml Methylenchlorid extrahiert. Die vereinigten Methylenchlorid-Extrakte werden mit 20 ml Wasser gewaschen, mit Natriumsulfat getrocknet und nach dem Entfernen des Trockenmittels im Rotationsverdampfer, zuletzt bei 80 °C und vollem Wasserstrahlvakuum, eingedampft. Der Rückstand wird aus 5 ml Aceton und 20 ml Petrolether kristallisiert. Man erhält die Titelverbindung in einer Ausbeute von 1,80 g (69,2 % d. Th.) und mit einem Schmelzpunkt von 78 °C.

Beispiel 4.3.8

5-Acetylamino-2-methylamino-tetralin-hydrochlorid

Ausgehend von 0,28 g (0,0014 Mol) 5-Acetylamino-2-amino-tetralin, 0,33 g (0,007 Mol) Ameisensäure und 0,07 g 30-%iger Formalin-Lösung (0,7 mMol) $CH_2O$) erhält man das 5-Acetylamino-2-methylamino-tetralin analog Beispiel 4.3.7 neben seinem 2-Dimethylamino-Homologen und nicht umgesetzter Ausgangsverbindung. Die Titelverbindung wird durch Säulenchromatographie auf Kieselgel mit Methylenchlorid/Methanol/konz. Ammoniak 80:20:1 abgetrennt und als Hydrochlorid kristallisiert. Ausbeute 0,1 g (30,8 % d. Th.), Schmelzpunkt 253 °C.

4.4 Verbindungen (3) aus (4)

Beispiel 4.4.1

5-Acetylamino-2-methylamino-tetralin-hydrochlorid

1,40 g (0,0041 Mol) 5-Acetylamino-2-(N-benzyl-N-methylamino)-tetralin-hydrochlorid (Beispiel 4.1.26) werden in 50 ml Methanol in Gegenwart von 0,4 g 10 %-iger Palladium-Kohle bei 60 °C und 4 bar hydriert bis dieWasserstoffaufnahme zum Stillstand kommt. Nach Absaugen des Katalysators wird im Rotationsverdampfer, zuletzt bei 80 °C und vollem Wasserstrahlvakuum, eingedampft. Der Rückstand wird aus 15 ml Methanol und 15 ml Diethylether kristallisiert. Nach Absaugen, Waschen mit Methanol/Ether und Trocknen bei 80 °C erhält man die Titelverbindung in einer Ausbeute von 0,85 g (81,5 % d. Th.) und mit einem Schmelzpunkt von 253 °C, der sich nach Umkristallisieren aus Methanol/Ether nicht ändert.

Beispiel 4.4.2

5-Acetylamino-2-(7-phenylheptyl-amino)-tetralin-hydrochlorid

Ausgehend von dem nach Beispiel 4.3.5 hergestellten 5-Acetylamino-2-[N-benzyl-N-(7-phenylheptyl)-amino]tetralin erhält man analog Beispiel 4.4.1 die Titelverbindung in Form der Base, die analog Beispiel 4.1.1 in das Hydrochlorid überführt wird. Ausbeute 3,10 g (74,7 % d. Th.) über beide Synthesestufen, Schmelzpunkt 260 °C, unverändert nach Umkristallisieren aus Methanol/Ether.

Beispiel 4.4.3

5-Acetylamino-2-(8-phenyloctyl-amino)-tetralin-hydrochlorid

Ausgehend von dem nach Beispiel 4.3.6 hergestellten 5-Acetylamino-2-[N-benzyl-N-(8-phenyloctyl)-amino]tetralin erhält man analog Beispiel 4.4.3 die Titelverbindung in einer Ausbeute von 2,05 g (47,8 % d. Th. über beide Synthesestufen) und mit einem Schmelzpunkt von 259 °C, der sich nach Umkristallisieren aus Methanol/Ether nicht ändert.

4.5 Verbindungen (5) und (6) aus (8) bzw. (15) über Zwischenprodukte (7) bzw. (14)

Beispiel 4.5.1

5-Amino-2-di-n-propylamino-tetralin-dihydrochlorid

a) 4-Acetylamino-2-(N-propionyl-N-n-propyl-amino)tetralin

32,9 g (0,10 Mol) 5-Acetylamino-2-n-propylamino-tetralin-hydrochlorid (Beispiel 4.1.1) und 25,3 g (0,25 Mol) Triethylamin werden in 500 ml absolutem Methylenchlorid 1 Stunde kräftig gerührt. Anschließend werden unter weiterem Rühren und Rückflußkühlung 10,2 g (0,11 Mol) Propionylchlorid, gelöst in 50 ml absolutem Methylenchlorid, innerhalb von 30 Minuten eingetropft. Danach wird die Reaktionsmischung 1,5 Stunden unter Rückfluß gekocht. Nach dem Erkalten wird sie dreimal mit je 100 ml Wasser gewaschen, mit Natriumsulfat getrocknet und nach dem Abtrennen des Trockenmittels im Rotationsverdampfer, zuletzt bei vollem Wasserstrahlvakuum und 80 °C Badtemperatur, eingedampft.

b) 5-Amino-2-(N-propionyl-N-n-propyl-amino)-tetralin

Der Eindampfungsrückstand der Reaktionsstufe a) wird in einer Mischung aus 250 ml Ethanol und 250 ml 6 n Salzsäure gelöst und die Lösung 2,5 Stunden unter Rückfluß gekocht. Nach Abdestillieren der Hauptmenge des Ethanols im Rotationsverdampfer wird abgekühlt, mit 250 ml Methylenchlorid versetzt und unter Eiskühlung ammoniakalisch gemacht (100 ml konz. Ammoniak). Die Methylenchlorid-Phase wird abgetrennt und die wäßrige Phase noch zweimal mit jeweils 100 ml Methylenchlorid extrahiert. Die vereinigten Methylenchlorid-Extrakte werden mit 50 ml Wasser gewaschen, mit Natriumsulfat getrocknet und nach dem Entfernen des Trockenmittels im Rotationsverdampfer, zuletzt bei 80 °C und vollem Wasserstrahlvakuum, bis zur Gewichtskonstanz eingeengt. Rückstand: 25,5 g.

c) 5-Amino-2-di-n-propylamino-tetralin-dihydrochlorid

Der Eindampfungsrückstand der Reaktionsstufe b) wird in 400 ml absolutem Tetrahydrofuran gelöst. Die Lösung wird unter Rühren und Eiskühlung bei 5 - 10 °C in eine Suspension von 11,4 g (0,30 Mol) Lithiumaluminiumhydrid in 300 ml absolutem Tetrahydrofuran innerhalb eines Zeitraumes von etwa 45 Minuten eingetropft und die Reaktionsmischung anschließend 1 Stunde unter Rühren und Rückfluß gekocht. Nach dem Erkalten wird unter kräftigem Rühren und Eiskühlung tropfenweise mit 130 ml Wasser versetzt. Nach anschließender Zugabe von 1300 ml gesättigter Diammoniumtartrat-Lösung wird durchgeschüttelt und im Scheidetrichter getrennt. Die Tetrahydrofuran-Phase wird im Rotationsverdampfer eingedampft, die wäßrige Phase erst mit 250 ml, dann mit 100 ml Methylenchlorid extrahiert. Der Eindampfungsrückstand wird mit den vereinigten Methylenchlorid-Extrakten gelöst und die Lösung nach Waschen mit 50 ml Wasser und Trocknen mit Natriumsulfat sowie Abfiltrieren des Trockenmittels eingedampft (Rotationsverdampfer, zuletzt bei 80 °C und vollem Wasserstrahlvakuum). Der Rückstand (24 g) wird mit 20 ml Methanol und 90 ml 2,5 n ethanolischer Salzsäure gelöst und die Lösung mit 100 ml Diethylether versetzt. Es kristallisiert die Titelverbindung, die nach dem Stehen über ca. 12 Stunden bei 5 °C abgesaugt, mit Methanol/Ether gewaschen wird und erst an der Luft bei Raumtemperatur dann im Trockenschrank bei 80 °C, bis zur Gewichtskonstanz getrocknet wird. Man erhält die Titelverbindung über drei Reaktionsstufen ein einer Totalausbeute von 28,7 g (89,9 d. Th.) mit einem Schmelzpunkt von > 300 °C, der sich nach Umkristallisieren aus Methanol/Ether nicht ändert.

Beispiel 4.5.2

5-Amino-2-[N-(2-phenylethyl)-N-propyl-amino]-tetralin-dihydrochlorid

Ausgehend von 2,82 g (0,0091 Mol) 5-Acetylamino-2-(2-phenylethylamino)-tetralin (Beispiel 4.1.4) erhält man analog Beispiel 4.5.1 über die Zwischenprodukte 5-Acetylamino-2-[N-propionyl-N-(2-phenyl-ethyl)-amino]tetralin (3,3 g = 90,5 % d. Th., Schmelzpunkt 143-145 °C) und 5-Amino-2-[N-propionyl-2-(2-phenylethyl)amino]-tetralin (2,2 g = 75,0 % d. Th., Schmelzpunkt 165-166 °C) die Titelverbindung in einer Totalausbeute von 2,30 g (66,3 %) und mit einem Schmelzpunkt von 248-250 °C (nach Sintern bei 187-190 °C), der sich nach Umkristallisieren aus Methanol/Wasser/Ether nicht ändert.

Beispiel 4.5.3

5-Amino-2-[N-(3-phenylpropyl)-N-n-propyl-amino]tetralin-dihydrochlorid

Ausgehend von 2.80 g (0,0081 Mol) 5-Acetylamino-2-(3-phenylpropyl-amino)-tetralin (Beispiel 4.1.5) erhält man analog Beispiel 4.5.1 über die Zwischenprodukte 5-Acetylamino-2-[N-propionyl-N-(3-phenylpropyl)-amino]tetralin (3,5 g, Schmelzpunkt 148-150 °C) und 5-Amino-2-[N-propionyl-N-(3-phenylpropyl)-amino]-tetralin (2,8 g) die Titelverbindung in einer Totalausbeute von 2,91 g (90,5 % d. Th.) und mit einem Schmelzpunkt von 135-137 °C.

Beispiel 4.5.4

5-Amino-2-[N-(4-phenylbutyl-N-n-propyl-amino]tetralin-dihydrochlorid

Ausgehend von 3.50 g (0,0094 Mol) 5-Acetylamino-2-(4-phenylbutylamino)-tetralin-hydrochlorid (Beispiel 4.1.6) erhält man analog Beispiel 4.5.1 über die Zwischenprodukte 5-Acetylamino-2-[N-propionyl-N-(4-phenylbutyl)-amino]-tetralin und 2-Amino-2-[N-propionyl-N-(4-phenylbutyl)-amino]-tetralin die Titelverbindung in einer Totalausbeute von 1,95 g (50,4 % d. Th.) und mit einem Schmelzpunkt von 160 °C, der sich nach Umkristallisieren aus Methanol/Ether nicht ändert.

Beispiel 4.5.5

5-Amino-2-[N-(5-phenylpentyl)-N-n-propyl-amino]tetralin-dihydrochlorid

Ausgehend von 2,00 g (0,0052 Mol) 5-Acetylamino-2-(5-phenylpentylamino)-tetralin-hydrochlorid (Beispiel 4.1.7) erhält man analog Beispiel 4.5.1 über die Zwischenprodukte 5-Acetylamino-2-[N-propionyl-N-(5-phenylpentyl)-amino]-tetralin und 5-Amino-2-[N-propionyl-N-(5-phenylpentyl)-amino]tetralin die Titelverbindung in einer Totalausbeute von 1,7 g (77,3 % d. Th.) und mit einem Schmelzpunkt von 240 °C, der sich nach Umkristallisieren aus Methanol/Ether nicht ändert.

Beispiel 4.5.6

5-Amino-2-[N-(6-phenylhexyl)-N-n-propylamino]-tetralin-dihydrochlorid

Ausgehend von 2,20 g (0,0055 Mol) 5-Acetylamino-2-(6-phenylhexyl-amino)-tetralin-hydrochlorid (Beispiel 4.1.8) erhält man analog Beispiel 4.5.1 über die Zwischenprodukte 5-Acetylamino-2-[N-propionyl-N-(6-phenylhexyl)-amino]-tetralin und 5-Amino-2-[N-propionyl-N-(6-phenylhexyl)-amino]-tetralin die Titelverbindung in einer Totalausbeute von 1,9 g (79,2 % d. Th.) und mit einem Schmelzpunkt von 242 °C, der sich

nach Umkristallisieren aus Methanol/Ether nicht ändert.

Beispiel 4.5.7

5-Amino-2-[N-(7-phenylheptyl)-N-n-propyl-amino]-tetralin dihydrochlorid

Ausgehend von 2,00 g (4,82 mMol) 5-Acetylamino-2-(7-phenylheptyl-amino)-tetralin-hydrochlorid (Beispiel 4.4.2) erhält man analog Beispiel 4.5.1 über die Zwischenprodukte 5-Acetylamino-2-[N-propionyl-N-(7-phenylheptyl)-amino]-tetralin und 5-Amino-2-[N-propionyl-N-(7-phenylheptyl)-amino]-tetralin die Titelverbindung in einer Totalausbeute von 1,32 g (60,7 % d. Th.) und mit einem Schmelzpunkt von 231 °C, der sich nach Umkristallisieren aus Methanol/Ether nicht ändert.

Beispiel 4.5.8

5-Amino-2-[N-(8-phenyloctyl)-N-n-propyl-amino]-tetralin dihydrochlorid

Ausgehend von 1,00 g (2,33 mMol) 5-Acetylamino-2-(8-phenyloctyl-amino)-tetralin-hydrochlorid (Beispiel 4.4.3) erhält man analog Beispiel 4.5.1 über die Zwischenprodukte 5-Acetylamin-2-[N-propionyl-N-(8-phenyl-octyl)-amino]-tetralin und 5-Amino-2-[N-propionyl-N-(8-phenyloctyl)-amino]-tetralin die Titelverbindung in einer Totalausbeute von 0,55 g (50,7 % d. Th.) und mit einem Schmelzpunkt von 225 °C, der sich nach Umkristallisieren aus Methanol/Ether nicht ändert.

Beispiel 4.5.9

5-Amino-2-[N-(9-phenylnonyl)-N-n-propyl-amino]-tetralin-dihydrochlorid

Ausgehend von 3,20 g (0,0072 Mol) 5-Acetylamino-2-(9-phenylnonyl-amino)-tetralin-hydrochlorid (Beispiel 4.1.9) erhält man analog Beispiel 4.5.1 über die Zwischenprodukte 5-Acetylamino-2-[N-propionyl-N-(9-phenylnonyl)-amino]-tetralin und 5-Amino-2-[N-propionyl-N-(9-phenylnonyl)-amino]-tetralin die Titelverbindung in einer Totalausbeute von 2,4 g (69,3 % d. Th.) und mit einem Schmelzpunkt von 228 °C, der sich nach Umkristallisieren aus Methanol/Ether nicht ändert.

Beispiel 4.5.10

5-Amino-2-[N-(cis-cinnamyl)-N-n-propyl-amino]-tetralin-dihydrochlorid

Ausgehend von 0,50 g (0,0014 Mol) 5-Acetylamino-2-(cis-cinnamyl-amino)-tetralin-hydrochlorid (Beispiel 4.1.18) erhält man analog Beispiel 4.5.1 über die Zwischenprodukte 5-Acetylamino-2-[N-(cis-cinnamyl)-N-propionyl-amino]-tetralin und 5-Amino-[N-(cis-cinnamyl)-N-propionyl-amino]-tetralin die Titelverbindung in einer Totalausbeute von 0,22 g (41,9 % d. Th.) und mit einem Schmelzpunkt von 166 °C, der sich nach Umkristallisieren aus Methanol/Ether nicht ändert.

Beispiel 4.5.11

5-Amino-2-[N-(trans-cinnamyl)-N-n-propyl-amino]-tetralin-dihydrochlorid

Ausgehend von 1,00 g (0,0028 Mol) 5-Acetylamino-2-(trans-cinnamyl-amino)-tetralin-hydrochlorid (Beispiel 4.1.19) erhält man analog Beispiel 4.5.1 über die Zwischenprodukte 5-Acetylamino-2-[N-(trans-cinnamyl)-N-propionyl-amino]-tetralin und 5-Amino-2-[N-(trans-cinnamyl)-N-propionyl-amino]-tetralin die Titelverbindung in einer Totalausbeute von 0,28 g (26,7 % d. Th.) und mit einem Schmelzpunkt von 172° C, der sich nach Umkristallisieren aus Methanol-Ether nicht ändert.

Beispiel 4.5.12

2-[N-Allyl-N-n-propyl-amino]-5-amino-tetralin-dihydrochlorid

Ausgehend von 1,80 g (0,0064 Mol) 5-Acetylamino-2-allylamino-tetralin-hydrochlorid (Beispiel 4.1.13) erhält man analog Beispiel 4.5.1 über die Zwischenprodukte 5-Acetylamino-2-(N-allyl-N-propionyl-amino)-tetralin und 2-(N-Allyl-N-propionyl-amino)-5-amino-tetralin die Titelverbindung in einer Totalausbeute von 1,15 g (57.5 % d. Th.) und mit einem Schmelzpunkt von 268° C, der sich nach Umkristallisieren aus Methanol/Ether nicht ändert.

4.6 Verbindungen (12) und (9) aus (3) bzw. (4)

Beispiel 4.6.1

5-Ethylamino-2-n-propylamino-tetralin-dihydrochlorid

1,00 g (0,0041 Mol) 5-Acetylamino-2-n-propylamino-tetralin (Beispiel 4.1.1) werden in 10 ml absolutem Tetrahydrofuran gelöst. Die Lösung wird unter Rühren und Eiskühlung in eine Suspension von 0,31 g (0,0081 Mol) Lithiumaluminiumhydrid in 10 ml absolutem Tetrahydrofuran eingetropft. Die Reaktionsmischung wird anschließend 2 Stunden unter Rückfluß gekocht. Dann wird unter Rühren und Eiskühlung mit 1,0 ml Wasser hydrolysiert. Nach Zugabe von 30 ml gesättigter Diammonium-tartrat-Lösung wird aufgearbeitet analog Beispiel 4.5.1, c). Man erhält die Titelverbindung in Form der Base, die unter Verwendung von 50 g Kieselgel und Methylenchlorid/Methanol/konz. Ammoniak 80:20:1 als Laufmittel durch Säulenchromatographie gereinigt wird. Die gereinigte Base wird analog Beispiel 4.1.1 unter Verwendung von 2 Äquivalenten ethanolischer Salzsäure in das Hydrochlorid überführt, das man in einer Ausbeute von 0,18 g (14,4 % d. Th.) isoliert. Schmelzpunkt 190° C, unverändert nach Umkristallisieren aus einer Ethanol/Ether-Mischung.

Beispiel 4.6.2

2-Di-n-propylamino-5-n-propylamino-tetralin-dihydrochlorid

Ausgehend von 1,00 g (0,0033 Mol) 2-Di-n-propylamino-5-propionyl-amino-tetralin (Beispiel 4.7.3, Basenform) erhält man analog Beispiel 4.6.1 die Titelverbindung in Form der säulenchromatographisch gereinigten Base. Diese wird analog Beispiel 4.1.1 unter Verwendung von 2 Äquivalenten ethanolischer 2,5 n Bromwasserstoffsäure als Dihydrobromid kristallisiert. Ausbeute 0,35 g (23,5 % d. Th.), Schmelzpunkt 203° C, unverändert nach Umkristallisieren aus Ethanol/Ether.

4.7 Verbindungen (10) und (16) aus (4) bzw. (3)

Beispiel 4.7.1

5-(N-Acetyl-N-methyl-amino)-2-di-n-propylamino-tetralin

0,38 g einer 75 %-igen Suspension von Natriumhydrid in Paraffinöl (0,012 Mol) werden in eine

Mischung aus 40 ml absolutem Dimethylformamid und 5 ml absolutem Tetrahydrofuran eingetragen. Anschließend werden 2,88 g (0,010 Mol) 5-Acetyl-2-di-n-propylamino-tetralin (Beispiel 4.3.4) - gelöst in 10 ml absolutem Dimethylformamid - unter Rühren und Eiskühlung eingetragen. Dann wird bei Raumtemperatur bis zur vollständigen Umsetzung weiter gerührt (einige Stunden; DC-Kontrolle). Nach Eindampfen im Rotationsverdampfer, zuletzt bei 95° C und vollem Wasserstrahlvakuum, wird der Rückstand mit 50 ml Wasser und 50 ml Methylenchlorid geschüttelt. Die abgetrennte wäßrige Phase wird noch zweimal mit jeweils 15 ml Methylenchlorid extrahiert. Die vereinigten Extrakte werden mit 20 ml Wasser gewaschen, mit Natriumsulfat getrocknet und nach dem Abfiltrieren des Trockenmittels und Rotationsverdampfer, zuletzt bei 80° C und vollem Wasserstrahlvakuum, eingedampft. Der Rückstand läßt sich weder als Base noch als Salz kristallisieren. Daher wird durch Säulenchromatographie auf 150 g Kieselgel mit Methylenchlorid/Methanol/konz. Ammoniak 90:10:0,5 gereinigt. Auch die so gereinigte Substanz (2,8 g, 92,6 % d. Th.) kristallisiert nicht, obwohl sie dünnschichtchromatographisch (Kieselgel, oben genanntes Lösungsmittelgemisch, $R_f$ = 0.64) und NMR-spektroskopisch rein ist.

Beispiel 4.7.2

5-(N-Acetyl-N-ethyl-amino)-2-di-n-propylamino-tetralin

Ausgehend von 2,88 g (0,010 Mol) 5-Acetylamino-2-n-di-propylamino-tetralin (Beispiel 4.3.4) und 1,87 g (0,012 Mol) Ethyliodid erhält man analog Beispiel 4.7.1 die Titelverbindung in Form eines nicht kristallisierenden Honigs in einer Ausbeute von 2,65 g (83,7 % d. Th.) und mit einem $R_f$-Wert von 0,65 (Lösungsmittelgemisch wie in 4.7.1 angegeben).

Beispiel 4.7.3

5-(N-Acetyl-N-n-propyl-amino)-2-di-n-propylamino-tetralin

Ausgehend von 2,88 g (0,010 Mol) 5-Acetylamino-2-di-n-propylamino-tetralin (Beispiel 4.3.4) und 2,04 g (0,012 Mol) n-Propyliodid erhält man analog Beispiel 4.7.1 die Titelverbindung in Form eines nicht kristallisierenden Honigs in einer Ausbeute von 2,70 g (89,4 % d. Th.) und mit einem $R_f$-Wert von 0,67 (Lösungsmittelgemisch wie unter 4.7.1 angegeben).

Beispiel 4.7.4

5-(N-Acetyl-N-n-butyl-amino)-2-di-n-propylamino-tetralin

Ausgehend von 2,88 g (0,010 Mol) 5-Acetylamino-2-di-n-propylamino-tetralin (Beispiel 4.3.4) und 2,2 g (0,012 Mol) n-Butyliodid erhält man analog Beispiel 4.7.1 die Titelverbindung in Form eines nicht kristallisierenden Honigs in einer Ausbeute von 2,9 g (87,8 % d. Th.) und mit einem $R_f$-Wert von 0,69 (Lösungsmittelgemisch wie unter 4.7.1 angegeben).

Beispiel 4.7.5

5-(N-Acetyl-N-allyl-amino)-2-di-n-propylamino-tetralin

Ausgehend von 2,88 g (0,010 Mol) 5-Acetylamino-2-di-n-propylamino-tetralin (Beispiel 4.3.4) und 1,45 g (0,012 Mol) Allylbromid erhält man analog Beispiel 4.7.1 die Titelverbindung in Form eines nicht kristallisierenden Sirups in einer Ausbeute von 2,25 g (68,4 % d. Th.) mit einem $R_f$-Wert von 0,66 (Lösungsmittelgemisch wie unter 4.7.1 angegeben).

Beispiel 4.7.6

5-(N-Acetyl-N-propargyl-amino)-2-di-n-propylamino-tetralin

Ausgehend von 2,88 g (0,010 Mol) 5-Acetylamino-2-di-n-propylamino-tetralin (Beispiel 4.3.4) und 1,43 g (0,012 Mol) Propargylbromid erhält man analog Beispiel 4.7.1 die Titelverbindung in Form eines nicht kristallisierenden sehr zähflüssigen Öls in einer Ausbeute von 2,33 g (71,4 % d. Th.) und mit einem $R_f$-Wert von 0,66 (Lösungsmittelgemisch wie unter 4.7.1 angegeben.

Beispiel 4.7.7

5-(N-Acetyl-N-benzyl-amino)-2-di-n-propylamino-tetralin

Ausgehend von 2,55 g (0,010 Mol) 5-Acetylamino-2-di-n-propylamino-tetralin (Beispiel 4.3.4) und 1,50 g (0,012 Mol) Benzylchlorid erhält man analog Beispiel 4.7.1 die Titelverbindung in Form eines Honigs in einer Ausbeute von 3,0 g (79,0 % d. Th.).

Beispiel 4.7.8

5-(N-Acetyl-N-methyl-amino)-2-n-propylamino-tetralin-hydrochlorid

Ausgehend von 1,15 g (0,0047 Mol) 5-Acetylamino-2-n-propylamino-tetralin (Beispiel 4.1.1) und 0,54 g (0,0049 Mol) Methansulfonsäure-methylester erhält man analog Beispiel 4.7.1 die Base der Titelverbindung, die nach Säulenchromatographie als Hydrochlorid kristallisiert wird. Ausbeute 0,66 g (47,1 % d. Th.), Schmelzpunkt 252-253 °C.

Beispiel 4.7.9

5-(N-Acetyl-N-ethyl-amino)-2-n-propylamino-tetralin-hydrochlorid

Ausgehend von 2,35 g (0,0095 Mol) 5-Acetylamino-2-n-propylamino-tetralin (Beispiel 4.1.1) und 1,07 g (0,0098 Mol) Ethylbromid erhält man analog Beispiel 4.7.1 die Base der Titelverbindung, die als Hydrochlorid kristallisiert wird. Ausbeute 1,60 g (54,2 % d. Th.), Schmelzpunkt 257 °C.

Beispiel 4.7.10

5-(N-Acetyl-N-allyl-amino)-2-n-propylamino-tetralin-hydrochlorid

Ausgehend von 1,23 g (0,0050 Mol) 5-Acetylamino-2-n-propylamino-tetralin (Beispiel 4.1.1) und 0,64 g (0,0053 Mol) Allylbromid erhält man analog Beispiel 4.7.1 die Base der Titelverbindung, die als Hydrochlorid kristallisiert wird. Ausbeute 0,82 g (49,4 % d. Th.), Schmelzpunkt 237-238 °C.

Beispiel 4.7.11

5-(N-Acetyl-N-propargyl-amino)-2-n-propylamino-tetralin-hydrochlorid

Ausgehend von 1,23 g (0,005 Mol) 5-Acetylamino-2-n-propylamino-tetralin (Beispiel 4.1.1) und 0,63 g

(0,0053 Mol) Propargylbromid erhält man analog Beispiel 4.7.1 die Base der Titelverbindung, die als Hydrochlorid kristallisiert wird. Ausbeute 0,89 g (55,6 % d. Th.), Schmelzpunkt 211 °C.

Beispiel 4.7.12

5-(N-Acetyl-N-cinnamyl-amino)-2-n-propylamino-tetralin-hydrochlorid

Ausgehend von 1,23 g (0,005 Mol) 4-Acetylamino-2-n-propylamino-tetralin (Beispiel 4.1.1) und 0,85 g (0,0053 Mol) Cinnamylbromid erhält man analog Beispiel 4.7.1 die Base der Titelverbindung, die als Hydrochlorid kristallisiert wird. Ausbeute 1,63 g (81,5 % d. Th.) Schmelzpunkt 233 °C.

4.8 Verbindungen (4) und (10) aus (5) und (9)

Beispiel 4.8.1

5-Acetylamino-2-[N-(4-phenylbutyl)-N-n-propyl-amino]-tetralin

0,90 g (0,0022 Mol) 5-Amino-2-[N-(4-phenylbutyl)-N-n-propyl-amino]-tetralin-dihydrochlorid (Beispiel 4.5.4) und 1,00 g (0,0099 Mol) Triethylamin werden in 15 ml absolutem Methylenchlorid unter Rühren und Eiskühlung mit 0,20 g (2,55 mMol) Acetylchlorid in 5 ml absolutem Methylenchlorid versetzt. Nach 3 Stunden bei Raumtemperatur oder 30 Minuten Kochen unter Rückfluß wird dreimal mit je 5 ml Wasser extrahiert, mit Natriumsulfat getrocknet und im Rotationsverdampfer, zuletzt bei 80 °C und vollem Wasserstrahlvakuum, eingedampft. Der Rückstand wird aus Diethylether/Petrolether kristallisiert. Man erhält 0,61 g (73,5 % d. Th.) der Titelverbindung mit einem Schmelzpunkt von 65 °C, der sich nach Umkrisallisieren aus Diethylether/Petrolether nicht ändert.

Beispiel 4.8.2

5-Acetylamino-2-[N-(5-phenylpentyl)-N-n-propyl-amino]-tetralin

Ausgehend von 1,20 g (2,83 mMol) 5-Amino-2-[N-(5-phenylpentyl)-N-n-propyl-amino]-tetralin-dihydrochlorid (Beispiel 4.5.5) erhält man analog Beispiel 4.8.1 unter Verwendung von Acetanhydrid an Stelle von Acetylchlorid die Titelverbindung in einer Ausbeute von 0,90 g (80,9 % d. Th.) und mit einem Schmelzpunkt von 70 °C, der sich nach Umkristallisieren aus Ether/Petrolether nicht ändert.

Beispiel 4.8.3

2-Di-n-propylamino-5-propionylamino-tetralin-hydrochlorid

Ausgehend von 1,60 g (0,005 Mol) 2-Amino-2-di-n-propylamino-tetralin-dihydrochlorid (Beispiel 4.5.1) erhält man analog Beispiel 4.8.1 unter Verwendung von Propionylchlorid die Titelverbindung in Form der Base, die analog Beispiel 4.1.1 in das Hydrochlorid überführt wird. Ausbeute 1,27 g (74,7 % d. Th.), Schmelzpunkt 221 °C. Umkristallisieren aus Methanol/Ether erhöht den Schmelzpunkt auf 228 °C.

Beispiel 4.8.4

5-Chloracetylamino-2-di-n-propylamino-tetralin-hydrochlorid

Ausgehend von 1,60 g (0,005 Mol) 2-Amino-2-di-n-propylamino-tetralin-dihydrochlorid (Beispiel 4.5.1)

erhält man analog Beispiel 4.8.1 unter Verwendung von Chloracetylchlorid die Titelverbindung in Form der Base, die analog 4.1.1 in das Hydrochlorid überführt wird. Ausbeute 1,10 g (61,1 % d. Th.), Schmelzpunkt 241 °C. Umkristallisieren aus Methanol/Ether erhöht den Schmelzpunkt auf 243 °C.

Beispiel 4.8.5

2-Di-n-propylamino-5-(trifluoracetylamino)-tetralin-hydrochlorid

Ausgehend von 1,60 g (0,005 Mol) 5-Amino-2-di-n-propylamino-tetralin-dihydrochlorid (Beispiel 4.5.1) erhält man analog Beispiel 4.8.1 unter Verwendung von Trifluoracetanhydrid die Titelverbindung in Form der Base, die analog Beispiel 4.1.1 als Hydrochlorid kristallisiert wird. Ausbeute 0,70 g (37,0 % d. Th.). Schmelzpunkt 233 °C, unverändert nach Umkristallisieren aus Methanol/Ether.

Beispiel 4.8.6

5-Benzoylamino-2-di-n-propylamino-tetralin

Ausgehend von 3,19 g (0,01 Mol) 5-Amino-2-di-n-propylamino-tetralin-dihydrochlorid (Beispiel 4.5.1) erhält man analog Beispiel 4.8.1 unter Verwendung von Benzoylchlorid die Titelverbindung (3,6 g Rohprodukt), die aus Diethylether/Petrolether kristallisiert wird. Ausbeute 2,8 g (79,9 % d. Th.), Schmelzpunkt 97 °C.

Beispiel 4.8.7

2-Di-n-propylamino-5-formylamino-tetralin-hydrochlorid

1,23 g (0,005 Mol) 5-Amino-2-di-n-propylamino-tetralin, Base, (Beispiel 4.5.1) werden mit 2,65 g (0,058 Mol) Ameisensäure und 5,60 g Acetanhydrid (0,055 Mol) 2 Stunden unter Rückfluß gekocht. Anschließend wird die Reaktionsmischung in 50 ml Eiswasser gegossen und mit 15 ml konz. Ammoniak versetzt. Die ausgeschiedene Base wird 3 mal mit Methylenchlorid (25, 15, 15 ml) extrahiert und die vereinigten Extrakte mit Wasser gewaschen, mit Natriumsulfat getrocknet und nach dem Abfiltrieren des Trockenmittels im Rotationsverdampfer, zuletzt bei 80 °C und vollem Wasserstrahlvakuum, eingedampft. Der Rückstand, bestehend aus der Base der Titelverbindung, wird analog Beispiel 4.1.1 in das Hydrochlorid überführt, das man in einer Ausbeute von 1,15 g (74,0 % d. Th.) und mit einem Schmelzpunkt von 250 °C erhält.

Beispiel 4.8.8

2-Di-n-propylamino-5-methoxycarbonylamino-tetralin-hydrochlorid

Ausgehend von 1,60 g (0,005 Mol) 5-Amino-2-di-n-propylamino-tetralin-dihydrochlorid (Beispiel 4.5.1) erhält man analog Beispiel 4.8.1 unter Verwendung von Chlorameisensäure-methylester die Titelverbindung in Form der Base. Diese wird durch Säulenchromatographie an 130 g Kieselgel unter Verwendung von Methylenchlorid/Methanol/konz. Ammoniak 90:10:0,5 als Elutionsmittel gereinigt und dann analog Beispiel 4.1.1 als Hydrochlorid kristallisiert. Ausbeute 1,0 g (58,8 % d. Th., Schmelzpunkt 210 °C, unverändert nach Umkristallisieren aus Methanol/Ether.

Beispiel 4.8.9

5-(Carbamoylamino)-2-di-n-propylamino-tetralin-hydrochlorid

1,60 g (0,005 Mol) 5-Amino-di-n-propylamino-tetralin-hydrochlorid (Beispiel 4.5.1) werden in 20 ml Wasser gelöst und die Lösung nach Zugabe von 1,10 g (0,013 Mol) Kaliumcyanat 15 Minuten auf 80 °C erwärmt. Nach Erkalten wird mit konz. Ammoniak ammoniakalisch gemacht und dreimal mit Essigester extrahiert. Die vereinigten Extrakte werden mit Wasser gewaschen, mit Natriumsulfat getrocknet und im Rotationsverdampfer, zuletzt bei 80 °C und vollem Wasserstrahlvakuum, eingedampft. Man erhält die Titelverbindung in Form der Base, die analog Beispiel 4.8.8 chromatographisch gereinigt und als Hydrochlorid kristallisiert wird. Ausbeute 1,12 g (68,7 % d. Th.), Schmelzpunkt 265 °C, unverändert nach Umkristallisieren aus Methanol/Ether.

4.9 Verbindungen (12) und (9) aus (17) bzw. (7)

Beispiel 4.9.1

2,5-Di-(n-propylamino)-tetralin-dihydrochlorid

a) 5-Acetylamino-2-propionylamino-tetralin

Ausgehend von 12,3 g (0,06 Mol) 5-Acetylamino-2-amino-tetralin (bekannte Substanz, hergestellt durch Entbenzylierung von 5-Acetylamino-2-benzylamino-tetralin (Beispiel 4.1.16) analog Beispiel 4.4.1) erhält man durch Propionylierung analog Beispiel 4.5.1 a) die Titelverbindung in einer Ausbeute von 13,5 g (86,3 % d. Th.).

b) 5-Amino-2-propionylamino-tetralin

Ausgehend von 13,5 (0,052 Mol) 5-Acetylamino-2-propionylamino-tetralin (siehe oben) erhält man durch selektive Abspaltung der N-Acetyl-Gruppe analog Beispiel 4.5.1 b) die Titelverbindung in einer Ausbeute von 7,9 g (69,8 % d. Th.)

c) 2,5-Di-(propionylamino)-tetralin

Ausgehend von 0,87 g (0,004 Mol) 5-Amino-2-propionylamino-tetralin (siehe oben) erhält man durch Propionylierung analog Beispiel 5.5.1 a) die Titelverbindung in einer Ausbeute von 0,98 g (89,9 % d. Th.) und mit einem Schmelzpunkt von 270 °C.

d) 2,5-Di-(n-propylamino)-tetralin-dihydrochlorid

Ausgehend von 0,82 g (0,003 Mol) 2,5-Di-(propionylamino)-tetralin (siehe oben) erhält man durch Reduktion mit Lithiumaluminiumhydrid (0,46 g, 0,012 Mol) analog Beispiel 4.5.1 c) zunächst die Base der Titelverbindung. Diese wird durch Säulenchromatographie an 70 g Kieselgel unter Verwendung von Methylenchlorid/Methanol/Ammoniak 90:10:0,5 gereinigt und als Dihydrochlorid kristallisiert. Ausbeute 0,42 g (43,8 % d. Th.), Schmelzpunkt 234 °C, unverändert nach Umkristallisieren aus Ethanol/Ether.

Beispiel 4.9.2

5-n-Butylamino-2-n-propylamino-tetralin-dihydrochlorid

Ausgehend von 0,87 g (0,004 Mol) 5-Amino-2-propionylamino-tetralin erhält man über das Zwischenprodukt 5-Butyroylamino-2-propionylamino-tetralin analog Beispiel 4.9.1 c) und d) die Titelverbindung in einer Totalausbeute von 0,49 g (36,8 % d. Th.) und mit einem Schmelzpunkt von 256 °C, der sich nach Umkristallisieren aus Ethanol/Ether nicht ändert.

Beispiel 4.9.3

5-Benzylamino-2-n-propylamino-tetralin-dihydrochlorid

Ausgehend von 2,60 g (0,012 Mol) 5-Amino-2-propionylamino-tetralin erhält man über das Zwischenprodukt 5-Benzylamino-2-propionylamino-tetralin analog Beispiel 4.9.1 c) und d) die Titelverbindung in einer Totalausbeute von 2,01 g (44,7 % d. Th.) und mit einem Schmelzpunkt von 259 °C, der sich nach Umkristallisieren aus Ethanol/Ether nicht ändert.

Beispiel 4.9.4

5-(2-Phenylethyl-amino)-2-n-propylamino-tetralin-dihydrochlorid

Ausgehend von 0,87 g (0,004 Mol) 5-Amino-2-propionylamino-tetralin analog Beispiel 4.9.1 c) und d) die Titelverbindung in einer Totalausbeute von 0,55 g (35,1 % d. Th.) und mit einem Schmelzpunkt von 245 °C, der sich nach Umkristallisieren aus Methanol/Ether nicht ändert.

Beispiel 4.9.5

5-(3-Phenylpropyl-amino)-2-n-propylamino-tetralin-dihydrobromid

Ausgehend von 0,87 g (0,004 Mol) 5-Amino-2-propionylamino-tetralin erhält man über das Zwischenprodukt 5-(3-Phenylpropionyl-amino-2-propionylamino-tetralin analog Beispiel 4.9.1 c) und d) die Titelverbindung in einer Totalausbeute von 0,124 g (6,4 % d. Th.) und mit einem Schmelzpunkt von 195 °C.

Beispiel 4.9.6

2-Di-n-propylamino-5-ethylamino-tetralin-hydrochlorid

Ausgehend von 28,3 g (0,10 Mol) 5-Acetylamino-2-(N-propionyl-N-n-propyl-amino)-tetralin (Beispiel 4.5.1 a)) erhält man durch Reduktion mit 12,5 g (0,33 Mol) Lithiumaluminiumhydrid analog Beispiel 4.9.1 d) die Titelverbindung zunächst in Form der Base in einer Ausbeute von 20,1 g (73,2 % d. Th.). Die Base ließ sich weder als solche noch als Dihydrochlorid kristallisieren. Eine Probe kristallisierte jedoch als Monohydrochlorid aus Ethanol/Ether. Schmelzpunkt 187 °C, unverändert nach Umkristallisieren aus Ethanol/Ether.

4.10 Verbindungen (12) und (9) aus (16) bzw. (10)

Beispiel 4.10.1

5-Methylamino-2-n-propylamino-tetralin-dihydrochlorid

0,50 g (1,92 mMol) 5-(N-Acetyl-N-methyl-amino)-2-n-propylamino-tetralin-hydrochlorid (Beispiel 4.7.8) werden mit 12.5 g (0,22 Mol) Kaliumhydroxid in 40 ml Diethylenglykol 24 Stunden bei 150 °C gerührt.

Anschließend wird abgekühlt, mit 400 ml Wasser verdünnt und dreimal mit Essigester (50, 15 und 15 ml) extrahiert. Die vereinigten Extrakte werden mit 20 ml Wasser gewaschen, mit Natriumsulfat getrocknet und -nach dem Abfiltrieren des Trockenmittels - im Rotationsverdampfer, zuletzt bei 80° C und vollem Wasserstrahlvakuum, eingedampft. Der Rückstand wird analog Beispiel 4.1.1 als Hydrochlorid kristallisiert. Man erhält die Titelverbindung in einer Ausbeute von 0,39 g (69,7 % d. Th.) und mit einem Schmelzpunkt von 245° C, der sich beim Umkristallisieren aus Ethanol/Ether nicht ändert.

Beispiel 4.10.2

5-Propargylamino-2-n-propylamino-tetralin-dihydrochlorid

0,48 g (0,0015 Mol) 5-(N-Acetyl-N-propargyl-amino)-2-n-propylamino-tetralin-hydrochlorid (Beispiel 4.7.11) werden in 10 ml Ethanol und 30 ml 6 n Salzsäure 16 Stunden unter Rückfluß gekocht. Anschließend wird im Rotationsverdampfer, zuletzt bei 80° C und vollem Wasserstrahlvakuum eingedampft. Der Rückstand wird mit 50 ml Wasser, 50 ml Methylenchlorid und überschüssigem Ammoniak geschüttelt. Die abgetrennte wäßrige Phase wird noch zweimal mit je 15 ml Methylenchlorid extrahiert. Die vereinigten Methylenchlorid-Extrakte werden mit 20 ml Wasser gewaschen und im Rotationsverdampfer wie oben beschrieben eingedampft. Der Rückstand wird analog Beispiel 4.1.1 als Dihydrochlorid kristallisiert. Ausbeute 0,12 g (25,2 % d. Th.), Schmelzpunkt 198° C, unverändert nach Umkristallisieren aus Ethanol/Ether.

4.11 Verbindungen (11) aus (10)

Beispiel 4.11.1

2-Di-n-propylamino-5-(N-ethyl-N-methyl-amino)-tetralin-dihydrochlorid

1,1 g (3,63 mMol) 5-(N-Acetyl-N-methyl-amino)-2-dimethylamino-tetralin (Beispiel 4.7.1) werden mit 0,49 g (0,013 Mol) Lithiumaluminiumhydrid analog Beispiel 4.5.1° C) reduziert. Die erhaltene Base der Titelverbindung wird durch Säulenchromatographie auf 100 g Kieselgel mit Methylenchlorid/Methanol/konz. Ammoniak 95:5:0,1 gereinigt und analog Beispiel 4.1.1 als Dihydrochlorid kristallisiert. Ausbeute 0,47 g (35,8 d. Th.),Schmelzpunkt 169° C, unverändert nach dem Umkristallisieren aus Methanol/Ether.

Beispiel 4.11.2

5-Diethylamino-2-di-n-propylamino-tetralin-dihydrochlorid

Ausgehend von 2,20 g (0,00695 Mol) 5-(N-Acetyl-N-ethylamino)-2-di-n-propylamino-tetralin (Beispiel 4.7.2) erhält man analog Beispiel 4.11.1 die Titelverbindung in einer Ausbeute von 1,40 g (53,6 % d. Th.) und mit einem Schmelzpunkt von 213° C, der sich nach Umkristallisieren aus Methanol/Ether nicht ändert.

Beispiel 4.11.3

5-(N-Ethyl-N-methyl-amino)-2-n-propylamino-tetralin-dihydrochlorid

Ausgehend von 0,56 g (1,89 mMol) 5-(N-Acetyl-N-methyl-amino)-2-n-propylamino-tetralin-hydrochlorid (Beispiel 4.7.8), das in die Base umgewandelt wird, erhält man durch Reduktion in 20 ml absolutem Tetrahydrofuran analog Beispiel 4.5.1 c), jedoch unter Verwendung von 1,90 ml 2-molarer Diboran-Lösung in Tetrahydrofuran (THF) als Reduktionsmittel, die Titelverbindung als Base. Diese wird nach Reinigung durch Säulenchromatographie (50 g Kieselgel, Methylenchlorid/Methanol/konz. Ammoniak 90:10:00,5) analog Beispiel 4.1.1 als Dihydrochlorid kristallisiert, das man in einer Ausbeute von 0,33 g (54.6 % d. Th.) und mit einem Schmelzpunkt von 200 °C erhält.

Beispiel 4.11.4

5-Diethylamino-2-n-propylamino-tetralin-dihydrochlorid

Ausgehend von 1.03 g (0,0033 Mol) 5-(N-Acetyl-N-ethylamino)-2-n-propylamino-tetralin-hydrochlorid (Beispiel 4.7.8), das in die Base umgewandelt wird, erhält man durch Reduktion in 30 ml absolutem Tetrahydrofuran analog Beispiel 4.5.1 c), jedoch unter Verwendung von 3,3 ml 2-molarer Diboran-dimethylsulfid-Komplex-Lösung in Tetrahydrofuran als Reduktionsmittel, die Titelverbindung in der Basenform. Diese wird durch Filtrieren über 16 g Aluminiumoxid (Aktivitätsstufe III, neutral) unter Verwendung von Methylenchlorid als Lösungsmittel gereinigt und analog Beispiel 4.1.1 als Dihydrochlorid kristallisiert, das man in einer Ausbeute von 0,54 g (49,1 %) und mit einem Schmelzpunkt von 201 °C erhält.

4.12 Verbindungen (9) aus (11)

Beispiel 4.12.1

2-Di-n-propylamino-5-methylamino-tetralin-hydrochlorid

a) 5-(N-Benzoyl-N-methyl-amino)-2-di-n-propylamino-tetralin

Ausgehend von 3,50 g (0,010 Mol) 5-Benzoylamino-2-di-n-propylamino-tetralin (Beispiel 4.8.6) erhält man durch Methylierung analog Beispiel 4.7.1 die Titelverbindung in einer Ausbeute von 3,30 g (90,5 %).

b) 5-(N-Benzyl-N-methyl-amino)-2-di-n-propylamino-tetralin

Man erhält die Titelverbindung durch Reduktion von 5-(N-Benzoyl-N-methyl-amino)-2-di-n-propylamino-tetralin (siehe oben) mit Diboran analog Beispiel 4.11.4.

c) 2-Di-n-propylamino-5-methylamino-tetralin-hydrochlorid

Man erhält die Titelverbindung in Form der Base durch katalytische Entbenzylierung von 5-(N-Benzyl-N-methyl)-2-di-n-propylamino-tetralin (siehe oben) analog Beispiel 4.4.1. Die Base wird analog Beispiel 4.1.1 in das Monohydrochlorid überführt. Totalausbeute über 3 Reaktionsstufen 1,2 g (40,4 % d. Th.), Schmelzpunkt 218 °C, unverändert nach Umkristallisieren aus Ethanol/Ether.

4.13 Enantiomerenreine Verbindungen

Beispiel 4.13.1

(R/S)- und (R/R)-5-Acetylamino-2-(1-phenylethylamino)-tetralin

a) 5-Acetylamino-2-(1-phenylethylamino)-tetralin (Diastereomerengemisch)

Ausgehend von 16,24 g (0,08 Mol) 5-Acetylamino-2-tetralon, 240 ml Methanol, 19,0 g (0,12 Mol) (R)-1-Phenylethylamin-hydrochlorid und 6,0 g (0,096 Mol) Natriumcyanborhydrid erhält man analog Beispiel 4.1.11 die Titelverbindung, die aus 75 ml Toluol kristallisiert wird. Ausbeute 18,5 g (74,9 % d. Th.), Schmelzpunkt 158 °C. Sie besteht aus etwa gleichen Teilen der (R/R)- und (S/R)-Diastereomeren.

b) (R/S)-5-Acetylamino-2-(1-phenylethylamino)-tetralin (D-Mandelat und freie Base)

18,5 g (0,060 Mol) des Diastereomerengemischs (siehe oben) werden in 300 ml Acetonitril mit 9,13 g (0,060 Mol) D-Mandelsäure gelöst. Beim Stehen bei Raumtemperatur (24 Stunden) erhält man eine Kristallfraktion, in der die (R/S)-Form angereichert ist (10,6 g). Umkristallisieren aus 115 ml Acetonitril gibt 8,65 g gereinigte (R/S)-Form mit einem Schmelzpunkt von 165 °C.

Das kristallisierte D-Mandelat der (R/S)-Form (8,65 g) wird mit Wasser, Methylenchlorid und überschüssigem Ammoniak in üblicher Weise in die (R/S)-Base überführt, die als Eindampfungsrückstand der Methylenchlorid-Extrakte erhalten wird. Beim Behandeln mit Diethylether tritt Kristallisation ein. Die kristallisierte (R/S)-Base (5,8 g, 31, 3 % d. Th.) schmilzt bei 111 °C und hat eine spezifische Drehung von $[\alpha]_D20 = + 29,8^\circ$ (c = 1, 1 n HCl/Methanol 1:1).

c) (R/R)-5-Acetylamino-2-(1-phenylethylamino)-tetralin

Die vereinigten Mutterlaugen der Abtrennung des (R/S)-Mandelats (siehe oben) werden eingedampft und der Rückstand mit Wasser, Methylenchlorid und überschüssigem Ammoniak in der üblichen Weise in die Basenform überführt, die man als Eindampfungsrückstand der Methylenchloridextrakte erhält. Dieser wird aus 100 ml Essigester kristallisiert. Nochmaliges Umkristallisieren aus der 10-fachen Menge Essigester liefert 4,7 g (25,4 % d. Th.) reine (R/R)-Base mit einem Schmelzpunkt von 141 °C und einer spezifischen Drehung von $[\alpha]_D20 = + 145,3^\circ$ (c = 1, 1 n HCl/Methanol 1:1).

Die (R/S)- und (R/R)-Diastereomeren können auch als Basen durch Säulenchromatographie auf Kieselgel unter Verwendung von Methylenchlorid/Methanol/Ammoniak 90:10:0,5 als Laufmittel getrennt werden, wobei die (R/S)-Form zuerst eluiert wird.

Beispiel 4.13.2

(-)-(S)-5-Acetylamino-2-amino-tetralin-hydrochlorid

3,20 g (0,0104 Mol) (R/S)-5-Acetylamino-2-(1-phenylethylamino)-tetralin (Beispiel 4.13.1 b) werden in 70 ml Methanol in Gegenwart von 0,7 g 10 % Palladium/Kohle bei 20 - 60 °C analog Beispiel 4.4.1 hydriert. Man erhält die Titelverbindung in einer Ausbeute von 2,00 g (79,9 % d. Th.), Schmelzpunkt 310 °C, spezifische Drehung $[\alpha]_D20 = - 50,0^\circ$ (c = 1, 1 n HCl/Methanol 1:1).

Beispiel 4.13.3

(+)-(R)-5-Acetylamino-2-amino-tetralin-hydrochlorid

Ausgehend von 2,80 g (0,0091 Mol) (R/R)-5-Acetylamino-2-(1-phenyl-ethylamino)-tetralin (Beispiel 4.13.1 c) erhält man analog Beispiel 4.13.2 die Titelverbindung in einer Ausbeute von 1,8 g (82,2 % d. Th. Schmelzpunkt 311 °C, spezifische Drehung $[\alpha]_D20 = + 50,2^\circ$ (c = 1, 1 n HCl/Methanol 1:1).

Beispiel 4.13.4

(-)-(S)-2,5-Diamino-tetralin-dihydrochlorid

Ausgehend von 0.20 g (0,832 mMol) (-)-(S)-5-Acetylamino-2-amino-tetralin-hydrochlorid (Beispiel 4.13.2) erhält man analog Beispiel 4.2.1 die Titelverbindung in einer Ausbeute von 0,19 (98,1 % d. Th.) und mit einem Schmelzpunkt von > 320° C. $[\alpha]_D20$ = - 60,2° (c = 1, 1 n HCl/Methanol 1:1).

Beispiel 4.13.5

(+)-(R)-2,5-Diamino-tetralin-dihydrochlorid

Ausgehend von 0,20 g (0.832 mMol) (+)-(R)-5-Acetylamino-2-amino-tetralin-hydrochlorid (Beispiel 4.13.3) erhält man analog Beispiel 4.2.1 die Titelverbindung in einer Ausbeute von 0,19 g (98,1 % d. Th.) und mit einem Schmelzpunkt von > 320° C. $[\alpha]_D20$ = + 60,1° (c = 1, 1 n HCl/Methanol 1:1).

Beispiel 4.13.6

(-)-(S)-5-Amino-2-n-propylamino-tetralin-dihydrochlorid

Man erhält die Titelverbindung analog Beispielen für die racemische Form. Schmelzpunkt > 300° C, $[\alpha]$-$_D$20 = -56,3° (c = 1, 1 n HCl/Methanol 1:1)

Beispiel 4.13.7

(+)-(R)-5-Amino-2-n-propylamino-tetralin-dihydrochlorid

Man erhält die Titelverbindung analog Beispielen für die racemische Form. Schmelzpunkt > 300° C $[\alpha]$-$_D$20 = +56,2° (c = 1, 1 n HCl/Methanol 1:1).

Beispiel 4.13.8

(-)-(S)-5-Acetylamino-2-di-n-propylamino-tetralin-hydrochlorid

Man erhält die Titelverbindung analog Beispielen für die racemische Form. Schmelzpunkt 236° C, $[\alpha]$-$_D$20 = -42,9° (c = 1, 1 n HCl/Methanol 1:1).

Beispiel 4.13.9

(+)-(R)-5-Acetylamino-2-di-n-propylamino-tetralin-hydrochlorid

Man erhält die Titelverbindung analog Beispielen für die racemische Form. Schmelzpunkt 236° C, $[\alpha]$-$_D$20 = +43,1° (c = 1, 1 n HCl/Methanol 1:1).

Beispiel 4.13.10

(-)-(S)-5-Amino-2-di-n-propylamino-tetralin-dihydrochlorid

Man erhält die Titelverbindung analog Beispielen für die racemische Form. Schmelzpunkt 278° C, $[\alpha]$-$_D$20 = -52,9° (c = 1, 1 n HCl/Methanol 1:1).

Beispiel 4.13.11

(+)-(R)-5-Amino-2-di-n-propylamino-tetralin-dihydrochlorid

Man erhält die Titelverbindung analog Beispielen für die racemische Form. Schmelzpunkt 278° C, $[\alpha]_D20$ = +54,8° (c = 1, 1 n HCl/Methanol 1:1)

## Ansprüche

1) 2,5-Diaminotetraline (2,5-Diamino-1,2,3,4-tetra-hydronaphthaline) der allgemeinen Formel 1,

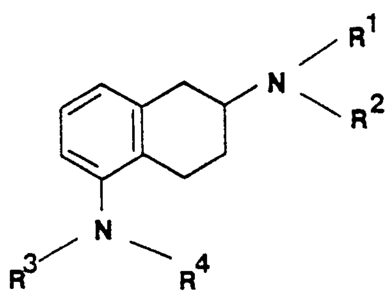

(1)

worin

R' Wasserstoff, $C_1$-$C_{12}$-Alkyl, $C_3$-$C_{12}$-Alkenyl, $C_3$-$C_{12}$-Alkinyl, $-(CH_2)_a$-Cycloalkyl, Aralkyl

und

a eine Zahl 1,2,3,4,5,6,7,8,9,10,11,12;

$R^2$ $C_1$-$C_{12}$-Alkyl, $C_3$-$C_{12}$-Alkenyl, $C_3$-$C_{12}$-Alkinyl, $-(CH_2)_b$-Cycloalkyl,

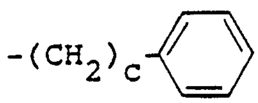 ,

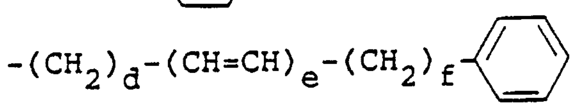 ,

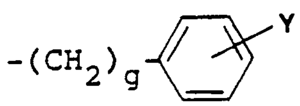 ,

$-(CH_2)_h$-Heteroaryl, Acyl

und

b eine Zahl 1,2,3,4,5,6,7,8,9,10,11,12,

c eine Zahl 1,2,3,4,5,6,7,8,9,10,11,12,

d eine Zahl 1,2,3,4,5,6,

e eine Zahl 1,2,3,

f eine Zahl 0,1,2,3,4,

g eine Zahl 1,2,3,4,5,6,

h eine Zahl 1,2,3,4,5,6,7,8,9,10,11,12,13,14,15, 16,17,18,19,20,

und

Y $C_1$-$C_{12}$-Alkyl, Halogen, Alkoxy, Hydroxy;

$R^3$ Wasserstoff, $C_1$-$C_{12}$-Alkyl, $C_3$-$C_{12}$-Alkenyl, $C_3$-$C_{12}$-Alkinyl, $-(CH_2)_i$-Cycloalkyl, Aralkyl, Formyl, Acyl, Alkylcarbonyl, Alkyloxycarbonyl,

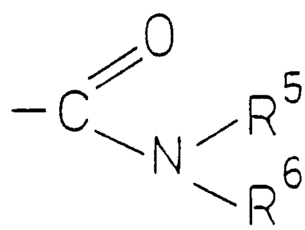

$R^5$ Alkyl,
$R^6$ Alkyl,
$R^5$ und $R^6$ auch zusammen mit dem Stickstoffatom einen Heterocyclus, der noch ein weiteres Heteroatom - wie z.B. Stickstoff, Sauerstoff oder Schwefel - enthalten kann - beispielsweise einen Morpholin-, Piperidin- oder Piperazinring - bilden können
und
i eine Zahl 1,2,3,4,5,6,7,8,9,10,11,12;
$R^4$ Wasserstoff oder $C_1$-$C_{12}$-Alkyl
bedeutet und deren Säureadditionssalze.

2) Verbindungen der allgemeinen Formel 1,
worin
$R^1$ Wasserstoff, $C_1$-$C_6$-Alkyl, $C_3$-$C_6$-Alkenyl, $C_3$-$C_6$-Alkinyl -$(CH_2)_a$-Cycloalkyl, Aralkyl mit 1 bis 6 Kohlenstoffatomen in aliphatischen Teil und
a eine Zahl 1,2,3,4,5,6;
$R^2$ $C_1$-$C_6$-Alkyl, $C_3$-$C_6$-Alkenyl, $C_3$-$C_6$-Alkinyl, -$(CH_2)_b$-Cycloalkyl,

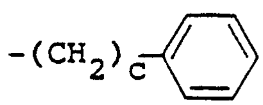 ,

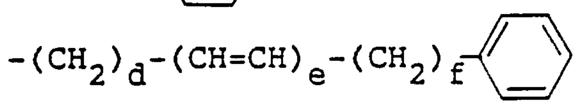 ,

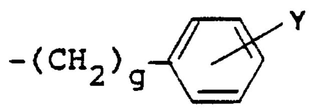 ,

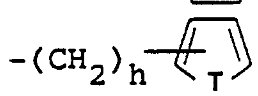 ,

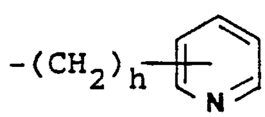 ,

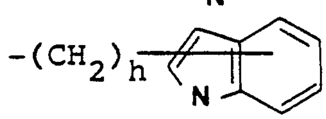

Acyl
und
b eine Zahl 1,2,3,4,5,6,
c eine Zahl 1,2,3,4,5,6,7,8,9,10,
d eine Zahl 1,2,3,
e eine Zahl 1,2,
f eine Zahl 0,1,2,
g eine Zahl 1,2,3,4,
h eine Zahl 1,2,3,4,5,6,7,8,9,10,12,13,14
und
Y $C_1$-$C_6$-Alkyl, Halogen, Niederalkoxy, Hydroxy,
T Sauerstoff, Schwefel, Stickstoff;
$R^3$ Wasserstoff, $C_1$-$C_6$-Alkyl, $C_3$-$C_6$-Alkenyl, $C_3$-$C_6$-Alkinyl, -$(CH_2)_i$-Cycloalkyl, Phenylalkyl, Formyl, Aryl, Alkylcarbonyl, Alkoxycarbonyl,

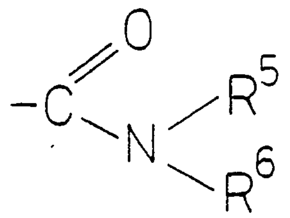,

$R^5$ Niederalkyl,
$R^6$ Niederalkyl,
$R^5$ und $R^6$ auch zusammen mit dem Stickstoffatom einen Heterocyclus, der noch ein weiteres Heteroatom - wie z.B. Stickstoff, Sauerstoff oder Schwefel enthalten kann - beispielsweise einen Morpholin-, Piperidin- oder Piperazinring - bilden können
und
i eine Zahl 1,2,3,4,5,6;
$R^4$ Wasserstoff oder Niederalkyl,
bedeutet und deren Säureadditionssalze.

3) Verbindungen der allgemeinen Formel 1, worin $R^1$ Wasserstoff, Methyl, Ethyl, Propyl, Allyl, Propargyl, Cyclopropylmethyl, Benzyl; $R^2$ Methyl, Ethyl, Propyl, Allyl, Propargyl, Cyclopropylmethyl,

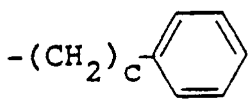, 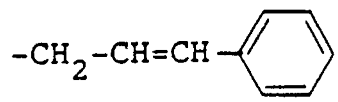,

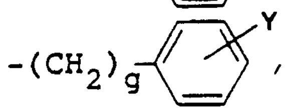,

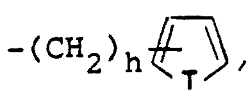,

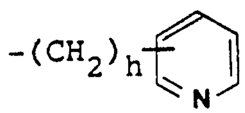, 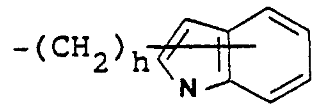,

Acyl
und
c eine Zahl 1,2,3,4,5,6,7,8,9,
g eine Zahl 2,3,
h eine Zahl 1,2,3,4,5,6,7,8,9,10,11,12,
Y Methyl, Fluor, Chlor, Brom, Methoxy, Hydroxy,
T Sauerstoff oder Schwefel;
$R^3$ Wasserstoff, Methyl, Ethyl, Propyl, Allyl, Propargyl, Cyclopropylmethyl, Benzyl, Phenylethyl, Phenylpropyl, Formyl, Acyl, Methylcarbonyl, Ethylcarbonyl, Trifluormethylcarbonyl, Methoxycarbonyl, Aminocarbonyl;
$R^4$ Wasserstoff, Methyl, Ethyl, n-Propyl und iso-Propyl
bedeutet und deren Säureadditionssalze.

4) (+)- bzw. (-)-5-Amino-2-(3-phenylpropyl-amino)-tetralin

5) (+)- bzw. (-)-5-Amino-2-[N-(3-phenylpropyl)-N-n-propyl-amino]-tetralin

6) (+)- bzw. (-)-5-Amino-2-(4-phenylbutyl-amino-tetralin

7) (+)- bzw. (-)-Amino-2-[N-(4-phenylbutyl)-N-n-propyl-amino]-tetralin

8) Pharmazeutische Zubereitung, gekennzeichnet durch einen Gehalt an einer Verbindung nach einem der Ansprüche 1 bis 7.

9) Verwendung von Verbindungen nach einem der Ansprüche 1 bis 7 bei der Herstellung von pharmazeutischen Zubereitungen zur Behandlung von Erkrankungen, die durch Störungen dopamimerger Systeme verursacht werden.

10) Verwendung von Verbindungen nach einem der Ansprüche 1 bis 7 als Arzneimittel.

11) Verfahren zur Herstellung von 2,5-Diaminotetralinen der allgemeinen Formel 1

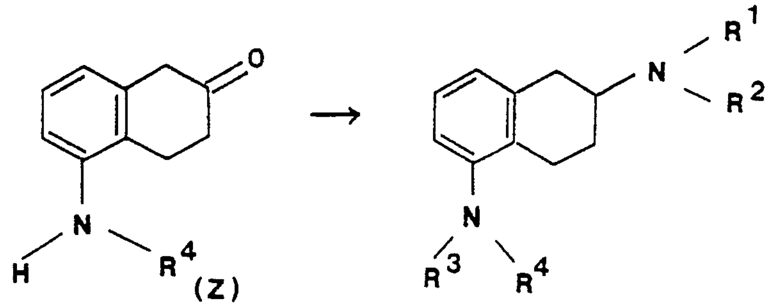

(2) (1)

worin $R^1$, $R^2$, $R^3$ und $R^4$ (= Z) die in Anspruch 1 angegebene Bedeutung haben, dadurch gekennzeichnet, daß man 5-Amino-2-tetralone der allgemeinen Formel 2 der reduktiven Aminierung unterwirft.

12) Verfahren zum Herstellen von Verbindungen der allgemeinen Formel 1

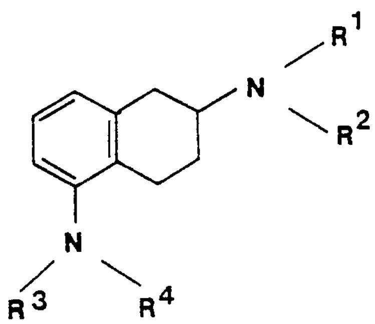

(1)

dadurch gekennzeichnet, daß man

a) - im Falle

$R^1$ mit Ausnahme von Wasserstoff die in Anspruch 1 angegebene Bedeutung hat;

$R^2$ Wasserstoff;

$R^3$ Benzyl, Acyl oder eine andere zum Schutz von Aminofunktionen geeignete Schutzgruppe Z;

$R^4$ Wasserstoff

bedeutet -

5-Amino-2-tetralone der allgemeinen Formel 2

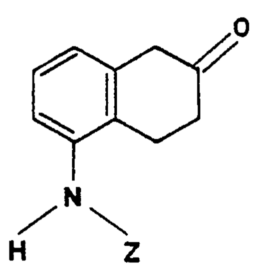

(2)

unter reduktiven Bedingungen in einem inerten Lösungsmittel unter an sich bekannten Reaktionsbedingungen -gegebenenfalls in Anwesenheit eines Katalysators - mit einem Amin der allgemeinen Formel 19

$H_2N$-$R^1$ (19)

- worin $R^1$ mit Ausnahme von Wasserstoff die in Anspruch 1 angegebene Bedeutung hat -umsetzt.

b) - im Falle

$R^1$ - mit Ausnahme von Wasserstoff - und $R^2$ die in Anspruch 1 angegebene Bedeutung haben

$R^3$ Benzyl, Acyl oder eine andere zum Schutz von Aminofunktionen geeignete Schutzgruppe Z;

$R^4$ Wasserstoff

bedeutet -

5-Amino-2-tetralone der allgemeinen Formel 2 unter reduktiven Bedingungen in einem inerten Lösungsmittel - gegebenenfalls in Anwesenheit eines Katalysators - mit einem Amin der allgmeinen Formel 20

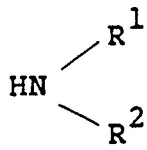 (20),

worin $R^1$ und $R^2$ mit Ausnahme von Wasserstoff die in Anspruch 1 angegebene Bedeutung haben umsetzt.
c) - im Falle
$R^1$ mit Ausnahme von Wasserstoff die in Anspruch 1 angegebene Bedeutung hat;
$R^2$, $R^3$ und $R^4$ Wasserstoff
bedeutet -
aus Verbindungen der allgemeinen Formel 1, worin
$R^1$ mit Ausnahme von Wasserstoff die in Anspruch 1 angegebene Bedeutung hat;
$R^2$ Wasserstoff;
$R^3$ Benzyl, Acyl oder eine andere zum Schutz von Aminofunktionen geeignete Schutzgruppe Z;
$R^4$ Wasserstoff
bedeutet
die Benzyl-, Acyl- oder gegebenenfalls eine andere zum Schutz von Aminofunktion geeignete Schutzgruppe Z unter an sich bekannten Reaktionsbedingungen abspaltet und mit Wasserstoff substituiert.
d) - im Falle
$R^1$ und $R^2$ mit Ausnahme von Wasserstoff die in Anspruch 1 angegebene Bedeutung haben;
$R^3$ und $R^4$ Wasserstoff
bedeuten -
aus Verbindungen der allgemeinen Formel 1, worin
$R^1$ und $R^2$ mit Ausnahme von Wasserstoff die in Anspruch 1 angegebene Bedeutung haben;
$R^3$ Benzyl, Acyl oder gegebenenfalls eine andere zum Schutz von Aminofunktionen geeignete Schutzgruppe Z;
$R^4$ Wasserstoff bedeutet
die Benzyl-, Acyl- oder gegebenenfalls eine andere Schutzgruppe Z unter an sich bekannten Reaktionsbedingungen abspaltet und mit Wasserstoff substituiert.
e) - im Falle
$R^1$ mit Ausnahme von Wasserstoff die in Anspruch 1 angegebene Bedeutung hat;
$R^2$ Acyl;
$R^3$ Benzyl, Acyl oder eine andere zum Schutz einer Aminofunktion geeignete Schutzgruppe;
$R^4$ Wasserstoff
bedeutet -
Verbindungen der allgemeinen Formel 1, worin
$R^1$ mit Ausnahme von Wasserstoff die in Anspruch 1 angegebene Bedeutung hat;
$R^2$ Wasserstoff;
$R^3$ Benzyl, Acyl oder gegebenenfalls eine andere zum Schutz einer Aminofunktion geeignete Schutzgruppe Z;
$R^4$ Wasserstoff
bedeutet -
mit einem Carbonsäurederivat der allgemeinen Formel 22

$$Q\overset{O}{\overset{\|}{C}}-R^8 \qquad (22)$$

wobei $R^8CH_2$ = $R^2$ in Anspruch 1 entspricht und $R^2$ die in Anspruch 1 angegebene Bedeutung besitzt und Q eine durch eine Aminofunktion substituierbare Gruppe bedeutet, umsetzt.
f) - im Falle
$R^1$ mit Ausnahme von Wasserstoff die in Anspruch 1 angegebene Bedeutung hat;
$R^2$ Acyl;
$R^3$ und $R^4$ Wasserstoff
bedeutet -
aus Verbindungen der allgemeinen Formel 1, worin
$R^1$ mit Ausnahme von Wasserstoff die in Anspruch 1 angegebene Bedeutung hat;

$R^2$ Alkylcarbonyl;
$R^3$ Benzyl, Acyl oder gegebenenfalls eine andere zum Schutz einer Aminofunktion geeignete Schutzgruppe Z;
$R^4$ Wasserstoff
bedeutet
die Benzyl, Acyl- oder gegebenenfalls eine andere zum Schutz einer Aminofunktion geeignete Schutzgruppe Z unter an sich bekannten Reaktionsbedingungen abspaltet und mit Wasserstoff substituiert.
g) - im Falle
$R^1$ mit Ausnahme von Wasserstoff - und
$R^2$ die in Anspruch 1 angegebene Bedeutung haben und
$R^3$ und $R^4$ Wasserstoff bedeuten -
Verbindungen der allgemeinen Formel 1, worin
$R^1$ mit Ausnahme von Wasserstoff die in Anspruch 1 angegebene Bedeutung hat;
$R^2$ eine Acylgruppe der allgemeinen Formel 23
O
‖
C -$R^8$ (23)
bedeutet, wobei $R^8$-$CH_2$ in Anspruch 1 $R^2$ entspricht und $R^2$ die dort angegebene Bedeutung hat;
$R^3$ und $R^4$ Wasserstoff
bedeuten -
unter an sich bekannten Reaktionsbedingungen reduziert.
h) - im Falle
$R^1$ mit Ausnahme von Wasserstoff die in Anspruch 1 angegebene Bedeutung hat;
$R^2$ die in Anspruch 1 angegebene Bedeutung hat;
$R^3$ mit Ausnahme von Wasserstoff die in Anspruch 1 angegebene Bedeutung hat;
$R^4$ Wasserstoff
bedeutet-
Verbindungen der allgemeinen Formel 1, worin
$R^1$ mit Ausnahme von Wasserstoff die in Anspruch 1 angegebene Bedeutung hat;
$R^2$ die in Anspruch 1 angegebene Bedeutung hat;
$R^3$ eine Acylgruppe der allgemeinen Formel 24
O
‖
C -$R^9$ (24)
bedeutet, wobei $R^9$-$CH_2$ = $R^3$ in Anspruch 1 entspricht und $R^3$ mit Ausnahme von Formyl, Acyl und Alkoxycarbonyl die in Anspruch 1 angegebene Bedeutung hat;
$R^4$ Wasserstoff bedeutet;
unter an sich bekannten Reaktionsbedingungen reduziert.
i) - im Falle
$R^1$, $R^2$ und $R^3$ Wasserstoff bedeuten,
$R^4$ Acyl, Benzyl oder gegebenenfalls eine andere zum Schutz einer Aminofunktion geeignete Schutzgruppe Z
bedeutet -
Verbindungen der allgemeinen Formel 2

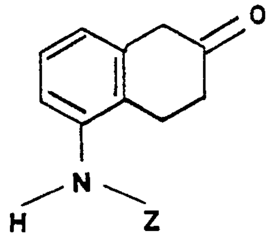

(2)

wobei Z = $R^4$ entspricht unter an sich bekannten Reaktionsbedingungen mit Ammoniak umsetzt.
j) - im Falle
$R^1$ mit Ausnahme von Wasserstoff die in Anspruch 1 angegebene Bedeutung hat;
$R^2$ und $R^3$ Wasserstoff;
$R^4$ Acyl, Benzyl oder gegebenenfalls eine andere zum Schutz einer Aminofunktion geeignete Schutzgruppe Z

bedeutet-
Verbindungen der allgemeinen Formel 2, worin
$R^1$, $R^2$ und $R^3$ Wasserstoff;
$R^4$ Acyl, Benzyl oder gegebenenfalls eine andere zum Schutz einer Aminofunktion geeignete Schutzgruppe Z
bedeutet -
mit einem Alkylierungsmittel der allgemeinen Formel 25
$R^1$-X (25)
unsetzt, worin
X ein Halogen, einen Sulfat-, einen Tosylat- oder einen Halogenmethansulfonatrest oder eine andere durch eine Aminogruppe substituierbare Austrittsgruppe bedeutet.